# EUROPEAN PATENT SPECIFICATION

(11) **EP 1 196 186 B1**
(45) Date of publication and mention of the grant of the patent: **31.10.2007**
(21) Application number: 00947204.4
(22) Date of filing: 11.07.2000
(51) Int. Cl.: A61K 38/17

(54) **PROMOTION OR INHIBITION OF ANGIOGENESIS AND CARDIOVASCULARIZATION BY TUMOR NECROSIS FACTOR LIGAND/RECEPTOR HOMOLOGS**
STIMULIERUNG ODER HEMMUNG VON ANGIOGENESE UND HERZVASKULARISIERUNG MIT TUMOR NEKROSE FAKTOR LIGAND/REZEPTOR HOMOLOGEN
STIMULATION OU INHIBITION DE L'ANGIOGENESE ET DE LA CARDIOVASCULARISATION AVEC DES HOMOLOGUES DE LIGANDS ET DE RECEPTEURS DU FACTEUR DE NECROSE TUMORALE

(30) Priority: 12.07.1999 US 143304 P
(43) Date of publication of application: 17.04.2002
(73) Proprietor: GENENTECH, INC., South San Francisco, CA 94080-4990 (US)
(72) Inventor: WILLIAMS, P., Mickey, Half Moon Bay, CA 94019 (US); GERRITSEN, Mary, E., San Mateo, CA 94402 (US)
(74) Representative: Kiddle, Simon John
(86) International application number: PCT/US2000/018867
(87) International publication number: WO 2001/003720

(56) References cited:
- WO-A-00/32221
- WO-A-00/53753
- WO-A-99/40196
- GURNEY A L ET AL: "IDENTIFICATION OF A NEW MEMBER OF THE TUMOR NECROSIS FACTOR FAMILY AND ITS RECEPTOR, A HUMAN ORTHOLOG OF MOUSE GITR" CURRENT BIOLOGY,CURRENT SCIENCE,,GB, vol. 9, no. 2, 1999, pages 215-218, XP000938588 ISSN: 0960-9822
- KWON BYUNGSUK ET AL: "Identification of a novel activation-inducible protein of the tumor necrosis factor receptor superfamily and its ligand" JOURNAL OF BIOLOGICAL CHEMISTRY,THE AMERICAN SOCIETY OF BIOLOGICAL CHEMISTS, INC.,,US, vol. 274, no. 10, 5 March 1999 (1999-03-05), pages 6056-6061, XP002147323 ISSN: 0021-9258

## Description

### Background of the Invention

### Field of the Invention

The present invention relates to compositions and methods for promoting or inhibiting angiogenesis and/or cardiovascularization in mammals in need of such biological effect. This includes the diagnosis and treatment of cardiovascular disorders as well as oncological disorders.

### Description of Background

### Cardiac Disorders and Factors

Heart failure affects approximately five million Americans, and new cases of heart failure number about 400,000 each year. It is the single most frequent cause of hospitalization for people age 65 and older in the US. Recent advances in the management of acute cardiac diseases, including acute myocardial infarction, are resulting in an expanding patient population that will eventually develop chronic heart failure. From 1979 to 1995 hospitalizations for congestive heart failure (CHF) rose from 377,000 to 872,000 (a 130 percent increase) and CHF deaths increased 116 percent.

CHF is a syndrome characterized by left ventricular dysfunction, reduced exercise tolerance, impaired quality of life, and markedly shortened life expectancy. The sine qua non of heart failure is an inability of the heart to pump blood at a rate sufficient to meet the metabolic needs of the body's tissues (in other words, there is insufficient cardiac output).

At least four major compensatory mechanisms are activated in the setting of heart failure to boost cardiac output, including peripheral vasoconstriction, increased heart rate, increased cardiac contractility, and increased plasma volume. These effects are mediated primarily by the sympathetic nervous system and the renin-angiotensin system. See Eichhorn, American Journal of Medicine, 104: 163-169 (1998). Increased output from the sympathetic nervous system increases vascular tone, heart rate, and contractility. Angiotensin II elevates blood pressure by 1) directly stimulating vascular smooth muscle contraction, 2) promoting plasma volume expansion by stimulating aldosterone and antidiuretic hormone secretion, 3) stimulating sympathetic-mediated vascular tone, and 4) catalyzing the degradation of bradykinin, which has vasodilatory and natriuretic activity. See review by Brown and Vaughan, Circulation, 97: 1411-1420 (1998). As noted below, angiotensin II may also have directly deleterious effects on the heart by promoting myocyte necrosis (impairing systolic function) and intracardiac fibrosis (impairing diastolic and in some cases systolic function). See Weber, Circulation, 96: 4065-4082 (1998).

A consistent feature of congestive heart failure (CHF) is cardiac hypertrophy, an enlargement of the heart that is activated by both mechanical and hormonal stimuli and enables the heart to adapt to demands for increased cardiac output. Morgan and Baker, Circulation 83: 13-25 (1991). This hypertrophic response is frequently associated with a variety of distinct pathological conditions such as hypertension, aortic stenosis, myocardial infarction, cardiomyopathy, valvular regurgitation, and intracardiac shunt, all of which result in chronic hemodynamic overload.

Hypertrophy is generally defined as an increase in size of an organ or structure independent of natural growth that does not involve tumor formation. Hypertrophy of the heart is due either to an increase in the mass of the individual cells (myocytes), or to an increase in the number of cells making up the tissue (hyperplasia), or both. While the enlargement of an embryonic heart is largely dependent on an increase in myocyte number (which continues until shortly after birth), post-natal cardiac myocytes lose their proliferative capacity. Further growth occurs through hypertrophy of the individual cells.

Adult myocyte hypertrophy is initially beneficial as a short term response to impaired cardiac function by permitting a decrease in the load on individual muscle fibers. With severe, long-standing overload, however, the hypertrophied cells begin to deteriorate and die. Katz, "Heart Failure", in: Katz A.M. ed., Physiology of the Heart (New York: Raven Press, 1992) pp. 638-668. Cardiac hypertrophy is a significant risk factor for both mortality and morbidity in the clinical course of heart failure. Katz, Trends Cardiovase. Med., 5: 37-44 (1995). For further details of the causes and pathology of cardiac hypertrophy see, e.g., Heart Disease. A Textbook of Cardiovascular Medicine, Braunwald, E. ed. (W.B. Saunders Co., 1988), Chapter 14, "Pathophysiology of Heart Failure."

On a cellular level, the heart is composed of myocytes and surrounding support cells, generically called non-myocytes. While non-myocytes are primarily fibroblast/mesenchymal cells, they also include endothelial and smooth muscle cells. Indeed, although myocytes make up most of the adult myocardial mass, they represent only about 30% of the total cell numbers present in heart. In response to hormonal, physiological, hemodynamic, and pathological stimuli, adult ventricular muscle cells can adapt to increased workloads through the activation of a hypertrophic process. This response is characterized by an increase in myocyte cell size and contractile protein content of individual cardiac muscle cells, without concomitant cell division and activation of embryonic genes, including the gene for atrial natriuretic peptide (ANP). Chien et al., FASEB J., 5: 3037-3046 (1991); Chien et al., Annu. Rev. Physiol., 55: 77-95 (1993). An increment in myocardial mass as a result of an increase in myocyte size that is associated with an accumulation of interstitial collagen within the extracellular matrix and around intramyocardial coronary arteries has been described in left ventricular hypertrophy secondary to pressure overload in humans. Caspari et al., Cardiovasc. Res., 11: 554-558 (1977); Schwarz et al., Am. J. Cardiol., 42: 895-903 (1978); Hess et al., Circulation, 63: 360-371 (1981); Pearlman et al., Lab. Invest., 46: 158-164 (1982).

It has also been suggested that paracrine factors produced by non-myocyte supporting cells may additionally be involved in the development of cardiac hypertrophy, and various non-myocyte derived hypertrophic factors, such as, leukocyte inhibitory factor (LIF) and endothelin, have been identified. Metcalf, Growth Factors, 7: 169-173 (1992); Kurzrock et al., Endocrine Reviews, 12: 208-217 (1991); Inoue et al., Proc. Natl. Acad: Sci. USA, 86: 2863-2867 (1989); Yanagisawa and Masaki, Trends Pharm. Sci., 10: 374-378 (1989); U.S. Patent No. 5,573,762 (issued November 12, 1996). Further exemplary factors that have been identified as potential mediators of cardiac hypertrophy include cardiotrophin-1 (CT-1) (Pennica et al., Proc. Nat. Acad. Sci. USA, 92: 1142-1146 (1995)), catecholamines, adrenocorticosteroids, angiotensin, and prostaglandins.

At present, the treatment of cardiac hypertrophy varies depending on the underlying cardiac disease. Catecholamines, adrenocorticosteroids, angiotensin, prostaglandins, LIF, endothelin (including endothelin-l, -2; and -3 and big endothelin), and CT-1 are among the factors identified as potential mediators of hypertrophy. For example, beta-adrenergic receptor blocking drugs (beta-blockers, e.g., propranolol, timolol, tertalolol, carteolol, nadolol, betaxolol, penbutolol, acetobutolol, atenolol, metoprolol, carvedilol, etc.) and verapamil have been used extensively in the treatment of hypertrophic cardiomyopathy. The beneficial effects of beta-blockers on symptoms (e.g., chest pain) and exercise tolerance are largely due to a decrease in the heart rate with a consequent prolongation of diastole and increased passive ventricular filling. Thompson et al., Br. Heart J., 44: 488-98 (1980); Harrison et al., Circulation, 29: 84-98 (1964). Verapamil has been described to improve ventricular filling and probably reducing myocardial ischemia. Bonow et al.. Circulation, 72: 853-64 (1985).

Nifedipine and diltiazem have also been used occasionally in the treatment of hypertrophic cardiomyopathy. Lorell et al., Circulation, 65: 499-507 (1982): Betocchi et al.. Am. J. Cardiol., 78: 451-457 (1996). However, because of its potent vasodilating properties, nifedipine may be harmful, especially in patients with outflow obstruction. Disopyramide has been used to relieve symptoms by virtue of its negative inotropic properties. Pollick, N. Engl. J. Med., 307: 997-999 (1982). In many patients, however, the initial benefits decrease with time. Wigle et al., Circulation, 92: 1680-1692 (1995).

Antihypertensive drug therapy has been reported to have beneficial effects on cardiac hypertrophy associated with elevated blood pressure. Examples of drugs used in antihypertensive therapy, alone or in combination, are calcium antagonists, e.g., nitrendipine; -adrenergic receptor blocking agents, e.g., those listed above; angiotensin converting enzyme (ACE) inhibitors such as quinapril, captopril, enalapril, ramipril, benazepril, fosinopril, and lisinopril; diuretics, e.g., chorothiazide, hydrochlorothiazide, hydroflumethazide, methylchlothiazide, benzthiazide, dichlorphenamide, acetazolamide, and indapamide; and calcium channel blockers, e.g., diltiazem, nifedipine, verapamil, and nicardipine.

For example, treatment of hypertension with diltiazem and captopril showed a decrease in left ventricular muscle mass, but the Doppler indices of diastolic function did not normalize. Szlachcic et al., Am. J. Cardiol., 63: 198-201 (1989); Shahi et al., Lancet, 336: 458-461 (1990). These findings were interpreted to indicate that excessive amounts of interstitial collagen may remain after regression of left ventricular hypertrophy. Rossi et al., Am. Heart J., 124: 700-709 (1992). Rossi *et al., supra,* investigated the effect of captopril on the prevention and regression of myocardial cell hypertrophy and interstitial fibrosis in pressure overload cardiac hypertrophy, in experimental rats.

Agents that increase cardiac contractility directly (iontropic agents) were initially thought to benefit patients with heart failure because they improved cardiac output in the short term. However, all positive inotropic agents except digoxigenin have been found to result in increased long-term mortality, in spite of short-term improvements in cardiac performance. Massie, Curr. Op. in Cardiology, 12: 209-217 (1997); Reddy et al., Curr. Opin. Cardiol., 12: 233-241 (1997). Beta-adrenergic receptor blockers have recently been advocated for use in heart failure. Evidence from clinical trials suggests that improvements in cardiac function can be achieved without increased mortality, though documented improvements patient survival have not yet been demonstrated. See also U.S. Pat. Nos. 5,624,806; 5,661,122: and 5,610,134 and WO 95/28173 regarding the use of cardiotropin-1 or antagonists thereof, or growth hormone and/or insulin-like growth factor-I in the treatment of CHF. Another treatment modality is heart transplantation, but this is limited by the availability of donor hearts.

Endothelin is a vasoconstricting peptide comprising 21 amino acids, isolated from swine arterial endothelial culture supernatant and structurally determined. Yanagisawa et al., Nature, 332: 411-415 (1988). Endothelin was later found to exhibit various actions, and endothelin antibodies as endothelin antagonists have proven effective in the treatment of myocardial infarction, renal failure, and other diseases. Since endothelin is present in live bodies and exhibits vasoconstricting action, it is expected to be an endogenous factor involved in the regulation of the circulatory system, and may be associated with hypertension, cardiovascular diseases such as myocardial infarction, and renal diseases such as acute renal failure. Endothelin antagonists are described, for example, in U.S. Pat. No. 5,773,414; JP Pat. Publ. 3130299/1991. EP 457,195; EP 460,679; and EP 552,489. A new endothelin B receptor for identifying endothelin receptor antagonists is described in U.S. Pat. No. 5,773,223.

Current therapy for heart failure is primarily directed to using angiotensin-converting enzyme (ACE) inhibitors, such as captopril, and diuretics. These drugs improve hemodynamic profile and exercise tolerance and reduce the incidence of morbidity and mortality in patients with CHF. Kramer et al., Circulation, 67(4): 807-816 (1983): Captopril Multicenter Research Group. J.A.C.C., 2(4): 755-763 (1983): The CONSENSUS Trial Study Group. N. Engl. J. Med., 316(23): 1429-1435 (1987); The SOLVD Investigators. N. Engl. J. Med., 325(5): 293-302 (1991). Further, they are useful in treating hypertension, left ventricular dysfunction., atherosclerotic vascular disease, and diabetic nephropathy. Brown and Vaughan, *supra.* However, despite proven efficacy, response to ACE inhibitors has been limited. For example, while prolonging survival in the setting of heart failure. ACE inhibitors appear to slow the progression towards end-stage heart failure, and substantial numbers of patients on ACE inhibitors have functional class III heart failure.

Moreover, improvement of functional capacity and exercise time is only small and mortality, although reduced, continues to be high. The CONSENSUS Trial Study Group, N. Engl: J. Med., 316(23): 1429-1453 (1987); The SOLVD Investigators, N. Engl. J. Med., 325(5): 293-302 (1991); Cohn et al., N. End. J. Med., 325(5): 303-310 (1991); The Captopril-Digoxin Multicenter Research Group, JAMA, 259(4): 539-544 (1988). Hence, ACE inhibitors consistently appear unable to relieve symptoms in more than 60% of heart failure patients and reduce mortality of heart failure only by approximately 15-20%. For further adverse effects, see Brown and Vaughan, *supra.*

An alternative to ACE inhibitors is represented by specific AT1 receptor antagonists. Clinical studies are planned to compare the efficacy of these two modalities in the treatment of cardiovascular and renal disease. However, animal model data suggests that the ACE/Ang II pathway, while clearly involved in cardiac hypertrophy, is not the only, or even the primary pathway active in this role. Mouse genetic "knockout" models have been made to test individual components of the pathway. In one such model, the primary cardiac receptor for Ang II, AT sub 1A, has been genetically deleted; these mice do not develop hypertrophy when Ang II is given experimentally (confirming the basic success of the model in eliminating hypertrophy secondary to Ang II). However, when the aorta is constricted in these animals (a model of hypertensive cardiac stress), the hearts still become hypertrophic. This suggests that alternative signaling pathways, not depending on this receptor (AT sub 1A), are activated in hypertension. ACE inhibitors would presumably not be able to inhibit these pathways. See Harada et al.; Circulation, 97: 1952-1959 (1998). See also Homey, Circulation, 97: 1890-1892 (1998) regarding the enigma associated with the process and mechanism of cardiac hypertrophy.

About 750,000 patients suffer from acute myocardial infarction (AMI) annually, and approximately one-fourth of all deaths in the United States are due to AMI. In recent years, thrombolytic agents, e.g. streptokinase, urokinase, and in particular tissue plasminogen activator (t-PA) have significantly increased the survival of patients who suffered myocardial infarction. When administered as a continuous intravenous infusion over 1.5 to 4 hours, t-PA produces coronary patency at 90 minutes in 69% to 90% of the treated patients. Topol et al., Am. J. Cardiol., 61, 723-728 (1988); Neuhaus et al., J. Am. Coll. Cardiol., 12: 581-587 (1988); Neuhaus et al., J. Am. Coll. Cardiol., 14: 1566-1569 (1989). The highest patency rates have been reported with high dose or accelerated dosing regimens. Topol, J. Am. Coll. Cardiol., 15: 922-924 (1990). t-PA may also be administered as a single bolus, although due to its relatively short half-life, it is better suited for infusion therapy. Tebbe et al., Am. J. Cardiol., 64: 448-453 (1989). A t-PA variant, specifically designed to have longer half-life and very high fibrin specificity, TNK t-PA (a T103N, N117Q, KHRR(296-299)AAAA t-PA variant, Keyt et al., Proc. Natl. Acad. Sci. USA, 91: 3670-3674 (1994)) is particularly suitable for bolus administration. However, despite all these advances, the long-term prognosis of patient survival depends greatly on the post-infarction monitoring and treatment of the patients, which should include monitoring and treatment of cardiac hypertrophy.

### Growth Factors

Various naturally occurring polypeptides reportedly induce the proliferation of endothelial cells. Among those polypeptides are the basic and acidic fibroblast growth factors (FGF) (Burgess and Maciag, Annual Rev. Biochem., 58: 575 (1989)), platelet-derived endothelial cell growth factor (PD-ECGF) (Ishikawa et al., Nature, 338: 557 (1989)), and vascular endothelial growth factor (VEGF). Leung et al., Science, 246: 1306 (1989); Ferrara and Henzel, Biochem. Biophys. Res. Commun., 161: 851 (1989); Tischer et al., Biochem. Biophys. Res. Commun., 165: 1198 (1989); EP 471.754B granted July 31. 1996.

Media conditioned by cells transfected with the human VEGF (hVEGF) cDNA promoted the proliferation of capillary endothelial cells, whereas control cells did not. Leung et al., Science, 246: 1306 (1989). Several additional cDNAs were identified in human cDNA libraries that encode 121-, 189-, and 206-amino acid isoforms of hVEGF (also collectively referred to as hVEGF-related proteins). The 121-amino acid protein differs from hVEGF by virtue of the deletion of the 44 amino acids between residues 116 and 159 in hVEGF. The 189-amino acid protein differs from hVEGF by virtue of the insertion of 24 amino acids at residue 116 in hVEGF, and apparently is identical to human vascular permeability factor (hVPF). The 206-amino acid protein differs from hVEGF by virtue of an insertion of 41 amino acids at residue 116 in hVEGF. Houck et al., Mol. Endocrin., 5: 1806 (1991); Ferrara et al., J. Cell. Biochem., 47: 211 (1991); Ferrara et al., Endocrine Reviews, 13: 18 (1992); Keck et al., Science, 246: 1309 (1989); Connolly et al., J. Biol. Chem., 264: 20017 (1989); EP 370.989 published May 30, 1990.

It is now well established that angiogenesis, which involves the formation of new blood vessels from preexisting endothelium, is implicated in the pathogenesis of a variety of disorders. These include solid tumors and metastasis, atherosclerosis, retrolental fibroplasia, hemangiomas, chronic inflammation, intraocular neovascular syndromes such as proliferative retinopathies, e.g., diabetic retinopathy, age-related macular degeneration (AMD), neovascular glaucoma, immune rejection of transplanted corneal tissue and other tissues, rheumatoid arthritis, and psoriasis. Folkman et al., J. Biol. Chem., 267: 10931-10934 (1992); Klagsbrun et al., Annu. Rev. Physiol., 53: 217-239 (1991); and Garner A, "Vascular diseases", In: Pathobiology of Ocular Disease. A Dynamic Approach, Garner A, Klintworth GK, Eds., 2nd Edition (Marcel Dekker, NY, 1994), pp 1625-1710.

In the case of tumor growth, angiogenesis appears to be crucial for the transition from hyperplasia to neoplasia, and for providing nourishment to the growing solid tumor. Folkman et aL, Nature. 339: 58 (1989). The neovascularization allows the tumor cells to acquire a growth advantage and proliferative autonomy compared to the normal cells. Accordingly, a correlation has been observed between density of microvessels in tumor sections and patient survival in breast cancer as well as in several other tumors. Weidner et al., N Engl J Med, 324: 1-6 (1991); Horak et al., Lancet, 340: 1120-1124 (1992); Macchiarini et al., Lancet, 340: 145-146 (1992).

The search for positive regulators of angiogenesis has yielded many candidates, including aFGF, bFGF, TGF-α, TGF-β, HGF, TNF-a, angiogenin, IL-8. etc. Folkman *et al.,* J.B.C., *supra,* and Klagsbrun *et al., supra.* The negative regulators so far identified include thrombosporidin (Good et al., Proc. Natl. Acad. Sci. USA., 87: 6624-6628 (1990)), the 16-kilodalton N-terminal fragment of prolactin (Clapp et al., Endocrinology, 133: 1292-1299 (1993)), angiostatin (O'Reilly et al. Cell, 79: 315-328 (1994)), and endostatin. O'Reilly et al., Cell, 88: 277-285 (1996).

Work done over the last several years has established the key role of VEGF, not only in stimulating vascular endothelial cell proliferation, but also in inducing vascular permeability and angiogenesis. Ferrara et al., Endocr. Rev., 18: 4-25 (1997). The finding that the loss of even a single VEGF allele results in embryonic lethality points to an irreplaceable role played by this factor in the development and differentiation of the vascular system. Furthermore. VEGF has been shown to be a key mediator of neovascularization associated with tumors and intraocular disorders. Ferrara *et al.,* Endocr. Rev., *supra.* The VEGF mRNA is overexpressed by the majority of human tumors examined. Berkman et al., J Clin Invest, 91: 153-159 (1993); Brown et al., Human Pathol., 26: 86-91 (1995); Brown et al., Cancer Res., 53: 4727-4735 (1993); Mattern et al., Brit. J. Cancer, 73: 931-934 (1996); Dvorak et al., Am J. Pathol., 146: 1029-1039 (1995).

Also, the concentration levels of VEGF in eye fluids are highly correlated to the presence of active proliferation of blood vessels in patients with diabetic and other ischemia-related retinopathies. Aiello et al., N. Eng. J. Med., 331: 1480-1487 (1994). Furthermore, recent studies have demonstrated the localization of VEGF in choroidal neovascular membranes in patients affected by AMD. Lopez et al., Invest. Ophthalmol. Vis. Sci., 37: 855-868 (1996).

Anti-VEGF neutralizing antibodies suppress the growth of a variety of human tumor cell lines in nude mice (Kim et al., Nature, 362: 841-844 (1993); Warren et al., J. Clin. Invest., 95: 1789-1797 (1995); Borgström et al., Cancer Res., 56: 4032-4039 (1996); Melnyk et al., Cancer Res., 56: 921-924 (1996)) and also inhibit intraocular angiogenesis in models of ischemic retinal disorders. Adamis et al., Arch. Ophthalmol., 114: 66-71 (1996). Therefore, anti-VEGF monoclonal antibodies or other inhibitors of VEGF action are promising candidates for the treatment of solid tumors and various intraocular neovascular disorders. Such antibodies are described, for example, in EP 817,648 published January 14, 1998 and in PCT/US 98/06724 filed April 3, 1998.

There exist several other growth factors and mitogens, including transforming oncogenes, that are capable of rapidly inducing a complex set of genes to be expressed by certain cells. Lau and Nathans, Molecular Aspects. of Cellular Regulation, 6: 165-202 (1991). These genes, which have been named immediate-early- or early-response genes, are transcriptionally activated within minutes after contact with a growth factor or mitogen, independent of *de novo* protein synthesis. A group of these intermediate-early genes encodes secreted, extracellular proteins that are needed for coordination of complex biological processes such as differentiation and proliferation, regeneration, and wound healing. Ryseck et al., Cell Growth Differ., 2: 235-233 (1991).

Highly-related proteins that belong to this group include *cef 10* (Simmons et al., Proc. Natl. Acad. Sci. USA, 86: 1178-1182 (1989)), *cyr* 61, which is rapidly activated by serum- or platelet-derived growth factor (PDGF) (O'Brien et al., Mol. Cell Biol., 10: 3569-3577 (1990), human connective tissue growth factor (CTGF) (Bradham et al., J. Cell. Biol., 114: 1285-1294 (1991)), which is secreted by human vascular endothelial cells in high levels after activation with transforming growth factor beta (TGF-β); exhibits PDGF-like biological and immunological activities, and competes with PDGF for a particular cell surface receptor, *fisp-12* (Ryseck et al., Cell Growth Differ., 2: 235-233 (1991)), human vascular IBP-like growth factor (VIGF) (WO 96/17931), and *nov,* normally arrested in adult kidney cells, which was found to be overexpressed in myeloblastosis-associated-virus-type-1-induced nephroblastomas. Joloit et al., Mol. Cell. Biol., 12: 10-21 (1992).

The expression of these immediate-early genes acts as "third messengers" in the cascade of events triggered by growth factors. It is also thought that they are needed to integrate and coordinate complex biological processes, such as differentiation and wound healing in which cell proliferation is a common event.

As additional mitogens, insulin-like growth factor binding proteins (IGFBPs) have been shown, in complex with insulin-like growth factor (IGF), to stimulate increased binding of IGF to fibroblast and smooth muscle cell surface receptors. Clemmons et al., J. Clin. Invest., 77: 1548 (1986). Inhibitory effects of IGFBP on various IGF actions *in vitro* include stimulation of glucose transport by adipocytes, sulfate incorporation by chondrocytes, and thymidine incorporation in fibroblast. Zapf et al., J. Clin. Invest., 63: 1077 (1979). In addition, inhibitory effects of IGFBPs on growth factor-mediated mitogen activity in normal cells have been shown.

### Tumor Necrosis Factor Receptors and Ligands

Various molecules, such as tumor necrosis factor-α ("TNF- α"), tumor necrosis factor-β ("TNF- β" or . "lymphotoxin-α"), lymphotoxin-β ("LT- β"), CD30 ligand, CD27 ligand, CD40 ligand, OX-40 ligand. 4-1BB ligand, Apo-1 ligand (also referred to as Fas ligand or CD95 ligand), and Apo-2 ligand (also referred to as TRAIL) have been identified as members of the tumor necrosis factor ("TNF") family of cytokines [See, e.g., Gruss and Dower, Blood, 85:3378-3404 (1995); Pitti et al., J. Biol. Chem., 271:12687-12690 (1996); Wiley et al., Immunity, 3:673-682 (1995); Browning et al., Cell, 72:847-856 (1993); Armitage et al. Nature, 357:80-82 (1992), WO 97/01633 published January 16, 1997; WO 97/25428 published July 17, 1997].

Induction of various cellular responses mediated by such TNF family cytokines is believed to be initiated by their binding to specific cell receptors. Two distinct TNF receptors of approximately 55-kDa (TNFR1) and 75-kDa (TNFR2) have been identified [Hohman et al., J. Biol. Chem., 264:14927-14934 (1989); Brockhaus et al., Proc. Natl. Acad. Sci., 87:3127-3131 (1990); EP 417,563; published March 20, 1991] and human and mouse cDNAs corresponding to both receptor types have been isolated and characterized (Loetscher et al., Cell, 61:351 (1990); Schall et al., Cell, 61:361 (1990); Smith et al., Science, 248:1019-1023 (1990); Lewis et al., Proc. Natl. Acad. Sci., 88:2830-2834 (1991); Goodwin et al., Mol. Cell. Biol., 11:3020-3026 (1991)]. Extensive polymorphisms have been associated with both TNF receptor genes [see, e.g., Takao et al., Immunogenetics, 37:199-203 (1993)]. Both TNFRs share the typical structure of cell surface receptors including extracellular, transmembrane and intracellular regions. The extracellular portions of both receptors are found naturally also as soluble TNF-binding proteins-[Nophar, Y. et al., EMBO J., 9:3269 (1990); and Kohno, T. et al., Proc. Natl. Acad. Sci. U.S.A., 87:8331 (1990)]. More recently, the cloning of recombinant soluble TNF receptors was reported by Hale et al. [J. Cell. Biochem. Supplement 15F, 1991, p. 113 (P424)].

The extracellular portion of type 1 and type 2 TNFRs (TNFR1 and TNFR2) contains a repetitive amino acid sequence pattern of four cysteine-rich domains (CRDs) designated 1 through 4, starting from the NH₂-terminus. Each CRD is about 40 amino acids long and contains 4 to 6 cysteine residues at positions which are well conserved [Schall et al., supra; Loetscher et al., supra; Smith et al., supra; Nophar et al., supra; Kohno et al., supra]. In TNFR1, the approximate boundaries of the four CRDs are as follows: CRD1- amino acids 14 to about 53; CRD2- amino acids from about 54 to about 97; CRD3- amino acids from about 98 to about 138; CRD4-amino acids from about 139 to about 167. In TNFR2, CRD1 includes amino acids 17 to about 54; CRD2- amino acids from about 55 to about 97; CRD3- amino acids from about 98 to about 140; and CRD4- amino acids from about 141 to about 179 [Banner et al., Cell, 73:431-435 (1993)]. The potential role of the CRDs in ligand binding is also described by Banner et al., supra.

A similar repetitive pattern of CRDs exists in several other cell-surface proteins, including the p75 nerve growth factor receptor (NGFR) [Johnson et al., Cell, 47:545 (1986); Radeke et al., Nature, 325:593 (1987)], the B cell antigen CD40 [Stamenkovic et al., EMBO J., 8:1403 (1989)], the T cell antigen OX40 [Mallet et al., EMBO J., 9:1063 (1990)] and the Fas antigen [Yonehara et al., supra and Itoh et al., Cell, 66:233-243 (1991)]. CRDs are also found in the soluble TNFR (sTNFR)-like T2 proteins of the Shope and myxoma poxviruses [Upton et al., Virology, 160:20-29 (1987): Smith et al., Biochem. Biophys. Res. Commun., 176:335 (1991): Upton et al., Virology, 184:370 (1991)]. Optimal alignment of these sequences indicates that the positions of the cysteine residues are well conserved. These receptors are sometimes collectively referred to as members of the TNF/NGF receptor superfamily. Recent studies on p75NGFR showed that the deletion of CRD I [Welcher. A.A. et al., Proc. Natl. Acad. Sci. USA, 88:159-163 (1991)] or a 5-amino acid insertion in this domain [Yan. H. and Chao. M.V., J. Biol. Chem., 266:12099-12104 (1991)] had little or no effect on NGF binding [Yan, H. and Chao. M.V., supra]. p75 NGFR contains a proline-rich stretch of about 60 amino acids, between its CRD4 and transmembrane region, which is not involved in NGF binding [Peetre, C. et al., Eur. J. Hematol., 41:414-419 (1988); Seckinger, P. et al., J. Biol. Chem., 264:11966-11973 (1989);. Yan. H. and Chao, M.V., supra]. A similar proline-rich region is found in TNFR2 but not in TNFR1.

The TNF family ligands identified to date, with the exception of lymphotoxin-α, are type II transmembrane proteins, whose C-terminus is extracellular. In contrast, most receptors in the TNF receptor (TNFR) family identified to date are type I transmembrane proteins. In both the TNF ligand and receptor families, however, homology identified between family members has been found mainly in the extracellular domain ("ECD"). Several of the TNF family cytokines, including TNF-α, Apo-1 ligand and CD40 ligand, are cleaved proteolytically at the cell surface; the resulting protein in each case typically forms a homotrimeric molecule that functions as a soluble cytokine. TNF receptor family proteins are also usually cleaved proteolytically to release soluble receptor ECDs that can function as inhibitors of the cognate cytokines.

Recently, other members of the TNFR family have been identified. Such newly identified members of the TNFR family include CAR1, HVEM and osteoprotegerin (OPG) [Brojatsch et al.; Cell, 87:845-855 (1996); Montgomery et al., Cell, 87:427-436 (1996); Marsters et al., J. Biol. Chem., 272:14029-14032 (1997); Simonet et al., Cell, 89:309-319 (1997)]. Unlike other known TNFR-like molecules, Simonet et al., supra, report that OPG contains no hydrophobic transmembrane-spanning sequence.

Moreover, a new member of the TNF/NGF receptor family has been identified in mouse, a receptor referred to as "GITR" for "glucocorticoid-induced tumor necrosis factor receptor family-related gene" [Nocentini et al., Proc. Natl. Acad. Sci. USA 94:6216-6221 (1997)]. The mouse GITR receptor is a 228 amino acid type I transmembrane protein that is expressed in normal mouse T lymphocytes from thymus, spleen and lymph nodes. Expression of the mouse GITR receptor was induced in T lymphocytes upon activation with anti-CD3 antibodies, Con A or phorbol 12-myristate 13-acetate. It was speculated by the authors that the mouse GITR receptor was involved in the regulation of T cell receptor-mediated cell death.

In Marsters et al., Curr. Biol., 6:750 (1996), investigators describe a full length native sequence human polypeptide, called Apo-3, which exhibits similarity to the TNFR family in its extracellular cysteine-rich repeats and resembles TNFR1 and CD95 in that it contains a cytoplasmic death domain sequence [see also Marsters et al., Curr. Biol., 6:1669 (1996)]. Apo-3 has also been referred to by other investigators as DR3, wsl-1 and TRAMP [Chinnaiyan et al., Science, 274:990 (1996); Kitson et al., Nature, 384:372 (1996); Bodmer et al., Immunity, 6:79 (1997)].

Pan et al. have disclosed another TNF receptor family member referred to as "DR4" [Pan et al., Science, 276:111-113 (1997)]. The DR4 was reported to contain a cytoplasmic death domain capable of engaging the cell suicide apparatus. Pan et al. disclose that DR4 is believed to be a receptor for the ligand known as Apo-2 ligand or TRAIL.

In Sheridan et al., Science, 277:818-921 (1997) and Pan et al., Science, 277:815-818 (1997), another molecule believed to be a receptor for the Apo-2 ligand (TRAIL) is described. That molecule is referred to as DR5 (it has also been alternatively referred to as Apo-2). Like DR4. DR5 is reported to contain a cytoplasmic death domain and be capable of signaling apoptosis.

In Sheridan et al., supra, a receptor called DcR1 (or alternatively, Apo-2DcR) is disclosed as being a potential decoy receptor for Apo-2 ligand (TRAIL). Sheridan et al. report that DcR1 can inhibit Apo-2 ligand function *in vitro.* See also, Pan et al., supra, for disclosure on the decoy receptor referred to as TRID.

For a review of the TNF family of cytokines and their receptors, see Gruss and Dower, supra.

### Need for Further Treatments

In view of the role of vascular endothelial cell growth and angiogenesis in many diseases and disorders, it is desirable to have a means of reducing or inhibiting one or more of the biological effects causing these processes. It is also desirable to have a means of assaying for the presence of pathogenic polypeptides in normal and diseased conditions, and especially cancer. Further, in a specific aspect, as there is no generally applicable therapy for the treatment of cardiac hypertrophy, the identification of factors that can prevent or reduce cardiac myocyte hypertrophy is of primary importance in the development of new therapeutic strategies to inhibit pathophysiological cardiac growth. While there are several treatment modalities for various cardiovascular and oncologic disorders, there is still a need for additional therapeutic approaches.

### Summary of the Invention

Accordingly, the present invention concerns compositions and methods for promoting or inhibiting angiogenesis and/or cardiovascularization in mammals. Described herein are cDNA clones that encode polypeptides designated in the present application as "PRO364" and "PRO175". These proteins tested positive in various cardiovascular assays that test promotion or inhibition of certain biological activities- Accordingly, the proteins are believed to be useful drugs for the diagnosis and/or treatment (including prevention) of disorders where such effects are desired, such as the promotion or inhibition of angiogenesis, inhibition of growth or proliferation of vascular endothelial cells, inhibition of tumor growth, and inhibition of angiogenesis-dependent tissue growth.

In one embodiment, the present invention concerns a composition comprising a PRO364 (hGITR) polypeptide in admixture with a pharmaceutically acceptable carrier and a cardiovascular, endothelial or angiogenic agent or angiostatic agent. In another embodiment, the present invention provides a composition comprising a PRO175 (hGITRL or GLITTER) polypeptide in admixture with a pharmaceutically acceptable carrier. In one aspect, the composition comprises a therapeutically effective amount of either or both polypeptides. In another aspect, the composition comprises a further active ingredient, namely, a cardiovascular, endothelial, or angiogenic agent or an angiostatic agent, preferably an angiogenic or angiostatic agent Preferably, the composition is sterile. PRO364 or PRO175 polypeptide may be administered in the form of a liquid pharmaceutical formulation, which may be preserved to achieve extended storage stability, Preserved liquid pharmaceutical formulations might contain multiple doses of PRO364 or PRO175 polypeptide, and might, therefore, be suitable for repeated use.

In a further embodiment, the invention supplies a method for preparing such composition for the treatment of a cardiovascular, endothelial, and angiogenic disorder comprising admixing a therapeutically effective amount of PRO364 or PRO175 polypeptide with the carrier.

In a still further aspect, the invention provides a pharmaceutical product comprising:
(a) such composition comprising a therapeutically effective dosage PRO364 polypeptide in admixture with a cardiovascular, endothelial or angiogenic agent or an angiostatic agent, or PRO175 polypeptide:
(b) a container containing said composition: and
(c) a label affixed to said container, or a package insert included in said pharmaceutical product referring to the use of said PRO364 or PRO175 polypeptide in the treatment of a cardiovascular, endothelial, and angiogenic disorder.

In a still further aspect, the invention provides a process for diagnosing a disease or a susceptibility to a disease related to a mutation in PR0364 or PRO175 polypeptide nucleic acid sequence comprising:
(a) isolating a nucleic acid sequence encoding PRO364 or PRO175 polypeptide from a sample derived from a host; and
(b) determining a mutation in the PRO364 or PRO175 polypeptide nucleic acid sequence.

In a still further aspect, the invention provides a diagnostic process comprising analyzing for the presence of PRO364 or PPO175 polypeptide in a sample derived from a host.

In yet another embodiment, the present invention concerns a method of diagnosing cardiovascular, endothelial, and angiogenic disorders in a mammal comprising detecting the level of expression of a gene encoding a PRO364 or PRO175 polypeptide (a) in a test sample of tissue cells obtained from the mammal, and (b) in a control sample of known normal tissue cells of the same cell type, wherein a higher or lower expression level in the test sample indicates the presence of a cardiovascular, endothelial, and angiogenic dysfunction in the mammal from which the test tissue cells were obtained.

In yet another embodiment, the invention concerns use of a PRO364 or PRO175 polypeptide for the preparation of a medicament for the treatment of a cardiovascular or endothelial or angiogenic disorder in a mammal. Preferably, the disorder is cardiac hypertrophy, trauma such as wounds or burns, or a type of cancer. In a further aspect, the mammal is further exposed to angioplasty or a drug that treats cardiovascular, endothelial, and angiogenic disorders such as ACE inhibitors or chemotherapeutic agents if the cardiovascular, endothelial, and angiogenic disorder is a type of cancer. Preferably, the mammal is human, preferably one who is at risk of developing cardiac hypertrophy and more preferably has suffered myocardial infarction.

In another preferred aspect, the cardiac hypertrophy is characterized by the presence of an elevated level of PGF_{2α}. Alternatively, the cardiac hypertrophy may be induced by myocardial infarction, wherein preferably the administration of PRO364 or PRO175 polypeptide is initiated within 48 hours, more preferably within 24 hours, following myocardial infarction.

In another preferred embodiment, the cardiovascular, endothelial, and angiogenic disorder is cardiac hypertrophy and said PRO364 or PRO175 polypeptide is administered together with a cardiovascular, endothelial, or angiogenic agent. The preferred cardiovascular, endothelial, or angiogenic agent for this purpose is selected from the group consisting of an antihypenensive drug, an ACE inhibitor, an endothelin receptor antagonist, and a thrombolytic agent. If a thrombolytic agent is administered, preferably the PRO364 or PRO175 polypeptide is administered following administration of such agent. More preferably, the thrombolytic agent is recombinant human tissue plasminogen activator.

In another preferred aspect, the cardiovascular, endotheliaL and angiogenic disorder is cardiac hypertrophy and the PRO364 or PRO175 polypeptide is administered following primary angioplasty for the treatment of acute myocardial infarction, preferably wherein the mammal is further exposed to angioplasty or a cardiovascular, endothelial, or angiogenic agent.

In another preferred embodiment the cardiovascular, endothelial and angiogenic disorder is a cancer and the PRO364 or PRO175 polypeptide is administered in combination with a chemotherapeutic agent, a growth inhibitory agent, or a cytotoxic agent.

In another embodiment, the invention furnishes a process for identifying agonists to a PRO364 or PRO175 polypeptide comprising:
(a) contacting cells and a compound to be screened under conditions suitable for the stimulation of cell proliferation by PRO364 or PRO175; and
(b) measuring the proliferation of the cells to determine if the compound is an effective agonist.

In another embodiment, the invention provides a method for identifying a compound that inhibits the expression or activity of a PRO364 or PRO175 polypeptide comprising contacting a candidate compound with a PRO364 or PRO175 polypeptide under conditions and for a time sufficient to allow the compound and polypeptide to interact. In a specific preferred aspect, either the candidate compound or the PR0364 or PRO175 polypeptide is immobilized on a solid support. In another preferred aspect, the non-immobilized component carries a detectable label. Preferably, this process comprises the steps of:
(a) contacting cells and a compound to be screened in the presence of PRO364 or PRO175 polypeptide under conditions suitable for the stimulation of cell proliferation by PRO364 or PRO175 polypeptide; and
(b) measuring the proliferation of the cells to determine if the compound is an effective antagonist.

Also described herein is use of an antagonist to a PRO364 or PRO175 polypeptide for the preparation of a medicament for the treatment of cardiovascular or endothelial or angiogenic disorder in a mammal. Preferably, the cardiovascular, endothelial, and angiogenic disorder is cardiac hypertrophy, trauma, a cancer, or age-related macular degeneration. Also preferred is where the mammal is human, and where an effective amount of an angiogenic or angiostatic agent is administered in conjunction with the antagonist.

One type of antagonist of a PRO364 or PRO175 polypeptide that inhibits one or more of the functions or activities of the PRO364 or PRO175 polypeptide is an antibody. Also described herein is an isolated antibody that binds a PRO364 or PRO175 polypeptide.

The antibody may be a monoclonal antibody, which preferably has rion-human complementarity-determining-region (CDR) residues and human framework-region (FR) residues. The antibody may be labeled and may be immobilized on a solid support. The antibody may be an antibody fragment, a single-chain antibody, or an anti-idiotypic antibody.

In another embodiment, the invention provides a method for determining the presence of a PRO364 or PRO175 polypeptide comprising exposing a cell suspected of containing the PRO364 or PRO175 polypeptide to an anti- PR0364 or -PR0175 antibody and determining binding of said antibody to said cell.

In a still further embodiment, the invention provides a method of diagnosing cardiovascular, endothelial, and angiogenic disorders in a mammal comprising (a) contacting an anti-PRO364 or -PRO175 antibody with a test sample of tissue cells obtained from the mammal, and (b) detecting the formation of a complex between the anti-PRO364 or PRO175 antibody and the PR0364 or PRO175 polypeptide in the test sample. The detection may be qualitative or quantitative, and may be performed in comparison with monitoring the complex formation in a control sample of known normal tissue cells of the same cell type. A larger or smaller quantity of complexes formed in the test sample indicates the presence of a cardiovascular, endothelial, and angiogenic dysfunction in the mammal from which the test tissue cells were obtained. The antibody preferably carries a detectable label. Complex formation can be monitored, for example, by light microscopy, flow cytometry, fluorimetry, or other techniques known in the art. The test sample is usually obtained from an individual suspected to have a cardiovascular, endothelial, and angiogenic disorder.

In further aspects, the invention provides a cancer diagnostic kit comprising an anti-PRO364 or -PRO175 antibody and a carrier in suitable packaging. Preferably, such kit further comprises instructions for using said antibody to detect the PRO364 or PRO175 polypeptide. Preferably, the carrier is a buffer, for example. In a further embodiment, the invention provides an article of manufacture, comprising:
a container;
a a label on the container, and
a composition comprising an anti- PRO364 or -PRO175 antibody contained within the container, wherein the label on the container indicates that the composition can be used for treating cardiovascular, endothelial, and angiogenic disorders.

In yet another embodiment, the invention provides use of an antibody that binds a PRO364 or PRO175 polypeptide for the preparation of a medicament inhibiting angiogenesis induced by PR0364 or PRO175 polypeptide in a mammal. Preferably, the mammal is a human, and more preferably the mammal has a tumor or a retinal disorder.

### BRIEF DESCRIPTION OF THE DRAWINGS

Figure I shows a nucleotide sequence (SEQ ID NO:1) containing the nucleotide sequence (SEQ ID NO:2) of a native sequence PRO364 cDNA (nucleotides 121-843), wherein the nucleotide sequence (SEQ ID NO:1) is a clone designated herein as "UNQ319" and/or "DNA47365-1206". Also presented is the position of the initiator methionine residue as well as the position of three oligonucleotide primers designated "47365.tm.f", "47365.tm.p" and "47365.tm.r" as underlined. The putative transmembrane domain of the protein is encoded by nucleotides 604-660 in the figure.
Figure 2 shows the PRO 364 amino acid sequence (SEQ ID NO:3) derived from nucleotides 121-843 of the nucleotide sequence shown in Figure I (SEQ ID NO:2). A potential transmembrane domain exists between and including amino acids 162 to 180 in the figure.
Figures 3A-C show a consensus nucleotide sequence (SEQ ID NO:4) designated "<consen01>".
Figure 4 shows the "<consen01>" consensus nucleotide sequence shown in Figures 3A-C designated in the present application as DNA44825 (SEQ ID NO:4). Also presented is the position of the oligonucleotide primers designated "44825.GITR.f" (SEQ ID NO:5), "44825.f1" (SEQ ID NO:6), "44825.GITR.p" (SEQ ID NO:7), "44825.r2" (SEQ ID NO:8), "44825.pl" (SEQ ID NO:9), "44825.GITR.r" (SEQ ID NO:10), "44825.f2" (SEQ ID NO:11), and "44825.r1" (SEQ ID NO:12) as underlined.
Figures 5A-B show the encoding nucleotide sequence (SEQ ID NO:13) and deduced amino acid sequence (SEQ ID NO:14) of a cDNA clone designated herein as PRO175-1150.
Figure 6 illustrates the relative mRNA expression of PRO364 in various human cells and tissues, as determined by quantitative reverse-transcriptase PCR.
Figure 7 illustrates the relative mRNA expression of PR0364 in primary human T cells and monocytes (treated with anti-CD3 antibody, PHA or LPS), as determined by quantitative reverse-transcriptase PCR.
Figure 8 shows a Northern blot analysis of PRO175 mRNA expression in human tissues (identified adult and fetal tissues) and tumor cell lines (HL60 promyelocytic leukemia, HeLa S3 cervical carcinoma, K562 chronic myelogenous leukemima, MOLT4 lymphoblastic leukemia, Raji Burkitt's lymphoma, SW480 colorectal adenocarcinoma, A549 lung carcinoma and G361 melanoma).
Figure 9 shows an analysis of soluble PRO175 polypeptide by SDS-PAGE.

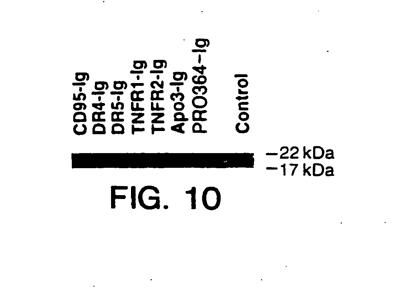

Figure 10 shows the results of a co-precipitation assay described in Example 6 below. The autoradiograph of the SDS-PAGE gel revealed the PRO364-IgG molecule bound to the radioiodinated PRO175 polypeptide. Binding was not observed for the other immunoadhesin constructs identified.
Figure 11A shows the results of FACS analysis of transfected 293 cells assayed for binding to the identified receptors or ligand immunoadhesin constructs.
Figure 11B shows the results of FACS analysis of HUVEC cells assayed for binding to the identified immunoadhesin constructs.
Figure 12 is a histogram showing the effect of PRO175. ligand on c-fos expression in human pericytes.

### Detailed Description of the Invention

### I. Definitions

The phrases "cardiovascular, endothelial, and angiogenic disorder" and "cardiovascular, endothelial, and angiogenic dysfunction" are used interchangeably and refer to systemic disorders that affect vessels, such as diabetes mellitus, as well as diseases of the vessels themselves, such as of the arteries, capillaries, veins and/or lymphatics. This would include indications that stimulate angiogenesis and/or cardiovascularization, and those that inhibit angiogenesis and/or cardiovascularization. Such disorders include, for example, arterial disease, such as atherosclerosis, hypertension, inflammatory vasculitides, Reynaud's disease and Reynaud's phenomenon, aneurysms, and arterial restenosis; venous and lymphatic disorders such as thrombophlebitis, lymphangitis, and lymphedema: and other vascular disorders such as peripheral vascular disease, cancer such as vascular tumors. *e.g.*, hemangioma (capillary and cavernous), glomus tumors, telangiectasia, bacillary angiomatosis, hemangioendothelioma, angiosarcoma, haemangiopericytoma, Kaposi's sarcoma, lymphangioma, and lymphangiosarcoma, rumor angiogenesis, trauma such as wounds, burns, and other injured tissue, implant fixation, scarring, ischemia reperfusion injury, rheumatoid arthritis, cerebrovascular disease, renal diseases such as acute renal failure, and osteoporosis. This would also include angina, myocardial infarctions such as acute myocardial infarctions, cardiac hypertrophy, and heart failure such as CHF.

"Hypertrophy", as used herein, is defined as an increase in mass of an organ or structure independent of natural growth that does not involve tumor formation. Hypertrophy of an organ or tissue is due either to an increase in the mass of the individual cells (true hypertrophy), or to an increase in the number of cells making up the tissue (hyperplasia), or both. Certain organs, such as the heart, lose the ability to divide shortly after birth. Accordingly, "cardiac hypertrophy" is defined as an increase in mass of the heart, which, in adults, is characterized by an increase in myocyte cell size and contractile protein content without concomitant cell division. The character of the stress responsible for inciting the hypertrophy, (*e.g.*, increased preload, increased afterload, loss of myocytes, as in myocardial infarction, or primary depression of contractility), appears to play a critical role in determining the nature of the response. The early stage of cardiac hypertrophy is usually characterized morphologically by increases in the size of mycrofibrils and mitochondria, as well as by enlargement of mitochondria and nuclei. At this stage, while muscle cells are larger than normal, cellular organization is largely preserved. At a more advanced stage of cardiac hypertrophy, there are preferential increases in the size or number of specific organelles, such as mitochondria, and new contractile elements are added in localized areas of the cells, in an irregular manner. Cells subjected to long-standing hypertrophy show more obvious disruptions in cellular organization, including markedly enlarged nuclei with highly lobulated membranes, which displace adjacent myofibrils and cause breakdown of normal Z-band registration. The phrase "cardiac hypertrophy" is used to include all stages of the progression of this condition, characterized by various degrees of structural damage of the heart muscle, regardless of the underlying cardiac disorder. Hence, the term also includes physiological conditions instrumental in the development of cardiac hypertrophy, such as elevated blood pressure, aortic stenosis, or myocardial infarction.

"Heart failure" refers to an abnormality of cardiac function where the heart does not pump blood at the rate needed for the requirements of metabolizing tissues. The heart failure can be caused by a number of factors, including ischemic, congenital, rheumatic, or idiopathic forms.

"Congestive heart failure" (CHF) is a progressive pathologic state where the heart is increasingly unable to supply adequate cardiac output (the volume of blood pumped by the heart over time) to deliver the oxygenated blood to peripheral tissues. As CHF progresses, structural and hemodynamic damages occur. While these damages have a variety of manifestations, one characteristic symptom is ventricular hypertrophy. CHF is a common end result of a number of various cardiac disorders.

"Myocardial infarction" generally results from atherosclerosis of the coronary arteries, often with superimposed coronary thrombosis. It may be divided into two major types: transmural infarcts, in which myocardial necrosis involves the full thickness of the ventricular wall, and subendocardial (nontransmural) infarcts, in which the necrosis involves the subendocardium, the intramural myocardium, or both, without extending all the way through the ventricular wall to the epicardium. Myocardial infarction is known to cause both a change in hemodynamic effects and an alteration in structure in the damaged and healthy zones of the heart. Thus, for example, myocardial infarction reduces the maximum cardiac output and the stroke volume of the heart. Also associated with myocardial infarction is a stimulation of the DNA synthesis occurring in the interstice as well as an increase in the formation of collagen in the areas of the heart not affected.

As a result of the increased stress or strain placed on the heart in prolonged hypertension due, for example, to the increased total peripheral resistance, cardiac hypertrophy has long been associated with "hypertension". A characteristic of the ventricle that becomes hypertrophic as a result of chronic pressure overload is an impaired diastolic performance. Fouad et al., J. Am. Coll. Cardiol., 4: 1500-1506 (1984); Smith et al., J. Am. Coll. Cardiol., 5: 869-874 (1985). A prolonged left ventricular relaxation has been detected in early essential hypertension, in spite of normal or supranormal systolic function. Hartford et al., Hypertension, 6: 329-338 (1984). However, there is no close parallelism between blood pressure levels and cardiac hypertrophy. Although improvement in left ventricular function in response to antihypertensive therapy has been reported in humans, patients variously treated with a diuretic (hydrochlorothiazide), a β-blocker (propranolol), or a calcium channel blocker (diltiazem), have shown reversal of left ventricular hypertrophy, without improvement in diastolic function. Inouye et al., Am. J. Cardiol., 53: 1583-7 (1984).

Another complex cardiac disease associated with cardiac hypertrophy is "hypertrophic cardiomyopathy". This condition is characterized by a great diversity of morphologic, functional, and clinical features (Maron et al., N. Engl. J. Med.; 316: 780-789 (1987); Spirito et al., N. Engl. J. Med., 320: 749-755 (1989); Louie and Edwards, Prog. Cardiovasc. Dis., 36: 275-308 (1994); Wigle et al., Circulation, 92: 1680-1692 (1995)), the heterogeneity of which is accentuated by the fact that it afflicts patients of all ages. Spirito et al., N. Engl. J. Med., 336: 775-785 (1997). The causative factors of hypertrophic cardiomyopathy are also diverse and little understood. In general, mutations in genes encoding sarcomeric proteins are associated with hypertrophic cardiomyopathy. Recent data suggest that β-myosin heavy chain mutations may account for approximately 30 to 40 percent of cases of familial hypertrophic cardiomyopathy. Watkins et al., N. Engl. J. Med., 326: 1108-1114 (1992); Schwartz et al, Circulation, 91: 532-540 (1995); Marian and Roberts, Circulation, 92: 1336-1347 (1995); Thierfelder et al., Cell, 77: 701-712 (1994); Watkins et al., Nat. Gen., 11: 434-437 (1995). Besides β-myosin heavy chain, other locations of genetic mutations include cardiac troponin T, alpha topomyosin, cardiac myosin binding protein C, essential myosin light chain, and regulatory myosin light chain. See Malik and Watkins, Curr. Opin. Cardiol., 12: 295-302 (1997).

Supravalvular "aortic stenosis" is an inherited vascular disorder characterized by narrowing of the ascending aorta, but other arteries, including the pulmonary arteries, may also be affected. Untreated aortic stenosis may lead to increased intracardiac pressure resulting in myocardial hypertrophy and eventually heart failure and death. The pathogenesis of this disorder is not fully understood, but hypertrophy and possibly hyperplasia of medial smooth muscle are prominent features of this disorder. It has been reported that molecular variants of the elastin gene are involved in the development and pathogenesis of aortic stenosis. U.S. Patent No. 5,650,282 issued July 22, 1997.

"Valvular regurgitation" occurs as a result of heart diseases resulting in disorders of the cardiac valves. Various diseases, like rheumatic fever, can cause the shrinking or pulling apart of the valve orifice, while other diseases may result in endocarditis, an inflammation of the endocardium or lining membrane of the atrioventricular orifices and operation of the heart. Defects such as the narrowing of the valve stenosis or the defective closing of the valve result in an accumulation of blood in the heart cavity or regurgitation of blood past the valve. If uncorrected, prolonged valvular stenosis or insufficiency may result in cardiac hypertrophy and associated damage to the heart muscle, which may eventually necessitate valve replacement.

The treatment of all these, and other cardiovascular, endothelial, and angiogenic disorders, which may or may not be accompanied by cardiac hypertrophy, is encompassed by the present invention.

The terms "cancer", "cancerous", and "malignant" refer to or describe the physiological condition in mammals that is typically characterized by unregulated cell growth. Examples of cancer include but are not limited to carcinoma including adenocarcinoma, lymphoma, blastoma, melanoma, sarcoma, and leukemia. More particular examples of such cancers include squamous cell cancer, small-cell lung cancer, non-small cell lung cancer, gastrointestinal cancer, Hodgkin's and non-Hodekin's lymphoma, pancreatic cancer, glioblastoma, cervical cancer, ovarian cancer, liver cancer such as hepatic carcinoma and hepatoma, bladder cancer, breast cancer, colon cancer, colorectal cancer, endometrial- carcinoma, salivary gland carcinoma; kidney cancer such as renal cell carcinoma and Wilms' tumors, basal cell carcinoma, melanoma, prostate cancer, vulval cancer, thyroid cancer, testicular cancer, esophageal cancer, and various types of head and neck cancer. The preferred cancers for treatment herein are breast, colon, lung, melanoma, ovarian, and others involving vascular tumors as noted above.

The term "cytotoxic agent" as used herein refers to a substance that inhibits or prevents the function of cells and/or causes destruction of cells. The term is intended to include radioactive isotopes (*e*.*g*., ¹³¹I, ¹²⁵I, ⁹⁰Y, and ¹³⁶Re), chemotherapeutic agents, and toxins such as enzymatically active toxins of bacterial, fungal, plant, or animal origin, or fragments thereof.

A "chemotherapeutic agent" is a chemical compound useful in the treatment of cancer. Examples of chemotherapeutic agents include alkylating agents, folic acid antagonists, anti-metabolites of nucleic acid metabolism, antibiotics, pyrimidine analogs, 5-fluorouracil, cisplatin, purine nucleosides, amines, amino acids, triazol nucleosides, or corticosteroids. Specific examples include Adriamycin, Doxorubicin, 5-Fluorouracil, Cytosine arabinoside ("Ara-C"), Cyclophosphamide, Thiotepa, Busulfan, Cytoxin, Taxol, Toxotere, Methotrexate, Cisplatin, Melphalan, Vinblastine, Bleomycin, Etoposide, Ifosfamide, Mitomycin C, Mitoxantrone, Vincreistine, Vinorelbine, Carboplatin, Teniposide, Daunomycin, Carminomycin, Aminopterin, Dactinomycin, Mitomycins, Esperamicins (see U.S. Pat. No. 4,675,187), Melphalan, and other related nitrogen mustards. Also included in this definition are hormonal agents that act to regulate or inhibit hormone action on tumors, such as tamoxifen and onapristone.

A "growth-inhibitory agent" when used herein refers to a compound or composition that inhibits growth of a cell, such as an Wnt-overexpressing cancer cell, either *in vitro* or *in vivo*. Thus, the growth-inhibitory agent is one which significantly reduces the percentage of malignant cells in S phase. Examples of growth-inhibitory agents include agents that block cell cycle progression (at a place other than S phase), such as agents that induce G1 arrest and M-phase arrest. Classical M-phase blockers include the vincas (vincristine and vinblastine), taxol, and topo II inhibitors such as doxorubicin, daunorubicin, etoposide, and bleomycin. Those agents that arrest G1 also spill over into S-phase arrest, for example, DNA alkylating agents such as tamoxifen, prednisone, dacarbazine, mechlorethamine, cisplatin, methotrexate. 5-fluorouracil, and ara-C. Further information can be found in The Molecular Basis of Cancer, Mendelsohn and Israel, eds., Chapter 1, entitled "Cell cycle regulation, oncogenes, and antineoplastic drugs" by Murakami et al. (WB Saunders: Philadelphia, 1995), especially p. 13. Additional examples include tumor necrosis factor (TNF), an antibody capable of inhibiting or neutralizing the angiogenic activity of acidic or basic FGF or hepatocyte growth factor (HGF), an antibody capable of inhibiting or neutralizing the coagulant activities of tissue factor, protein C, or protein S (see WO 91/01753. published 21 February 1991), or an antibody capable of binding to HER2 receptor (WO 89/06692), such as the 4D5 antibody (and functional equivalents thereof) (*e.g*., WO 92/22653).

"Treatment" is an intervention performed with the intention of preventing the development or altering the pathology of a cardiovascular, endothelial, and angiogenic disorder. The concept of treatment is used in the broadest sense, and specifically includes the prevention (prophylaxis), moderation, reduction, and curing of cardiovascular, endothelial, and angiogenic disorders of any stage. Accordingly, "treatment" refers to both therapeutic treatment and prophylactic or preventative measures, wherein the object is to prevent or slow down (lessen) a cardiovascular, endothelial, and angiogenic disorder such as hypertrophy. Those in need of treatment include those already with the disorder as well as those prone to have the disorder or those in whom the disorder is to be prevented. The disorder may result from any cause, including idiopathic, cardiotrophic, or myotrophic causes, or ischemia or ischemic insults, such as myocardial infarction.

"Chronic" administration refers to administration of the agent(s) in a continuous mode as opposed to an acute mode, so as to maintain the initial effect, such as an anti-hypertrophic effect, for an extended period of time.

"Mammal" for purposes of treatment refers to any animal classified as a mammal, including humans, domestic and farm animals, and zoo; sports, or pet animals; such as dogs, horses, cats, cows, sheep, pigs, etc. Preferably, the mammal is human.

Administration "in combination with" one or more further therapeutic agents includes simultaneous (concurrent) and consecutive administration in any order.

The phrase "cardiovascular, endothelial, or angiogenic agents" refers generically to any drug that acts in treating cardiovascular, endothelial, and angiogenic disorders. Examples or cardiovascular agents are those that promote vascular homeostasis by modulating blood pressure, heart rate, heart contractility, and endothelial and smooth muscle biology, all of which factors have a role in cardiovascular disease. Specific examples of these include angiotensin-II receptor antagonists; endothelin receptor antagonists such as, for example,-BOSENTAN^{™} and MOXONODIN^{™}; interferon-gamma (IFN-γ); des-aspartate-angiotensin I; thrombolytic agents, *e.g.,* streptokinase, urokinase, t-PA, and a t-PA variant specifically designed to have longer half-life and very high fibrin specificity, TNK t-PA (a T103N, N117Q, KHRR(296-299)AAAA t-PA variant, Keyt et al., Proc. Natl. Acad. Sci. USA 91, 3670-3674 (1994)); inotropic or hypertensive agents such as digoxigenin and β-adrenergic receptor blocking agents, *e.g*., propranolol, timolol, tertalolol, carteolol, nadolol, betaxolol, penbutolol, acetobutolol, atenolol, metoprolol, and carvedilol; angiotensin converting enzyme (ACE) inhibitors, e.g., quinapril, captopril, enalapril, ramipril, benazepril, fosinopril, and lisinopril; diuretics, *e.g*., chorothiazide, hydrochlorothiazide, hydroflumethazide, methylchlothiazide, benzthiazide, dichlorphenamide, acetazolamide, and indapamide; and calcium channel blockers, *e.g*., diltiazem, nifedipine, verapamil, nicardipine. One preferred category of this type is a therapeutic agent used for the treatment of cardiac hypertrophy or of a physiological condition instrumental in the development of cardiac hypertrophy, such as elevated blood pressure, aortic stenosis, or myocardial infarction.

"Angiogenic agents" and "endothelial agents" are active agents that promote angiogenesis and/or endothelial cell growth, or, if applicable, vasculogenesis. This would include factors that accelerate wound healing, such as growth hormone, insulin-like growth factor-I (IGF-I), VEGF, VIGF, PDGF, epidermal growth factor (EGF), CTGF and members of its family, FGF, and TGF-α and TGF-β.

"Angiostatic agents" are active agents that inhibit angiogenesis or vasculogenesis or otherwise inhibit or prevent growth of cancer cells. Examples include antibodies or other antagonists to angiogenic agents as defined above, such as antibodies to VEGF. They additionally include cytotherapeutic agents such as cytotoxic agents, chemotherapeutic agents, growth-inhibitory agents, apoptotic agents, and other agents to treat cancer, such as anti-HER-2, anti-CD20; and other bioactive and organic chemical agents.

In a pharmacological sense, in the context of the present invention, a "therapeutically effective amount" of an active agent such as a PRO364 or PRO175 polypeptide or antagonist thereto, refers to an amount effective in the treatment of a cardiovascular, endothelial, and angiogenic disorder.

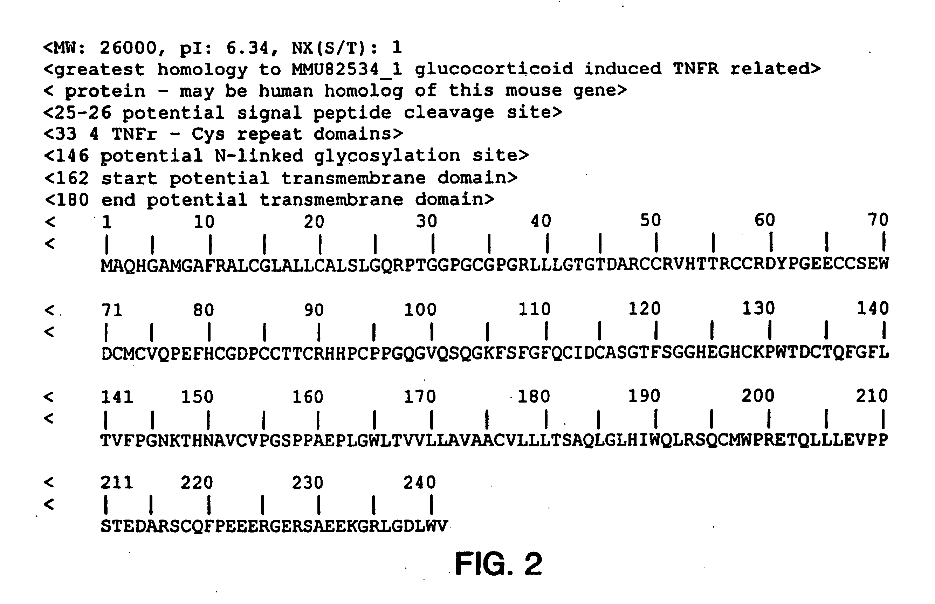

As used herein, a "PRO364 polypeptide" is used to refer to a native-sequence PRO364 polypeptide having the same amino acid sequence as a PRO364 (SEQ ID NO:3) polypeptide derived from nature: Such native sequence PR0364 polypeptide can be isolated from nature or can be produced by recombinant or synthetic means. The term specifically encompasses naturally-occurring truncated or secreted forms of a PR0364 (*e.g.*, soluble forms containing for instance, an extracellular domain sequence), naturally-occurring variant forms (*e*.*g*., alternatively spliced forms) and naturally-occurring allelic variants of the PRO364 polypeptide. The terms "PR0364 polypeptide" and "PRO364" when used herein refer to the same polypeptides referred to in the literature as "GITR". In one embodiment of the invention, the native sequence PRO364 polypeptide is a mature or full-length native sequence PRO364 polypeptide comprising amino acids 1 to 241 of Figure I (SEQ ID NO:3). Additional embodiments are directed to PRO364 polypeptide comprising amino acids 26-241 of Figure 2 (SEQ ID NO:3). In yet another embodiment of the invention, the native sequence PRO364 polypeptide is an extracellular domain sequence of the full-length PRO364 protein, wherein the putative transmembrane domain of the full-length PRO364 protein includes amino acids 162-180 of the sequence shown in Figure 2 (SEQ ID NO:3). Alternatively, the PRO364 polypeptide is obtained or obtainable by expressing the polypeptide encoded by the cDNA insert of the vector DNA47365-1206 deposited as ATCC 209436. Thus, additional embodiments of the present invention are directed to polypeptides comprising amino acids 1-161 or 26-161 of the amino acid sequence shown in Figure 2 (SEQ ID NO:3).

The term " extracellular domain" when used in reference to a PRO364 polypeptide refers to a form of the PRO364 polypeptide which is essentially free of its transmembrane and cytoplasmic domains. Ordinarily, an ECD will have less than 1% of such transmembrane and/or cytoplasmic domains and preferably, will have less than 0.5% of such domains. Included are deletion variants or fragments of the full length or ECD in which one or more amino acids are deleted from the N- or C- terminus. Preferably, such deletion variants or fragments possess a desired activity, such as described herein. It will be understood that any transmembrane domain identified for the PRO364 polypeptide of the present invention is identified pursuant to criteria routinely employed in the art for identifying that type of hydrophobic domain. The exact boundaries of a transmembrane domain may vary but most likely by no more than about 5 amino acids at either end of the domain as initially identified. Accordingly, the PRO364 polypeptide ECD may optionally comprise amino acids Y to X of Figure 2 (SEQ ID NO:3), wherein Y is any one of amino acid residues I to 26 and X is any one of amino acid residues 157 to 167 of Figure 2 (SEQ ID NO:3).

The term "variant" when used in reference to a PRO364 polypeptide is defined below as having at least about 80% amino acid sequence identity with the PRO364 polypeptide having the deduced amino acid sequence shown in Figure 2 (SEQ ID NO:3) for a full-length native sequence PR0364 polypeptide or an extracellular domain sequence thereof. Such PR0364 polypeptide variants include, for instance, PR0364 polypeptides wherein one or more amino acid residues are added, or deleted, at the N- or C-terminus of the sequence of Figure 2 (SEQ ID NO:3). Ordinarily, a PRO364 polypeptide variant will have at least about 80% amino acid sequence identity, preferably at least about 85% amino acid sequence identity, more preferably at least about 90% amino acid sequence identity, even more preferably at least about 95% amino acid sequence identity and yet more preferably 98% amino acid sequence identity with the amino acid sequence of Figure 2 (SEQ ID NO:3)

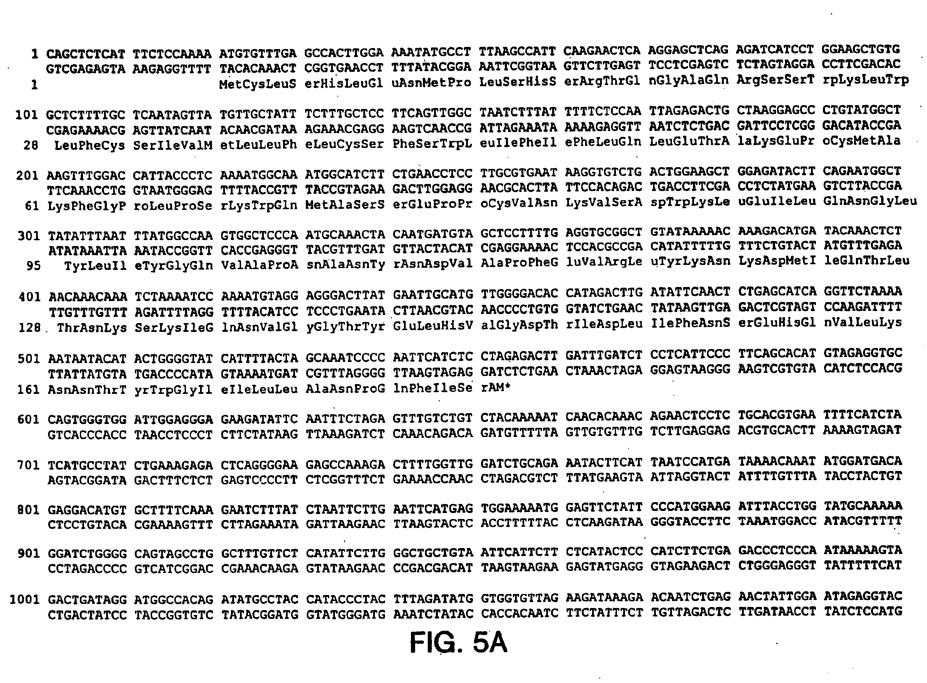

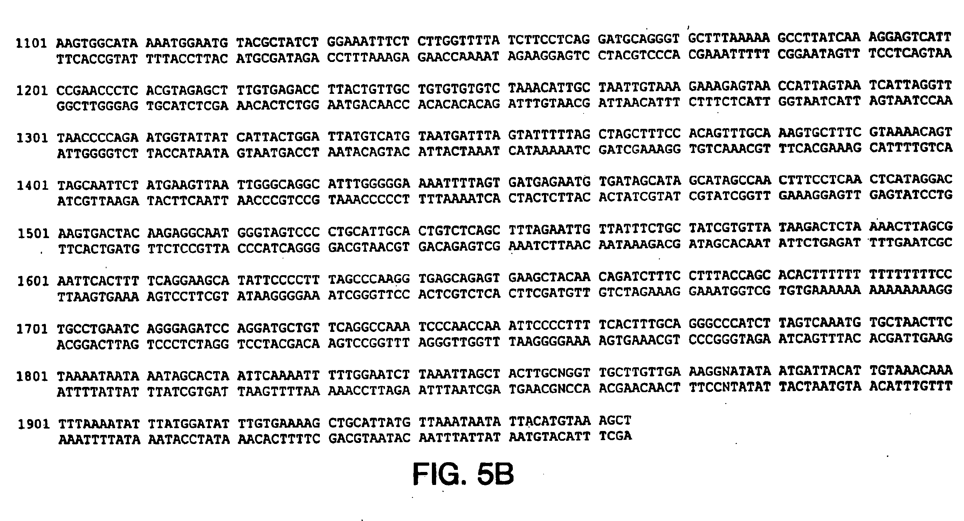

As used herein, a "PRO175 polypeptide" is used to refer to a native-sequence PRO175 polypeptide having the same amino acid sequence as a PRO175 (SEQ 10 NO:14) polypeptide derived from nature. Such native sequence PRO175 polypeptide can be isolated from nature or can be produced by recombinant or synthetic means. The term specifically encompasses naturally-occurring truncated or secreted forms of a PRO175 polypeptide (*e.g.,* soluble forms containing for instance, an extracellular domain sequence), naturally-occurring variant forms (*e.g.*, alternatively spliced forms) and naturally-occurring allelic variants of the PRO175 polypeptide. The terms "PRO175 polypeptide" and "PRO175" when used herein refer to the same polypeptides referred to in the literature as "GLITTER". In one embodiment of the invention, the native sequence polypeptide encoded by PRO175 is a mature or full-length native-sequence polypeptide comprising amino acids 1 to 177 of Figure 5 (SEQ ID NO:14). In yet another embodiment of the invention, the PRO175 polypeptide comprises amino acids 52 to 177 of Figure 5 (SEQ ID NO:14). Optionally the PRO175 polypeptide is obtained or obtainable by expressing the polypeptide encoded by the cDNA insert of the vector deposited as ATCC 209466. Thus, additional embodiments of the present invention are directed to polypeptides comprising amino acids I to 177 or 52 to 177 of Figure 5 (SEQ ID NO:14).

The term " extracellular domain" when used in reference to a PRO175 polypeptide refers to a form of the PRO175 polypeptide which is essentially free of its transmembrane and cytoplasmic domains. Ordinarily, an ECD will have less than 1% of such transmembrane and/or cytoplasmic domains and preferably, will have less than 0.5% of such domains. Included are deletion variants or fragments of the full length or ECD in which one or more amino acids are deleted from the N- or C- terminus. Preferably, such deletion variants or fragments possess a desired activity, such as described herein. It will be understood that any transmembrane domain identified for the PR0175 polypeptide of the present invention is identified pursuant to criteria routinely employed in the art for identifying that type of hydrophobic domain. The exact boundaries of a transmembrane domain may vary but most likely by no more than about 5 amino acids at either end of the domain as initially identified. Accordingly, the PRO175 polypeptide ECD will comprise amino acid residues X to 177 of Figure 5 (SEQ ID NO: 14), wherein X is any one of amino acid residues 48 to 57 of Figure 5 (SEQ ID NO: 14).

The term "variant" when used in reference to a PR0175 polypeptide is defined below as having at least about 80% amino acid sequence identity with the PRO175 polypeptide having the deduced amino acid sequence shown in Figure 5 (SEQ ID NO:14) for a full-length native sequence PRO175 polypeptide or an extracellular domain sequence thereof. A PRO175 polypeptide variant will include, for instance, a PRO175 polypeptide wherein one or more amino acid residues are added, or deleted, at the N- or C-terminus of the sequence of Figure 5 (SEQ ID NO:14). Ordinarily, a PRO175 polypeptide variant will have at least about 80% amino acid sequence identity, preferably at least about 85% amino acid sequence identity, more preferably at least about 90% amino acid sequence identity, even more preferably at least about 95% amino acid sequence identity and yet more preferably 98% amino acid sequence identity with the amino acid sequence of Figure 5 (SEQ ID NO: 14).

"Percent (%) amino acid sequence identity" with respect to the sequences identified herein is defined as the percentage of amino acid residues in a candidate sequence that are identical with the amino acid residues in the PRO 364 polypeptide sequence or the PRO175 polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art. One skilled in the an can determine appropriate parameters for measuring alignment, including assigning algorithms needed to achieve maximal alignment over the full-length sequences being compared. For purposes herein, percent amino acid identity values can be obtained using the sequence comparison computer program. ALIGN-2. which was authored by Genentech. Inc. and the source code of which has been filed with user documentation in the US Copyright Office, Washington, DC. 20559. registered under the US Copyright Registration No. TXU510087. The ALIGN-2 program is publicly available through Genentech, Inc., South San Francisco, CA. All sequence comparison parameters are set by the ALIGN-2 program and do not vary. Methods for performing sequence alignment and determining sequence identity are known to.the skilled artisan, may be performed without undue experimentation, and calculations of % identity values may be obtained with definiteness.

"Isolated," when used to describe the various polypeptides disclosed herein, means polypeptide that has been identified and separated and/or recovered from a component of its natural environment. Contaminant components of its natural environment are materials that would typically interfere with diagnostic or therapeutic uses for the polypeptide, and may include enzymes, hormones, and other proteinaceous or non-proteinaceous solutes. In preferred embodiments, the polypeptide will be purified (1) to a degree sufficient to obtain at least 15 residues of N-terminal or internal amino acid sequence by use of a spinning cup sequenator, or (2) to homogeneity by SDS-PAGE under non-reducing or reducing conditions using Coomassie blue or, preferably, silver stain. Isolated polypeptide includes polypeptide in *situ* within recombinant cells, since at least one component of the PR0364 or the PRO175 ligand polypeptide natural environment will not be present. Ordinarily, however, isolated polypeptide will be prepared by at least one purification step.

An "isolated" PR0364 or PRO175 nucleic acid molecule is a nucleic acid molecule that is identified and separated from at least one contaminant nucleic acid molecule with which it is ordinarily associated in the natural source of the PR0364 or PRO175 nucleic acid. An isolated PR0364 or PRO175 nucleic acid molecule is other than in the form or setting in which it is found in nature. Isolated PR0364 or PRO175 nucleic acid molecules therefore are distinguished from the PRO364 or PRO175 nucleic acid molecule as they exist in natural cells. However, an isolated PR0364 or PRO175 nucleic acid molecule includes PR0364 or PRO175 nucleic acid molecules contained in cells that ordinarily express PR0364 or PRO175 where, for example, the nucleic acid molecule is in a chromosomal location different from that of natural cells.

The term "control sequences" refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers.

Nucleic acid is "operably linked" when it is placed into a functional relationship with another nucleic acid sequence. For example, DNA for a presequence or secretory leader is operably linked to DNA for a PR0364 or PRO175 polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

"Stringency" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to reanneal when complementary strands are present in an environment below their melting temperature. The higher the degree of desired homology between the probe and hybridizable sequence, the higher the relative temperature that can be used. As a result, it follows that higher relative temperatures would tend to make the reaction conditions more stringent, while lower temperatures less so. For additional details and explanation of stringency of hybridization reactions, see Ausubel et al., Current Protocols in Molecular Biology (Wiley Interscience Publishers. 1995).

"Stringent conditions" or "high-stringency conditions", as defined herein, may be identified by those that: (1) employ low ionic strength and high temperature for washing, for example, 0.015 M sodium chloride/0.0015 M sodium citrate/0.1% sodium dodecyl sulfate at 50°C; (2) employ during hybridization a denaturing agent, such as formamide, for example, 50% (v/v) formamide with 0.1% bovine serum albumin/0.1% Ficoll/0.1% polyvinylpyrrolidone/50mM sodium phosphate buffer at pH 6.5 with 750 mM sodium chloride. 75 mM sodium citrate at 42°C; or (3) employ 50% formamide, 5 x SSC (0.75 M NaCl, 0.075 M sodium citrate), 50 mM sodium phosphate (pH 6.8), 0.1% sodium pyrophosphate, 5 x Denhardt's solution, sonicated salmon sperm DNA (50 µg/ml), 0.1% SDS, and 10% dextran sulfate at 42°C, with washes at 42°C in 0.2 x SSC (sodium chloride/sodium citrate) and 50% formamide at 55 °C, followed by a high-stringency wash consisting of 0.1 x SSC containing EDTA at 55°C.

"Moderately-stringent conditions" may be identified as described by Sambrook et al., Molecular Cloning: A Laboratory Manual (New York: Cold Spring Harbor Press, 1989), and include the use of washing solution and hybridization conditions (*e.g.*, temperature, ionic strength, and %SDS) less stringent than those described above. An example of moderately stringent conditions is overnight incubation at 37°C in a solution comprising: 20% formamide, 5 x SSC (150 mM NaCl, 15 mM trisodium citrate), 50 mM sodium phosphate (pH 7.6), 5 x Denhardt's solution, 10% dextran sulfate, and 20 mg/mL denatured sheared salmon sperm DNA, followed by washing the filters in 1 x SSC at about 37-50°C. The skilled artisan will recognize how to adjust the temperature, ionic strength, etc. as necessary to accommodate factors such as probe length and the like.

The modifier "epitope-tagged" when used herein refers to a chimeric polypeptide comprising a PR0364 or PRO175 polypeptide fused to a "tag polypeptide". The tag polypeptide has enough residues to provide an epitope against which an antibody can be made, yet is short enough such that it does not interfere with activity of the polypeptide to which it is fused. The tag polypeptide preferably also is fairly unique so that the antibody does not substantially cross-react with other epitopes. Suitable tag polypeptides generally have at least six amino acid residues and usually between about 8 and 50 amino acid residues (preferably, between about 10 and 20 amino acid residues).

"Active" or "activity" in the context of PRO364 or PRO175 variants refers to form(s) of PR0364 or PRO175 proteins that retain the biologic and/or immunologic activities of a native or naturally-occurring PR0364 or PRO175 polypeptide.

"Biological activity" in the context of a molecule that antagonizes PRO364 or PRO175 polypeptide that can be identified by the screening assays disclosed herein (*e.g*., an organic or inorganic small molecule, peptide, etc.) is used to refer to the ability of such molecules to bind or complex with the PRO364 or PRO175 polypeptide identified herein, or otherwise interfere with the interaction of the PR0364 or PRO175 polypeptides with other cellular proteins. Particularly preferred biological activity includes cardiac hypertrophy, activity that acts on systemic disorders that affect vessels, such as diabetes mellitus, as well as diseases of the arteries, capillaries, veins, and/or lymphatics, and cancer.

The term "antagonist" is used in the broadest sense, and includes any molecule that partially or fully blocks, inhibits, or neutralizes one or more of the biological activities of a native PR0364 or PRO175 polypeptide disclosed herein, for example, if applicable, its mitogenic or angiogenic activity. Antagonists of PR0364 or PRO175 polypeptide may act by interfering with the binding of PRO364 or PRO175 polypeptide to a cellular receptor, by incapacitating or killing cells that have been activated by PR0364 or PRO175 polypeptide, or by interfering with vascular endothelial cell activation after binding of PR0364 or PRO175 polypeptide to a cellular receptor. All such points of intervention by a PR0364 or PRO175 polypeptide antagonist shall be considered equivalent for purposes of this invention. The antagonists inhibit the mitogenic, angiogenic, or other biological activity of PR0364 or PRO175 polypeptides, and thus are useful for the treatment of diseases or disorders characterized by undesirable excessive neovascularization, including by way of example tumors, and especially solid malignant tumors, rheumatoid arthritis, psoriasis, atherosclerosis, diabetic and other retinopathies, retrolental fibroplasia, age-related macular degeneration, neovascular glaucoma, hemangiomas, thyroid hyperplasias (including Grave's disease), corneal and other tissue transplantation, and chronic inflammation. The antagonists also are useful for the treatment of diseases or disorders characterized by undesirable excessive vascular permeability, such as edema associated with brain tumors, ascites associated with malignancies, Meigs' syndrome, lung inflammation, nephrotic syndrome, pericardial effusion (such as that associated with pericarditis), and pleural effusion. In a similar manner, the term "agonist" is used in the broadest.sense and includes any molecule that mimics a biological activity of a native PR0364 or PRO175 polypeptide disclosed herein. Suitable agonist or antagonist molecules specifically include agonist or antagonist antibodies or antibody fragments, fragments, or amino acid sequence variants of native PRO364 or PRO175 polypeptides.

"Antibodies" (Abs) and "immunoglobulins" (Igs) are glycoproteins having the same structural characteristics. While antibodies exhibit binding specificity to a specific antigen, immunoglobulins include both antibodies and other antibody-like molecules that lack antigen specificity. Polypeptides of the latter kind are, for example, produced at low levels by the lymph system and at increased levels by myelomas. The term "antibody" is used in the broadest sense and specifically covers, without limitation, intact monoclonal antibodies, polyclonal antibodies, multispecific antibodies (*e.g.*, bispecific antibodies) formed from at least two intact antibodies, and antibody fragments, so long as they exhibit the desired biological activity.

"Native antibodies" and "native immunoglobulins" are usually heterotetrameric glycoproteins of about 150,000 daltons, composed of two identical light (L) chains and two identical heavy (H) chains. Each light chain is linked to a heavy chain by one covalent disulfide bond, while the number of disulfide linkages varies among . the heavy chains of different immunoglobulin isotypes. Each heavy and light chain also has regularly spaced intrachain disulfide bridges. Each heavy chain has at one end a variable domain (V_{H}) followed by a number of constant domains. Each light chain has a variable domain at one end (V_{L}) and a constant domain at its other end; the constant domain of the light chain is aligned with the first constant domain of the heavy chain, and the light-chain variable domain is aligned with the variable domain of the heavy chain. Particular amino acid residues are believed to form an interface between the light- and heavy-chain variable domains.

The term "variable" refers to the fact that certain portions of the variable domains differ extensively in sequence among antibodies and are used in the binding and specificity of each particular antibody to and for its particular antigen. However, the variability is not evenly distributed throughout the variable domains of antibodies. It is concentrated in three segments called complementarity-determining regions (CDRs) or hypervariable regions both in the light-chain and the heavy-chain variable domains. The more highly conserved portions of variable domains are called the framework regions (FR). The variable domains of native heavy and light chains each comprise four FR regions, largely adopting a β-sheet configuration, connected by three CDRs, which form loops connecting, and in some cases forming part of, the β-sheet structure. The CDRs in each chain are held together in close proximity by the FR regions and, with the CDRs from the other chain, contribute to the formation of the antigen-binding, site of antibodies. See Kabat et al., N1H Publ. No.91-3242. Vol. 1. pages 647-669 (1991). The constant domains are not involved directly in binding an antibody to an antigen, but exhibit various effector functions, such as participation of the antibody in antibody-dependent cellular toxicity.

"Antibody fragments" comprise a portion of an intact antibody, preferably the antigen-binding or variable region of the intact antibody. Examples of antibody fragments include Fab. Fab', F(ab')₂, and Fv fragments; diabodies; linear antibodies (Zapata et al., Protein Eng., 8(10): 1057-1062 (1995)); single-chain antibody molecules; and multispecific antibodies formed from antibody fragments.

Papain digestion of antibodies produces two identical antigen-binding fragments, called "Fab" fragments, each with a single antigen-binding site, and a residual "Fc" fragment, whose name reflects its ability to crystallize readily. Pepsin treatment yields an F(ab')₂ fragment that has two antigen-combining sites and is still capable of cross-linking antigen.

"Fv" is the minimum antibody fragment that contains a complete antigen-recognition and -binding site. This region consists of a dimer of one heavy- and one light-chain variable domain in tight, non-covalent association. It is in this configuration that the three CDRs of each variable domain interact to define an antigen-binding site on the surface of the V_{H}-V_{L} dimer. Collectively, the six CDRs confer antigen-binding specificity to the antibody. However, even a single variable domain (or half of an Fy comprising only three CDRs specific for an antigen) has the ability to recognize and bind antigen, although at a lower affinity than the entire binding site.

The Fab fragment also contains the constant domain of the light chain and the first constant domain (CH1) of the heavy chain. Fab' fragments differ from Fab fragments by the addition of a few residues at the carboxy terminus of the heavy chain CHl domain including one or more cysteines from the antibody hinge region. Fab'-SH is the designation herein for Fab' in which the cysteine residue(s) of the constant domains bear a free thiol group. F(ab')₂ antibody fragments originally were produced as pairs of Fab' fragments that have hinge cysteines between them. Other chemical couplings of antibody fragments are also known.

The "light chains" of antibodies (immunoglobulins) from any vertebrate species can be assigned to one of two clearly distinct types, called kappa (κ) and lambda (λ), based on the amino acid sequences of their constant domains.

Depending on the amino acid sequence of the constant domain of their heavy chains, immunoglobulins can be assigned to different classes. There are five major classes of immunoglobulins: IgA, IgD, IgE, IgG, and IgM; and several of these may be further divided into subclasses (isotypes), *e.g.*, IgG1, IgG2, IgG3, IgG4, IgA, and IgA2. The heavy-chain constant domains that correspond to the different classes of immunoglobulins are called α, δ, ε, γ, and µ, respectively. The subunit structures and three-dimensional configurations of different classes of immunoglobulins are well known.

The term "monoclonal antibody" as used herein refers to an antibody obtained from a population of substantially homogeneous antibodies, *i.e.*, the individual antibodies comprising the population are identical except for possible naturally-occurring mutations that may be present in minor amounts. Monoclonal antibodies are highly specific, being directed against a single antigenic site. Furthermore, in contrast to conventional (polyclonal) antibody preparations that typically include different antibodies directed against different determinants (epitopes), each monoclonal antibody is directed against a single determinant on the antigen. In addition to their specificity, the monoclonal antibodies are advantageous in that they are synthesized by the hybridoma culture, uncontaminated by other immunoglobulins. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler et al., Nature, 256: 495 (1975), or may be made by recombinant DNA methods (see, *e.g.,.* U.S. Patent No. 4,816,567). The "monoclonal antibodies" may also be isolated from phage antibody libraries using the techniques described in Clackson et al.. Nature, 352: 624-628 (1991) and Marks et al., J. Mol. Biol., 222: 581-597 (1991), for example.

The monoclonal antibodies herein specifically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity. U.S. Patent No. 4,816,567; Morrison et al., Proc. Natl. Acad. Sci. USA, 81: 6851-6855 (1984).

"Humanized" forms of non-human (*e.g.*, murine) antibodies are chimeric immunoglobulins, immunoglobulin chains, or fragments thereof (such as Fv, Fab, Fab', F(ab')_{2.} or other antigen-binding subsequences of antibodies) that contain minimal sequence derived from non-human immunoglobulin. For the most part, humanized antibodies are human immunoglobulins (recipient antibody) in which residues from a CDR of the recipient are replaced by residues from a CDR of a non-human species (donor antibody) such as mouse, rat or rabbit having the desired specificity, affinity, and capacity. In some instances, Fv FR residues of the human immunoglobulin are replaced by corresponding non-human residues. Furthermore, humanized antibodies may comprise residues that are found neither in the recipient antibody nor in the imported CDR or framework sequences. These modifications are made to further refine and maximize antibody performance. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin sequence. The humanized antibody preferably also will comprise at least a portion of an immunoglobulin constant region (Fc), typically that of a human immunoglobulin. For further details, see Jones et al., Nature, 321: 522-525 (1986); Reichmann et al., Nature, 332: 323-329 (1988); and Presta, Curr. Op. Struct. Biol., 2: 593-596 (1992). The humanized antibody includes a PRIMATIZED^{™} antibody wherein the antigen-binding region of the antibody is derived from an antibody produced by immunizing macaque monkeys with the antigen of interest.

"Single-chain Fv" or "sFv" antibody fragments comprise the V_{H} and V_{L} domains of an antibody, wherein these domains are present in a single polypeptide chain. Preferably, the Fv polypeptide further comprises a polypeptide linker between the V_{H} and V_{L} domains that enables the sFv to form the desired structure for antigen binding. For a review of sFv see Pluckthun in The Pharmacology of Monoclonal Antibodies, vol. 113, Rosenburg and Moore, eds. (Springer-Verlag: New York, 1994), pp. 269-315.

The term "diabodies" refers to small antibody fragments with two antigen-binding sites, which fragments comprise a heavy-chain variable domain (V_{H}) connected to a light-chain variable domain (V_{L}) in the same polypeptide chain (V_{H} - V_{L}). By using a linker that is too short to allow pairing between the two domains on the same chain, the domains are forced to pair with the complementary domains of another chain and create two antigen-binding sites. Diabodies are described more fully in, for example, EP 404.097: WO 93/1 1161; and Hollinger et al., Proc. Natl. Acad. Sci. USA, 90: 6444-6448 (1993).

An "isolated" antibody is one that has been identified and separated and/or recovered from a component of its natural environment. Contaminant components of its natural environment are materials that would interfere with diagnostic or therapeutic uses for the antibody, and may include enzymes, hormones, and other proteinaceous or nonproteinaceous solutes. In preferred embodiments, the antibody will be purified (1) to greater than 95% by weight of antibody as determined by the Lowry method, and most preferably more than 99% by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue or, preferably, silver stain. Isolated antibody includes the antibody *in situ* within recombinant cells, since at least one component of the antibody's natural environment will not be present. Ordinarily, however, isolated antibody will be prepared by at least one purification step.

The word "label" when used herein refers to a detectable compound or composition that is conjugated directly or indirectly to the antibody so as to generate a "labeled" antibody. The label may be detectable by itself (*e.g*., radioisotope labels or fluorescent labels) or, in the case of an enzymatic label, may catalyze chemical alteration of a substrate compound or composition that is detectable.

By "solid phase" is meant a non-aqueous matrix to which an antibody of the present invention can adhere. Examples of solid phases encompassed herein include those formed partially or entirely of glass (*e.g.,* controlled pore glass), polysaccharides (*e.g*., agarose), polyacrylamides, polystyrene, polyvinyl alcohol and silicones. In certain embodiments, depending on the context, the solid phase can comprise the well of an assay plate; in others it is a purification column (*e.g.,* an affinity chromatography column). This term also includes a discontinuous solid phase of discrete particles, such as those described in U.S. Patent No. 4,275,149.

A "liposome" is a small vesicle composed of various types of lipids, phospholipids and/or surfactant that is useful for delivery of a drug (such as the PR0364 or PRO175 polypeptide or antibodies thereto disclosed herein) to a mammal. The components of the liposome are commonly arranged in a bilayer formation, similar to the lipid arrangement of biological membranes.

As used herein, the term "immunoadhesin" designates antibody-like molecules that combine the binding specificity of a heterologous protein (an "adhesin") with the effector functions of immunoglobulin constant domains. Structurally, the immunoadhesins comprise a fusion of an amino acid sequence with the desired binding specificity that is other than the antigen recognition and binding site of an antibody (*i.e.,* is "heterologous"), and an immunoglobulin constant domain sequence. The adhesin part of an immunoadhesin molecule typically is a contiguous amino acid sequence comprising at least the binding site of a receptor or a ligand. The immunoglobulin constant domain sequence in the immunoadhesin may be obtained from any immunoglobulin, such as IgG-1, IgG-2, IgG-3, or IgG-4 subtypes, IgA (including IgA-1 and IgA-2). IgE, IgD, or IgM.

### II. Compositions and Methods of the Invention

### A. Preparation of the PRO364 or PRO175 Polypeptides

The present invention utilizes isolated nucleotide sequences encoding polypeptides referred to in the present application as PR0364 (also referred to as PR0687 or UNQ 319) and as PRO175. In particular, cDNAs encoding PRO364 and PRO175 polypeptides have been isolated and used, as disclosed in further detail in the Examples below. It is noted that proteins produced in separate expression rounds may be given different PRO numbers but the UNQ number is unique for any given DNA and the encoded protein, and will not be changed.

The description below relates primarily to production of PR0364 and PRO175 polypeptide by culturing cells transformed or transfected with a vector containing nucleic acid encoding PR0364 or PRO175 polypeptide. It is of course contemplated that alternative methods that are well known in the art may be employed to prepare PR0364 or PRO175. For instance, the PR0364 or PRO175 polypeptide sequence, or portions thereof, may be produced by direct peptide synthesis using solid-phase techniques. See, *e.g.*, Stewart et al., Solid-Phase Peptide Synthesis (W.H. Freeman Co.: San Francisco, CA, 1969); Merrifield. J. Am. Chem. Soc., 85: 2149-2154 (1963). *In vitro* protein synthesis may be performed using manual techniques or by automation. Automated synthesis may be accomplished, for instance, with an Applied Biosystems Peptide Synthesizer (Foster City, CA) using manufacturer's instructions. Various portions PR0364 or PRO175 may be chemically synthesized separately and combined using chemical or enzymatic methods to produce the full-length PR0364 or PRO175 polypeptide.

### Isolation of DNA Encoding PRO364 or PRO175

DNA encoding PR0364 or PRO175 polypeptide may be obtained from a cDNA library prepared from tissue believed to possess the mRNA encoding PR0364 or PRO175 and to express it at a detectable level. Accordingly, DNAs encoding human PR0364 or human PRO175 can be conveniently obtained from cDNA libraries prepared from human tissues, such as described in the Examples. The gene encoding PR0364 or PRO175 polypeptide may also be obtained from a genomic library or by oligonucleotide synthesis.

Libraries can be screened with probes (such as antibodies to the PRO364 or PRO175 polypeptide or oligonucleotides of at least about 20-80 bases) designed to identify the gene of interest or the protein encoded by it. Screening the cDNA or genomic library with the selected probe may be conducted using standard procedures, such as described in Sambrook *et al., supra.* An alternative means to isolate the gene encoding PR0364 or PRO175 is to use PCR methodology. Sambrook *et al., supra;* Dieffenbach et al., PCR Primer A Laboratory Manual (New York: Cold Spring Harbor Laboratory Press, 1995).

The Examples below describe techniques for screening a cDNA library. The oligonucleotide sequences selected as probes should be of sufficient length and sufficiently unambiguous that false positives are minimized. The oligonucleotide is preferably labeled such that it can be detected upon hybridization to DNA in the library being screened. Methods of labeling are well known in the art, and include the use of radiolabels like ³²P-labeled ATP, biotinylation, or enzyme labeling. Hybridization conditions, including moderate stringency and high stringency, are provided in Sambrook *et al., supra.*

Sequences identified in such library screening methods can be compared and aligned to other known sequences deposited and available in public databases such as GenBank or other private sequence databases. Sequence identity (at either the amino acid or nucleotide level) within defined regions of the molecule or across the full-length sequence can be determined through sequence alignment using computer software programs such as ALIGN, DNAstar, and INHERIT.

Nucleic acid having protein coding sequence may be obtained by screening selected cDNA or genomic libraries using the deduced amino acid sequence disclosed herein for the first time, and, if necessary, using conventional primer extension procedures as described in Sambrook *et al., supra,* to detect precursors and processing intermediates of mRNA that may not have been reverse-transcribed into cDNA.

In addition to the full-length native sequence PR0364 or PRO175 polypeptide described herein, it is contemplated that PR0364 or PRO175 variants can be prepared. PRO364 or PRO175 variants can be prepared by introducing appropriate nucleotide changes into the PRO364- or PRO175-encoding DNA or by synthesis of the desired PRO364 or PRO175 polypeptide. Those skilled in the art will appreciate that amino acid changes may alter post-translational processes of the PR0364 or PRO175 polypeptide, such as changing the number or position of glycosylation sites or altering the membrane anchoring characteristics.

Variations in the native full-length sequence PRO364 or PRO175 or in various domains of the PR0364 or PRO175 polypeptide described herein, can be made, for example, using any of the techniques and guidelines for conservative and non-conservative mutations set forth, for instance, in U.S. Patent No. 5,364,934. Variations may be a substitution, deletion or insertion of one or more codons encoding the PR0364 or PRO175 polypeptide that results in a change in the amino acid sequence of the PRO364 or PRO175 polypeptide as compared with the native sequence PR0364 or PRO175. Optionally the variation is by substitution of at least one amino acid with any other amino acid in one or more of the domains of the PR0364 or PRO175 polypeptide. Guidance in determining which amino acid residue may be inserted, substituted or deleted without adversely affecting the desired activity may be found by comparing the sequence of the PR0364 or PRO175 polypeptide with that of homologous known protein molecules and minimizing the number of amino acid sequence changes made in regions of high homology. Amino acid substitutions can be the result of replacing one amino acid with another amino acid having similar structural and/or chemical properties, such as the replacement of a leucine with a serine, i.e., conservative amino acid replacements. Insertions or deletions may optionally be in the range of 1 to 5 amino acids. The variation allowed may be determined by systematically making insertions, deletions or substitutions of amino acids in the sequence and testing the resulting variants for activity in any of the in *vitro* assays described in the Examples below.

The variations can be made using methods known in the art such as oligonucleotide-mediated (site-directed) mutagenesis, alanine scanning, and PCR mutagenesis. Site-directed mutagenesis [Carter et al., Nucl. Acids Res., 13:4331 (1986); Zoller et al., Nucl. Acids Res., 10:6487 (1987)], cassette mutagenesis [Wells et al., Gene, 34:315 (1985)], restriction selection mutagenesis [Wells et al., Philos. Trans. R. Soc. London SerA, 317:415 (1986)] or other known techniques can be performed on the cloned DNA to produce the PRO364-encoding variant DNA.

Scanning amino acid analysis can also be employed to identify one or more amino acids along a contiguous sequence. Among the preferred scanning amino acids are relatively small, neutral amino acids. Such amino acids include alanine, glycine, serine, and cysteine. Alanine is typically a preferred scanning amino acid among this group because it eliminates the side-chain beyond the beta-carbon and is less likely to alter the main-chain conformation of the variant. Alanine is also typically preferred because it is the most common amino acid. Further, it is frequently found in both buried and exposed positions [Creighton, The Proteins, (W.H. Freeman & Co., N.Y.); Chothia, J. Mol. Biol., 150:1 (1976)]. If alanine substitution does not yield adequate amounts of variant, an isoteric amino acid can be used.

### Modifications of PR0364

Covalent modifications of PR0364 or PRO175 polypeptides are included within the scope of this invention. One type of covalent modification includes reacting targeted amino acid residues of a PR0364 or PRO175 polypeptide with an organic derivatizing agent that is capable of reacting with selected side chains or the N- or C- terminal residues of a PR0364 or PRO175 polypeptide. Derivatization with bifunctional agents is useful, for instance, for crosslinking PR0364 or PRO175 to a water-insoluble support matrix or surface for use in the method for purifying anti-PR0364 or -PRO175 antibodies, and vice-versa. Commonly used crosslinking agents include. *e.g.,* 1,1-bis(diazoacetyl)-2-phenylethane, glutaraldehyde. N-hydroxysuccinimide esters, for example, esters with 4-azidosalicylic acid, homobifunctional imidoesters, including disuccinimidyl esters such as 3.3'-dithiobis(succinimidylpropionate), bifunctional maleimides such as bis-N-maleimido-1.8-octane and agents such as methyl-3-[(p-azidophenyl)dithio]propioimidate.

Other modifications include deamidation of glutaminyl and asparaginyl residues to the corresponding glutamyl and aspartyl residues, respectively, hydroxylation of proline and lysine, phosphorylation of hydroxyl groups of seryl or threonyl residues, methylation of the alpha-amino groups of lysine, arginine, and histidine side chains [T.E. Creighton, Proteins: Structure and Molecular Properties, W.H. Freeman & Co.. San Francisco, pp. 79-86 (1983)J, acetylation of the N-terminal amine, and amidation of any C-terminal carboxyl group.

Another type of covalent modification of the PR0364 or PRO175 polypeptide included within the scope of this invention comprises altering the native glycosylation pattern of the polypeptide. "Altering the native glycosylation pattern" is intended for purposes herein to mean deleting one or more carbohydrate moieties found in native sequence PRO364 or PRO175 polypeptide, and/or adding one or more glycosylation sites that are not present in the native sequence PRO364 or PRO175 polypeptide.

Addition of glycosylation sites to PRO364 or PRO175 polypeptides may be accomplished by altering the amino acid sequence thereof. The alteration may be made, for example, by the addition of, or substitution by, one or more serine or threonine residues to the native sequence PRO364 or PRO175 polypeptide (for O-linked glycosylation sites). The PRO364 or PRO175 amino acid sequence may optionally be altered through changes at the DNA level, particularly by mutating the DNA encoding the PRO364 or PRO175 polypeptide at preselected bases such that codons are generated that will translate into the desired amino acids.

Another means of increasing the number of carbohydrate moieties on the PR0364 or PRO175 polypeptide is by chemical or enzymatic coupling of glycosides to the polypeptide. Such methods are described in the art, *e.g.*, in WO 87/05330 published 11 September 1987, and in Aplin and Wriston, CRC Crit. Rev. Biochem., pp. 259-306 (1981).

Removal of carbohydrate moieties present on the PR0364 or PRO175 polypeptide may be accomplished chemically or enzymatically or by mutational substitution of codons encoding for amino acid residues that serve as targets for glycosylation. Chemical deglycosylation techniques are known in the art and described, for instance, by Hakimuddin, et al., Arch. Biochem. Biophys., 259:52 (1987) and by Edge et al., Anal. Biochem., 118:131 (1981). Enzymatic cleavage of carbohydrate moieties on polypeptides can be achieved by the use of a variety of endo- and exo-glycosidases as described by Thotakura et al., Meth. Enzymol., 138:350 (1987).

Another type of covalent modification of PR0364 or PRO175 comprises linking the PR0364 or PRO175 polypeptide to one of a variety of nonproteinaceous polymers, *e.g.,* polyethylene glycol, polypropylene glycol, or polyoxyalkylenes, in the manner set forth in U.S. Patent Nos. 4,640,835; 4,496,689; 4,301,144; 4,670,417; 4,791,192 or 4,179,337.

PR0364 or PRO175 polypeptides of the present invention may also be modified in a way to form chimeric molecules comprising a PR0364 or PRO175 polypeptide fused to another, heterologous polypeptide or amino acid sequence. In one embodiment, such a chimeric molecule comprises a fusion of a PR0364 or PRO175 polypeptide with a tag polypeptide which provides an epitope to which an anti-tag antibody can selectively bind. The epitope tag is generally placed at the amino- or carboxyl- terminus of the PR0364 or PRO175 polypeptide. The presence of such epitope-tagged forms of a PR0364 or PRO175 polypeptide can be detected using an antibody against the tag polypeptide. Also, provision of the epitope tag enables the PR0364 or PRO175 polypeptide to be readily purified by affinity purification using an anti-tag antibody or another type of affinity matrix that binds to the epitope tag. In an alternative embodiment, the chimeric molecule may comprise a fusion of a PR0364 or PRO175 polypeptide with an immunoglobulin or a particular region of an immunoglobulin. For a bivalent form of the chimeric molecule, such a fusion could be to the Fc region of an IgG molecule. Optionally, the chimeric molecule will comprise a PR0364 or PRO175 ECD sequence fused to an Fc region of an IgG molecule.

Various tag polypeptides and their respective antibodies are well known in the art. Examples include poly-histidine (poly-his) or poly-histidine-glycine (poly-his-gly) tags; the flu HA tag polypeptide and its antibody 12CA5 [Field et al., Mol. Cell. Biol., 8:2159-2165 (1988)]; the c-myc tag and the 8F9, 3C7, 6E10, G4, B7 and 9E10 antibodies thereto [Evan et al., Molecular and Cellular Biology, 5:3610-3616 (1985)]; and the Herpes Simplex virus glycoprotein D (gD) tag and its antibody [Paborsky et al., Protein Engineering, 3(6):547-553 (1990)]. Other tag polypeptides include the Flag-peptide [Hopp et al., BioTechnology, 6:1204-1210 (1988)]; the KT3 epitope peptide [Martin et al., Science, 255:192-194 (1992)]; an alpha-tubulin epitope peptide [Skinner et al., J. Biol. Chem., 266:15163-15166 (1991)]; and the T7 gene 10 protein peptide tag (Lutz-Freyermuth et al., Proc. Natl. Acad. Sci. USA, 87:6393-6397 (1990)].

The PR0364 or PROI75 polypeptide of the present invention may also be modified in a way to form a chimeric molecule comprising a PRO364 or PRO175 polypeptide fused to a leucine zipper. Various leucine zipper polypeptides have been described in the art. See, *e.g.*, Landschulz et al., Science 240:1759 (1988); WO 94/10308; Hoppe et al., FEBS Letters 344:1991 (1994); Maniatis et al., Nature 341:24 (1989). It is believed that use of a leucine zipper fused to a PR0364 or PRO175 polypeptide may be desirable to assist in dimerizing or trimerizing soluble PR0364 or PRO175 polypeptide in solution. Those skilled in the art will appreciate that the leucine zipper may be fused at either the N- or C-terminal end of the PR0364 or PRO175 molecule.

### Selection and Transformation of Host Cells

Host cells are transfected or transformed with expression or cloning vectors described herein for PR0364 or PRO175 production and cultured in conventional nutrient media modified as appropriate for inducing promoters, selecting transformants, or amplifying the genes encoding the desired sequences. The culture conditions, such as media, temperature, pH, and the like, can be selected by the skilled artisan without undue experimentation. In general, principles, protocols, and practical techniques for maximizing the productivity of cell cultures can be found in Mammalian Cell Biotechnology: A Practical Approach, M. Butler, ed. (IRL Press, 1991) and Sambrook *et al., supra.*

Methods of transfection are known to the ordinarily skilled artisan, for example, CaPO₄ treatment and electroporation. Depending, on the host cell used, transformation is performed using standard techniques appropriate to such cells. The calcium treatment employing calcium chloride, as described in Sambrook *et al., supra,* or electroporation is generally used for prokaryotes or other cells that contain substantial cell-wall barriers. Infection with *Agrobacterium tumefaciens* is used for transformation of certain plant cells, as described by Shaw et al., Gene, 23: 315 (1983) and WO 89/05859 published 29 June 1989. For mammalian cells without such cell walls, the calcium phosphate precipitation method of Graham and van der Eb, Virology, 52:456-437 (1978) can be employed. General aspects of mammalian cell host system transformations have been described in U.S. Patent No. 4,399,216. Transformations into yeast are typically carried out according to the method of Van Solingen et al., J. Bact., 130: 946 (1977) and Hsiao et al, Proc. Natl. Acad. Sci. (USA), 76: 3829 (1979). However, other methods for introducing DNA into cells, such as by nuclear microinjection, electroporation, bacterial protoplast fusion with intact cells, or polycations, *e.g.*, polybrene or polyomithine, may also be used. For various techniques for transforming mammalian cells, see Keown et al., Methods in Enzymology, 185: 527-537 (1990) and Mansour et al., Nature, 336: 348-352 (1988).

Suitable host cells for cloning or expressing the DNA in the vectors herein include prokaryote, yeast, or higher eukaryote cells. Suitable prokaryotes include, but are not limited to, eubacteria, such as Gram-negative or Gram-positive organisms, for example, Enterobacteriaceae such as *E*. *coli.* Various *E. coli* strains are publicly available, such as *E. coli* K12 strain MM294 (ATCC 31,446); *E. coli* X1776 (ATCC 31,537); *E. coli* strain W3110 (ATCC 27,325); and K5 772 (ATCC 53,635).

In addition to prokaryotes, eukaryotic microbes such as filamentous fungi or yeast are suitable cloning or expression hosts for vectors encoding PR0364 or PRO175. *Saccharomyces cerevisiae* is a commonly used lower eukaryotic host microorganism.

Suitable host cells for the expression of nucleic acid encoding glycosylated PR0364 or PRO175 are derived from multicellular organisms. Examples of invertebrate cells include insect cells such as Drosophila S2 and Spodoptera Sf9, as well as plant cells. Examples of useful mammalian host cell lines include Chinese hamster ovary (CHO) and COS cells. More specific examples include monkey kidney CV1 line transformed by SV40 (COS-7, ATCC CRL 1651); human embryonic kidney line (293 or 293 cells subcloned for growth in suspension culture, Graham et al., J. Gen Virol., 36: 59 (1977)); Chinese hamster ovary cells/-DHFR (CHO, Urlaub and Chasin, Proc. Natl. Acad. Sci. USA, 77:4216 (1980)); mouse sertoli cells (TM4, Mather, Biol. Reprod., 23:243-251 (1980)); human lung cells (W138, ATCC CCL 75); human liver cells (Hep G2, HB 8065); and mouse mammary tumor (MMT 060562, ATCC CCL51). The selection of the appropriate host cell is deemed to be within the skill in the art.

### Selection and Use of a Replicable Vector

The nucleic acid (*e.g.*, cDNA or genomic DNA) encoding PR0364 or PRO175 may be inserted into a replicable vector for cloning (amplification of the DNA) or for expression. Various vectors are publicly available. The vector may, for example, be in the form of a plasmid, cosmid, viral particle, or phage. The appropriate nucleic acid sequence may be inserted into the vector by a variety of procedures. In general, DNA is inserted into an appropriate restriction endonuclease site(s) using techniques known in the art. Vector components generally include, but are not limited to, one or more of a signal sequence if the sequence is to be secreted, an origin of replication, one or more marker genes, an enhancer element, a promoter, and a transcription termination sequence. Construction of suitable vectors containing one or more of these components employs standard ligation techniques that are known to the skilled artisan.

The PR0364 or PRO175 may be produced recombinantly not only directly, but also as a fusion polypeptide with a heterologous polypeptide, which may be a signal sequence or other polypeptide having a specific cleavage site at the N-terminus of the mature protein or polypeptide. In general, the signal sequence may be a component of the vector, or it may be a pan of the DNA PR0364 or PRO175 that is inserted into the vector. The signal sequence may be a prokaryotic signal sequence selected for example, from the group of the alkaline phosphatase, penicillinase, lpp, or heat-stable enterotoxin 11 leaders. For yeast secretion the signal sequence may be, *e.g.,* the yeast invertase leader, alpha factor leader (including *Saccharomyces* and *Kluyveromyces* α-factor leaders, the latter described in U.S. Patent No. 5,010,182), or acid phosphatase leader, the *C. albicans* glucoamylase leader (EP 362,179 published 4 April 1990), or the signal described in WO 90/13646 published 15 November 1990. In mammalian cell expression, mammalian signal sequences may be used to direct secretion of the protein, such as signal sequences from secreted polypeptides of the same or related species, as well as viral secretory leaders.

Both expression and cloning vectors contain a nucleic acid sequence that enables the vector to replicate in one or more selected host cells. Such sequences are well known for a variety of bacteria, yeast, and viruses. The origin of replication from the plasmid pBR322 is suitable for most Gram-negative bacteria, the 2µ plasmid origin is suitable for yeast, and various viral origins (SV40, polyoma, adenovirus, VSV, or BPV) are useful for cloning vectors in mammalian cells.

Expression and cloning vectors will typically contain a selection gene, also termed a selectable marker. Typical selection genes encode proteins that (a) confer resistance to antibiotics or other toxins, *e.g.*, ampicillin, neomycin, methotrexate, or tetracycline, (b) complement auxotrophic deficiencies, or (e) supply critical nutrients not available from complex media, *e.g.,* the gene encoding D-alanine racemase for *Bacilli.*

An example of suitable selectable markers for mammalian cells are those that enable the identification of cells competent to take up the nucleic acid encoding PR0364 or PRO175, such as DHFR or thymidine kinase. An appropriate host cell when wild-type DHFR is employed is the CHO cell line deficient in DHFR activity, prepared and propagated as described by Urlaub et al., Proc. Natl. Acad. Sci. USA, 77: 4216 (1980). A suitable selection gene for use in yeast is the *trp*1 gene present in the yeast plasmid YRp7. Stinchcomb et al., Nature, 282: 39 (1979); Kingsman et al., Gene, 7: 141 (1979); Tschemper et al., Gene, 10: 157 (1980). The *trp*1 gene provides a selection marker for a mutant strain of yeast lacking the ability to grow in tryptophan, for example, ATCC No. 44076 or PEP4-1. Jones, Genetics, 85: 12 (1977).

Expression and cloning vectors usually contain a promoter operably linked to the nucleic acid sequence encoding PR0364 or PRO175 to direct mRNA synthesis. Promoters recognized by a variety of potential host cells are well known. Promoters suitable for use with prokaryotic hosts include the β-lactamase and lactose promoter systems (Chang et al., Nature, 275: 615 (1978); Goeddel et al., Nature, 281: 544 (1979)), alkaline phosphatase, a tryptophan (trp) promoter system (Goeddel, Nucleic Acids Res., 8: 4057 (1980); EP 36,776), and hybrid promoters such as the tac promoter. deBoer et al., Proc. Natl. Acad. Sci. USA, 80: 21-25 (1983). Promoters for use in bacterial systems also will contain a Shine-Dalgarno (S.D.) sequence operably linked to the DNA encoding PR0364 or PRO175.

Examples of suitable promoting sequences for use with yeast hosts include the promoters for 3-phosphoglycerate kinase (Hitzeman et al., J. Biol. Chem., 255: 2073 (1980)) or other glycolytic enzymes (Hess et al., J. Adv. Enzyme Reg., 7: 149 (1968); Holland, Biochemistry, 17: 4900 (1978)), such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

Other yeast promoters that are inducible promoters having the additional advantage of transcription controlled by growth conditions are the promoter regions for alcohol dehydrogenase 2. isocytochrome C. acid phosphatase, degradative enzymes associated with nitrogen metabolism, metallothionein, glyceraldehyde-3-phosphate dehydrogenase, and enzymes responsible for maltose and galactose utilization. Suitable vectors and promoters for use in yeast expression are further described in EP 73,657.

PR0364 or PRO175 nucleic acid transcription from vectors in mammalian host cells is controlled, for example, by promoters obtained from the genomes of viruses such as polyoma virus, fowlpox virus (UK 2.211,504 published 5 July 1989), adenovirus (such as Adenovirus 2), bovine papilloma virus, avian sarcoma virus, cytomegalovirus, a retrovirus, hepatitis-B virus, and Simian Virus 40 (SV40); by heterologous mammalian promoters, *e.g.*, the actin promoter or an immunoglobulin promoter; and by heat-shock promoters, provided such promoters are compatible with the host cell systems.

Transcription of a DNA encoding the PR0364 or PRO175 by higher eukaryotes may be increased by inserting an enhancer sequence into the vector. Enhancers are cis-acting elements of DNA, usually about from 10 to 300 bp, that act on a promoter to increase its transcription. Many enhancer sequences are now known from mammalian genes (globin, elastase, albumin, α-fetoprotein, and insulin). Typically, however, one will use an enhancer from a eukaryotic cell virus. Examples include the SV40 enhancer on the late side of the replication origin (bp 100-270), the cytomegalovirus early promoter enhancer, the polyoma enhancer on the late side of the replication origin, and adenovirus enhancers. The enhancer may be spliced into the vector at a position 5' or 3' to the sequence coding for PRO364 or PRO175, but is preferably located at a site 5' from the promoter.

Expression vectors used in eukaryotic host cells (yeast, fungi, insect, plant, animal, human, or nucleated cells from other multicellular organisms) will also contain sequences necessary for the termination of transcription and for stabilizing the mRNA. Such sequences are commonly available from the 5' and, occasionally 3', untranslated regions of eukaryotic or viral DNAs or cDNAs. These regions contain nucleotide segments transcribed as polyadenylated fragments in the untranslated portion of the mRNA encoding PR0364 or PRO175.

Still other methods, vectors, and host cells suitable for adaptation to the synthesis of PR0364 or PRO175 in recombinant vertebrate cell culture are described in Gething et al., Nature, 293: 620-625 (1981); Mantei et al., Nature, 281: 40-46 (1979); EP 117,060; and EP 117,058.

### Detecting Gene Amplification/Expression

Gene amplification and/or expression may be measured in a sample directly, for example, by conventional Southern blotting, Northern blotting to quantitate the transcription of mRNA (Thomas, Proc. Natl. Acad. Sci. USA, 77:5201-5205 (1980)), dot blotting (DNA analysis), or *in situ* hybridization, using an appropriately labeled probe, based on the sequences provided herein. Alternatively, antibodies may be employed that can recognize specific duplexes, including DNA duplexes, RNA duplexes, and DNA-RNA hybrid duplexes or DNA-protein duplexes. The antibodies in turn may be labeled and the assay may be carried out where the duplex is bound to a surface, so that upon the formation of duplex on the surface, the presence of antibody bound to the duplex can be detected.

Gene expression, alternatively, may be measured by immunological methods, such as immunohistochemical staining of cells or tissue sections and assay of cell culture or body fluids, to quantitate directly the expression of gene product. Antibodies useful for immunohistochemical staining and/or assay of sample fluids may be either monoclonal or polyclonal, and may be prepared in any mammal. Conveniently, the antibodies may be prepared against a native- sequence PR0364 or PRO175 polypeptide or against a synthetic peptide based on the DNA sequences provided herein or against exogenous sequence fused to DNA encoding PR0364 or PRO175 and encoding a specific antibody epitope.

### Purification of Polypeptide

Forms of PRO364 or PRO175 may be recovered from culture medium or from host cell lysates. If membrane-bound, it can be released from the membrane using a suitable detergent solution (*e.g*., TRITON-X^{™} 100) or by enzymatic cleavage. Cells employed in expression of nucleic acid encoding PR0364 or PRO175 can be disrupted by various physical or chemical means, such as freeze-thaw cycling, sonication, mechanical disruption, or cell-lysing agents.

It may be desired to purify PRO364 or PRO175 polypeptide from recombinant cell proteins or polypeptides. The following procedures are exemplary of suitable purification procedures: by fractionation on an ion-exchange column; ethanol precipitation; reverse phase HPLC; chromatography on silica or on a cation-exchange resin such as DEAE; chromatofocusing; SDS-PAGE; ammonium sulfate precipitation; gel filtration using, for example, Sephadex G-75; protein A Sepharose columns to remove contaminants such as IgG; and metal chelating columns to bind epitope-tagged forms of the PR0364 or PRO175. Various methods of protein purification may be employed and such methods are known in the art and described, for example, in Deutscher, Methods in Enzymology, 182 (1990); Scopes, Protein Purification: Principles and Practice (Springer-Verlag: New York, 1982). The purification step(s) selected will depend, for example, on the nature of the production process used and the particular PR0364 or PRO175 produced.

### Uses of the PRO364 or PRO175 Polypeptides

### i. Assays for Cardiovascular, Endothelial, and Angiogenic Activity

Various assays can be used to test the polypeptide herein for cardiovascular, endothelial, and angiogenic activity. Such assays include those provided in the Examples below.

Assays for testing for endothelin antagonist activity, as disclosed in U.S. Pat. No. 5,773,414, include a rat heart ventricle binding assay where the polypeptide is tested for its ability to inhibit iodinized endothelin-1 binding in a receptor assay, an endothelin receptor binding assay testing for intact cell binding of radiolabeled endothelin-1 using rabbit renal artery vascular smooth muscle cells, an inositol phosphate accumulation assay where functional activity is determined in Rat-1 cells by measuring intra-cellular levels of second messengers, an arachidonic acid release assay that measures the ability of added compounds to reduce endothelin-stimulated arachidonic acid release in cultured vascular smooth muscles, *in vitro* (isolated vessel) studies using endothelium from male New Zealand rabbits, and *in vivo* studies using male Sprague-Dawley rats.

Assays for tissue generation activity include, without limitation, those described in WO 95/16035 (bone, cartilage, tendon); WO 95/05846 (nerve, neuronal), and WO 91/07491 (skin, endothelium).

Assays for wound-healing activity include, for example, those described in Winter, Epidermal Wound Healing, Maibach, HI and Rovee, DT, eds. (Year Book Medical Publishers, Inc., Chicago), pp. 71-112, as modified by the article of Eaglstein and Mertz, J. Invest. Dermatol., 71: 382-384 (1978).

An assay to screen for a test molecule relating to a PR0364 or PRO175 polypeptide that binds an endothelin B₁ (ETB₁) receptor polypeptide and modulates signal transduction activity involves providing a host cell transformed with a DNA encoding endothelin B₁ receptor polypeptide, exposing the cells to the test candidate, and measuring endothelin B₁ receptor signal transduction activity, as described, *e.g.,* in U.S. Pat. No. 5,773,223.

There are several cardiac hypertrophy assays. *In vitro* assays include induction of spreading of adult rat cardiac myocytes. In this assay, ventricular myocytes are isolated from a single (male Sprague-Dawley) rat, essentially following a modification of the procedure described in detail by Piper et al., "Adult ventricular rat heart muscle cells" in Cell Culture Techniques in Heart and Vessel Research, H.M. Piper, ed. (Berlin: Springer-Verlag, 1990), pp. 36-60. This procedure permits the isolation of adult ventricular myocytes and the long-term culture of these cells in the rod-shaped phenotype. Phenylephrine and Prostaglandin F_{2α} (PGF_{2α}) have been shown to induce a spreading response in these adult cells. The inhibition of myocyte spreading induced by PGF_{2α} or PGF_{2α} analogs (*e.g.*, fluprostenol) and phenylephrine by various potential inhibitors of cardiac hypertrophy is then tested.

One example of an *in vivo* assay is a test for inhibiting cardiac hypertrophy induced by fluprostenol in *vivo.* This pharmacological model tests the ability of the PR0364 or PRO175 polypeptide to inhibit cardiac hypertrophy induced in rats (*e.g*., male Wistar or Sprague-Dawley) by subcutaneous injection of fluprostenol (an agonist analog of PGF_{2α}). It is known that rats with pathologic cardiac hypertrophy induced by myocardial infarction have chronically elevated levels of extractable PGF_{2α} in their myocardium. Lai et al., Am. J. Phvsiol. (Heart Circ. Physiol.), 271: H2197-H2208 (1996). Accordingly, factors that can inhibit the effects of fluprostenol on myocardial growth in *vivo* are potentially useful for treating cardiac hypertrophy. The effects of the PR0364 or PRO175 polypeptide on cardiac hypertrophy are determined by measuring the weight of heart, ventricles, and left ventricle (normalized by body weight) relative to fluprostenol-treated rats not receiving the PR0364 or PRO175 polypeptide.

Another example of an in *vivo* assay is the pressure-overload cardiac hypertrophy assay. For in *vivo* testing it is common to induce pressure-overload cardiac hypertrophy by constriction of the abdominal aorta of test animals. In a typical protocol, rats (*e.g.*, male Wistar or Sprague-Dawley) are treated under anesthesia, and the abdominal aorta of each rat is narrowed down just below the diaphragm. Beznak M., Can. J. biochem. Physiol., 33: 985-94 (1955). The aorta is exposed through a surgical incision, and a blunted needle is placed next to the vessel. The aorta is constricted with a ligature of silk thread around the needle, which is immediately removed and which reduces the lumen of the aorta to the diameter of the needle. This approach is described, for example, in Rossi et al., Am. Heart J., 124: 700-709 (1992) and O'Rourke and Reibel, P.S.E.M.B., 200: 95-100 (1992).

In yet another *in vivo* assay, the effect on cardiac hypertrophy following experimentally induced myocardial infarction (MI) is measured. Acute MI is induced in rats by left coronary artery ligation and confirmed by electrocardiographic examination. A sham-operated group of animals is also prepared as control animals. Earlier data have shown that cardiac hypertrophy is present in the group of animals with MI, as evidenced by an 18% increase in heart weight-to-body weight ratio. Lai *et al., supra.* Treatment of these animals with candidate blockers of cardiac hypertrophy, *e.g.*, PR0364 or PRO175 polypeptide, provides valuable information about the therapeutic potential of the candidates tested. One further such assay test for induction of cardiac hypertrophy is disclosed in U.S. Pat. No. 5,773,415, using Sprague-Dawley rats.

For cancer, a variety of well-known animal models can be used to further understand the role of the genes identified herein in the development and pathogenesis of tumors, and to test the efficacy of candidate therapeutic agents, including antibodies and other antagonists of the native PR0364 or PRO175 polypeptides, such as small-molecule antagonists. The *in vivo* nature of such models makes them particularly predictive of responses in human patients. Animal models of tumors and cancers (*e*.*g*., breast cancer, colon cancer, prostate cancer, lung cancer, etc.) include both non-recombinant and recombinant (transgenic) animals. Non-recombinant animal models include, for example, rodent, *e.g.*, murine models. Such models can be generated by introducing tumor cells into syngeneic mice using standard techniques, *e.g.,* subcutaneous injection, tail vein injection, spleen implantation, intraperitoneal implantation, implantation under the renal capsule, or orthopin implantation, *e.g.,* colon cancer cells implanted in colonic tissue. See. *e.g*., PCT publication No. WO 97/33551, published September 18, 1997.

Probably the most often used animal species in oncological studies are immunodeficient mice and, in particular, nude mice. The observation that the nude mouse with thymic hypo/aplasia could successfully act as a host for human tumor xenografts has lead to its widespread use for this purpose. The autosomal recessive nu gene has been introduced into a very large number of distinct congenic strains of nude mouse, including, for example, ASW, A/He, AKR, BALB/c, B10.LP, C17. C3H, C57BL, C57, CBA, DBA, DDD, I/st, NC, NFR, NFS, NFS/N, NZB, NZC, NZW, P, RIII, and SJL. In addition, a wide variety of other animals with inherited immunological defects other than the nude mouse have been bred and used as recipients of tumor xenografts. For further details see, *e.g.*, The Nude Mouse in Oncology Research, E. Boven and B. Winograd, eds. (CRC Press, Inc., 1991).

The cells introduced into such animals can be derived from known tumor/cancer cell lines, such as any of the above-listed tumor cell lines, and, for example, the B104-1-1 cell line (stable NIH-3T3 cell line transfected with the neu protooncogene); ras-transfected MH-3T3 cells; Caco-2 (ATCC HTB-37); or a moderately well-differentiated grade II human colon adenocarcinoma cell line, HT-29 (ATCC HTB-38); or from tumors and cancers. Samples of tumor or cancer cells can be obtained from patients undergoing surgery, using standard conditions involving freezing and storing in liquid nitrogen. Karmali et al., Br. J. Cancer, 48: 689-696 (1983).

Tumor cells can be introduced into animals such as nude mice by a variety of procedures. The subcutaneous (s.c.) space in mice is very suitable for tumor implantation. Tumors can be transplanted s.c. as solid blocks, as needle biopsies by use of a trochar, or as cell suspensions. For solid-block or trochar implantation, tumor tissue fragments of suitable size are introduced into the s.c. space. Cell suspensions are freshly prepared from primary tumors or stable tumor cell lines, and injected subcutaneously. Tumor cells can also be injected as subdermal implants. In this location, the inoculum is deposited between the lower part of the dermal connective tissue and the s.c. tissue.

Animal models of breast cancer can be generated, for example, by implanting rat neuroblastoma cells (from which the neu oncogene was initially isolated), or *neu*-transformed NIH=3T3 cells into nude mice, essentially as described by Drebin et al. Proc. Nat. Acad. Sci. USA, 83: 9129-9133 (1986).

Similarly, animal models of colon cancer can be generated by passaging colon cancer cells in animals, *e.g.*, nude mice, leading to the appearance of tumors in these animals. An orthotopic transplant model of human colon cancer in nude mice has been described, for example, by Wang et al., Cancer Research, 54: 4726-4728 (1994) and Too et al., Cancer Research, 55: 681-684 (1995). This model is based on the so-called □METAMOUSE□^{™} sold by AntiCancer, Inc. (San Diego, California).

Tumors that arise in animals can be removed and cultured *in vitro.* Cells from the in *vitro* cultures can then be passaged to animals. Such tumors can serve as targets for further testing or drug screening. Alternatively, the tumors resulting from the passage can be isolated and RNA from pre-passage cells and cells isolated after one or more rounds of passage analyzed for differential expression of genes of interest. Such passaging techniques can be performed with any known tumor or cancer cell lines.

For example, Meth A, CMS4, CMS5, CMS21, and WEHI-164 are chemically induced fibrosarcomas of BALB/c female mice (DeLeo et al., J. Exp. Med., 146: 720 (1977)), which provide a highly controllable model system for studying the anti-tumor activities of various agents. Palladino et al., J. Immunol., 138: 4023-4032 (1987). Briefly, tumor cells are propagated *in vitro* in cell culture. Prior to injection into the animals, the cell lines are washed and suspended in buffer, at a cell density of about 10x10⁶ to 10x10⁷ cells/ml. The animals are then infected subcutaneously with 10 to 100 µl of the cell suspension, allowing one to three weeks for a tumor to appear.

In addition, the Lewis lung (3LL) carcinoma of mice, which is one of the most thoroughly studied experimental tumors, can be used as an investigational tumor model. Efficacy in this tumor model has been correlated with beneficial effects in the treatment of human patients diagnosed with small-cell carcinoma of the lung (SCCL). This tumor can be introduced in normal mice upon injection of tumor fragments from an affected mouse or of cells maintained in culture. Zupi et al.. Br. J. Cancer, 41: suppl. 4. 30 (1980). Evidence indicates that tumors can be started from injection of even a single cell and that a very high proportion of infected tumor cells survive. For further information about this tumor model see Zacharski, Haemostasis, 16: 300-320 (1986).

One way of evaluating the efficacy of a test compound in an animal model with an implanted tumor is to measure the size of the tumor before and after treatment. Traditionally, the size of implanted tumors has been measured with a slide caliper in two or three dimensions. The measure limited to two dimensions does not accurately reflect the size of the tumor, therefore, it is usually converted into the corresponding volume by using a mathematical formula. However, the measurement of tumor size is very inaccurate. The therapeutic effects of a drug candidate can be better described as treatment-induced growth delay and specific growth delay. Another important variable in the description of tumor growth is the tumor volume doubling time. Computer programs for the calculation and description of tumor growth are also available, such as the program reported by Rygaard and Spang-Thomsen, Proc. 6th Int. Workshop on Immune-Deficient Animals, Wu and Sheng eds. (Basel, 1989), p. 301. It is noted, however, that necrosis and inflammatory responses following treatment may actually result in an increase in tumor size, at least initially. Therefore, these changes need to be carefully monitored, by a combination of a morphometric method and flow cytometric analysis.

Further, recombinant (transgenic) animal models can be engineered by introducing the coding portion of the PR0364 or PRO175 genes identified herein into the genome of animals of interest, using standard techniques for producing transgenic animals. Animals that can serve as a target for transgenic manipulation include, without limitation, mice, rats, rabbits, guinea pigs, sheep, goats, pigs, and non-human primates, *e.g.*, baboons, chimpanzees and monkeys. Techniques known in the art to introduce a transgene into such animals include pronucleic microinjection (U.S. Patent No. 4,873,191); retrovirus-mediated gene transfer into germ lines (*e.g.*, Van der Putten et al., Proc. Natl. Acad. Sci. USA, 82: 6148-615 (1985)); gene targeting in embryonic stem cells (Thompson et al., Cell, 56: 313-321 (1989)); electroporation of embryos (Lo, Mol. Cell. Biol., 3: 1-803-1814 (1983)); and sperm-mediated gene transfer. Lavitrano et al., Cell, 57: 717-73 (1989). For a review, see, for example, U.S. Patent No. 4,736,866.

For the purpose of the present invention, transgenic animals include those that carry the transgene only in part of their cells ("mosaic animals"). The transgene can be integrated either as a single transgene, or in concatamers, *e.g*., head-to-head or head-to-tail tandems. Selective introduction of a transgene into a particular cell type is also possible by following, for example, the technique of Lasko et al., Proc. Natl. Acad. Sci. USA, 89: 6232-636 (1992).

The expression of the transgene in transgenic animals can be monitored by standard techniques. For example, Southern blot analysis or PCR amplification can be used to verify the integration of the transgene. The level of mRNA expression can then be analyzed using techniques such as *in situ* hybridization, Northern blot analysis, PCR, or immunocytochemistry. The animals are further examined for signs of tumor or cancer development.

Alternatively, "knock-out" animals can be constructed that have a defective or altered gene encoding a PRO364 or PRO175 polypeptide identified herein, as a result of homologous recombination between the endogenous gene encoding the PRO364 or PRO175 polypeptide and altered genomic DNA encoding the same polypeptide introduced into an embryonic cell of the animal. For example, cDNA encoding a particular PR0364 or PRO175 polypeptide can be used to clone genomic DNA encoding that polypeptide in accordance with established techniques. A portion of the genomic DNA encoding a particular PR0364 or PRO175 polypeptide can be deleted or replaced with another gene, such as a gene encoding a selectable marker that can be used to monitor integration. Typically, several kilobases of unaltered flanking DNA (both at the 5' and 3' ends) are included in the vector. See, *e.g.*, Thomas and Capecchi, Cell, 51: 503 (1987) for a description of homologous recombination vectors. The vector is introduced into an embryonic stem cell line (*e.g*., by electroporation) and cells in which the introduced DNA has homologously recombined with the endogenous DNA are selected. See, *e.g.,* Li et al., Cell, 69: 915 (1992). The selected cells are then injected into a blastocyst of an animal *(e.g.,* a mouse or rat) to form aggregation chimeras. See, *e.g.*, Bradley, in Teratocarcinomas and Embryonic Stem Cells: A Practical Approach, E. J. Robertson, ed. (IRL: Oxford, 1987), pp. 113-152. A chimeric embryo can then be implanted into a suitable pseudopregnant female foster animal and the embryo brought to term to create a "knock-out" animal. Progeny harboring the homologously recombined DNA in their germ cells can be identified by standard techniques and used to breed animals in which all cells of the animal contain the homologously recombined DNA. Knockout animals can be characterized, for instance, by their ability to defend against certain pathological conditions and by their development of pathological conditions due to absence of the PR0364 or PRO175 polypeptide.

The efficacy of antibodies specifically binding the PR0364 or PRO175 polypeptides identified herein, and other drug candidates, can be tested also in the treatment of spontaneous animal tumors. A suitable target for such studies is the feline oral squamous cell carcinoma (SCC). Feline oral SCC is a highly invasive, malignant tumor that is the most common oral malignancy of cats, accounting for over 60% of the oral tumors reported in this species. It rarely metastasizes to distant sites, although this low incidence of metastasis may merely be a reflection of the short survival times for cats with this tumor. These tumors are usually not amenable to surgery, primarily because of the anatomy of the feline oral cavity. At present, there is no effective treatment for this tumor. Prior to entry into the study, each cat undergoes complete clinical examination and biopsy, and is scanned by computed tomography (CT). Cats diagnosed with sublingual oral squamous cell rumors are excluded from the study. The tongue can become paralyzed as a result of such tumor, and even if the treatment kills the tumor, the animals may not be able to feed themselves. Each cat is treated repeatedly, over a longer period of time. Photographs of the tumors will be taken daily during the treatment period, and at each subsequent recheck. After treatment, each cat undergoes another CT scan. CT scans and thoracic radiograms are evaluated every 8 weeks thereafter. The data are evaluated for differences in survival, response, and toxicity as compared to control groups. Positive response may require evidence of tumor regression, preferably with improvement of quality of life and/or increased life span.

In addition, other spontaneous animal tumors, such as fibrosarcoma, adenocarcinoma, lymphoma, chondroma, or leiomyosarcoma of dogs, cats, and baboons can also be tested. Of these, mammary adenocarcinoma in dogs and cats is a preferred model as its appearance and behavior are very similar to those in humans. However, the use of this model is limited by the rare occurrence of this type of tumor in animals.

Other *in vitro* and *in vivo* cardiovascular, endothelial, and angiogenic tests known in the an are also suitable herein.

### ii. Tissue Distribution

The results of the cardiovascular, endothelial, and angiogenic assays herein can be verified by further studies, such as by determining mRNA expression in various human tissues.

As noted before, gene amplification and/or gene expression in various tissues may be measured by conventional Southern blotting, Northern blotting to quantitate the transcription of mRNA (Thomas. Proc. Natl. Acad. Sci. USA, 77:5201-5205 (1980)), dot blotting (DNA analysis), or *in situ* hybridization, using an appropriately labeled probe, based on the sequences provided herein. Alternatively, antibodies may be employed that can recognize specific duplexes, including DNA duplexes, RNA duplexes, and DNA-RNA hybrid duplexes or DNA-protein duplexes.

Gene expression in various tissues, alternatively, may be measured by immunological methods, such as immunohistochemical staining of tissue sections and assay of cell culture or body fluids, to quantitate directly the expression of gene product. Antibodies useful for immunohistochemical staining and/or assay of sample fluids may be either monoclonal or polyclonal, and may be prepared in any mammal. Conveniently, the antibodies may be prepared against a native-sequence PR0364 or PRO175 polypeptide or against a synthetic peptide based on the DNA sequences provided herein or against exogenous sequence fused to PR0364 or PRO175 DNA and encoding a specific antibody epitope. General techniques for generating antibodies, and special protocols for in *situ* hybridization are provided hereinbelow.

### iii. Antibody Binding Studies

The results of the cardiovascular, endothelial, and angiogenic study can be further verified by antibody binding studies, in which the ability of anti- PR0364 or -PRO175 antibodies to inhibit the effect of the PRO364 or PRO175 polypeptides on endothelial cells or other cells used in the cardiovascular, endothelial, and angiogenic assays is tested. Exemplary antibodies include polyclonal, monoclonal, humanized, bispecific, and heteroconjugate antibodies, the preparation of which will be described hereinbelow.

Antibody binding studies may be carried out in any known assay method, such as competitive binding assays, direct and indirect sandwich assays, and immunoprecipitation assays. Zola, Monoclonal Antibodies: A Manual of Techniques (CRC Press, Inc., 1987), pp.147-158.

Competitive binding assays rely on the ability of a labeled standard to compete with the test sample analyte for binding with a limited amount of antibody. The amount of target protein in the test sample is inversely proportional to the amount of standard that becomes bound to the antibodies. To facilitate determining the amount of standard that becomes bound, the antibodies preferably are insolubilized before or after the competition, so that the standard and analyte that are bound to the antibodies may conveniently be separated from the standard and analyte that remain unbound.

Sandwich assays involve the use of two antibodies, each capable of binding to a different immunogenic portion, or epitope, of the protein to be detected. In a sandwich assay, the test sample analyte is bound by a first antibody that is immobilized on a solid support, and thereafter a second antibody binds to the analyte, thus forming an insoluble three-part complex. See, *e.g.*, US Pat No. 4.376,110. The second antibody may itself be labeled with a detectable moiety (direct sandwich assays) or may be measured using an anti-immunoglobulin antibody that is labeled with a detectable moiety (indirect sandwich assay). For example, one type of sandwich assay is an ELISA assay, in which case the detectable moiety is an enzyme.

For immunohistochemistry, the tissue sample may be fresh or frozen or may be embedded in paraffin and fixed with a preservative such as formalin, for example.

### iv. Cell-Based Tumor Assays

Cell-based assays and animal models for cardiovascular, endothelial, and angiogenic disorders, such as tumors, can be used to verify the findings of a cardiovascular, endothelial, and angiogenic assay herein, and further to understand the relationship between the genes identified herein and the development and pathogenesis of undesirable cardiovascular, endothelial, and angiogenic cell growth. The role of gene products identified herein in the development and pathology of undesirable cardiovascular, endothelial and angiogenic cell growth, *e.g.*, tumor cells, can be tested by using cells or cells lines that have been identified as being stimulated or inhibited by the PR0364 or PRO175 polypeptide herein. Such cells include, for example, those set forth in the Examples below.

In a different approach, cells of a cell type known to be involved in a particular cardiovascular, endothelial, and angiogenic disorder are transfected with the cDNAs herein, and the ability of these cDNAs to induce excessive growth or inhibit growth is analyzed. If the cardiovascular, endothelial, and angiogenic disorder is cancer, suitable rumor cells include, for example, stable tumor cells lines such as the B104-1-1 cell line (stable N1H-3T3 cell line transfected with the *neu* protooncogene) and *ras*-transfected N1H-3T3 cells, which can be transfected with the desired gene and monitored for tumorigenic growth. Such transfected cell lines can then be used to test the ability of poly- or monoclonal antibodies or antibody compositions to inhibit tumorogenic cell growth by exerting cytostatic or cytotoxic activity on the growth of the transformed cells, or by mediating antibody-dependent cellular cytotoxicity (ADCC). Cells transfected with the coding sequences of the genes identified herein can further be used to identify drug candidates for the treatment of cardiovascular, endothelial, and angiogenic disorders such as cancer.

In addition, primary cultures derived from tumors in transgenic animals (as described above) can be used in the cell-based assays herein, although stable cell lines are preferred. Techniques to derive continuous cell lines from transgenic animals are well known in the art. See, *e.g.,* Small et al., Mol. Cell. Biol. 5: 642-648 (1985).

### v. Gene Therapy

The PRO364 or PRO175 polypeptide herein and polypeptidyl agonists and antagonists may be employed by expression of such polypeptides in *vivo*, which is often referred to as gene therapy.

There arc two major approaches to getting the nucleic acid (optionally contained in a vector) into the patient's cells: *in vivo* and ex *vivo.* For *in vivo* delivery the nucleic acid is injected directly into the patient, usually at the sites where the PR0364 or PRO175 polypeptide is required, *i.e.,* the site of synthesis of the PR0364 or PRO175 polypeptide, if known, and the site (*e.g.,* wound) where biological activity of PR0364 or PRO175 polypeptide is needed. For *ex vivo* treatment, the patient's cells are removed, the nucleic acid is introduced into these isolated cells, and the modified cells are administered to the patient either directly or, for example, encapsulated within porous membranes that are implanted into the patient (sec, *e.g*., U.S. Pat. Nos. 4,892,538 and 5,283,187).

There are a variety of techniques available for introducing nucleic acids into viable cells. The techniques vary depending upon whether the nucleic acid is transferred into cultured cells *in vitro,* or transferred *in vivo* in the cells of the intended host. Techniques suitable for the transfer of nucleic acid into mammalian cells *in vitro* include the use of liposomes, electroporation, microinjection, transduction, cell fusion, DEAE-dextran, the calcium phosphate precipitation method, etc. Transduction involves the association of a replication-defective, recombinant viral (preferably retroviral) particle with a cellular receptor, followed by introduction of the nucleic acids contained by the particle into the cell. A commonly used vector for *ex vivo* delivery of the gene is a retrovirus.

The currently preferred *in vivo* nucleic acid transfer techniques include transfection with viral or non-viral vectors (such as adenovirus, lentivirus. Herpes simplex I virus, or adeno-associated virus (AAV)) and lipid-based systems (useful lipids for lipid-mediated transfer of the gene are, for example, DOTMA, DOPE, and DC-Chol; see, *e.g.*, Tonkinson et al., Cancer Investieation, 14(1): 54-65 (1996)). The most preferred vectors for use in gene therapy are viruses, most preferably adenoviruses, AAV, lentiviruses, or retroviruses. A viral vector such as a retroviral vector includes at least one transcriptional promoter/enhancer or locus-defining element(s), or other elements that control gene expression by other means such as alternate splicing, nuclear RNA export, or post-translational modification of messenger. In addition, a viral vector such as a retroviral vector includes a nucleic acid molecule that, when transcribed in the presence of a gene encoding PR0364 or PRO175 polypeptide, is operably linked thereto and acts as a translation initiation sequence. Such vector constructs also include a packaging signal, long terminal repeats (LTRs) or portions thereof, and positive and negative strand primer binding sites appropriate to the virus used (if these are not already present in the viral vector). In addition, such vector typically includes a signal sequence for secretion of the PR0364 or PRO175 polypeptide from a host cell in which it is placed. Preferably the signal sequence for this purpose is a mammalian signal sequence, most preferably the native signal sequence for PR0364 or PRO175 polypeptide. Optionally, the vector construct may also include a signal that directs polyadenylation, as well as one or more restriction sites and a translation termination sequence. By way of example, such vectors will typically include a 5' LTR, a tRNA binding site, a packaging signal, an origin of second-strand DNA synthesis, and a 3' LTR or a portion thereof. Other vectors can be used that are non-viral, such as cationic lipids, polylysine, and dendrimers.

In some situations, it is desirable to provide the nucleic acid source with an agent that targets the target cells, such as an antibody specific for a cell-surface membrane protein or the target cell, a ligand for a receptor on the target cell, etc. Where liposomes are employed, proteins that bind to a cell-surface membrane protein associated with endocytosis may be used for targeting and/or to facilitate uptake, e.g,. capsid proteins or fragments thereof tropic for a particular cell type, antibodies for proteins that undergo internalization in cycling, and proteins that target intracellular localization and enhance intracellular half-life. The technique of receptor-mediated endocytosis is described, for example, by Wu et al., J. Biol. Chem., 262: 4429-4432 (1987); and Wagner et al., Proc. Natl. Acad. Sci. USA, 87: 3410-3414 (1990). For a review of the currently known gene marking and gene therapy protocols, see Anderson et al. Science, 256: 808-813 (1992). See also WO 93/25673 and the references cited therein.

Suitable gene therapy and methods for making retroviral particles and structural proteins can be found in, *e.g.,* U.S. Pat. No. 5,681,746.

### vi. Use of Gene as Diagnostic

This invention is also related to the use of the gene encoding the PRO364 or PRO175 polypeptide as a diagnostic. Detection of a mutated form of the PR0364 or PRO175 polypeptide will allow a diagnosis of a cardiovascular, endothelial, and angiogenic disease or a susceptibility to a cardiovascular, endothelial, and angiogenic disease, such as a tumor, since mutations in the PR0364 or PRO175 polypeptide may cause tumors.

Individuals carrying mutations in the genes encoding human PRO364, or human PRO175 polypeptide may be detected at the DNA level by a variety of techniques. Nucleic acids for diagnosis may be obtained from a patient's cells, such as from blood, urine, saliva, tissue biopsy, and autopsy material. The genomic DNA may be used directly for detection or may be amplified enzymatically by using PCR (Saiki et al., Nature, 324: 163-166 (1986)) prior to analysis. RNA or cDNA may also be used for the same purpose. As an example. PCR primers complementary to the nucleic acid encoding the PR0364 or PRO175 polypeptide can be used to identify and PR0364 or PRO175 polypeptide mutations. For example, deletions and insertions can be detected by a change in size of the amplified product in comparison to the normal genotype. Point mutations can be identified by hybridizing amplified DNA to radiolabeled RNA encoding PR0364 or PRO175 polypeptide, or alternatively, radiolabeled antisense DNA sequences encoding PR0364 or PRO175 polypeptide. Perfectly matched sequences can be distinguished from mismatched duplexes by RNase A digestion or by differences in melting temperatures.

Genetic testing based on DNA sequence differences may be achieved by detection of alteration in electrophoretic mobility of DNA fragments in gels with or without denaturing agents. Small sequence deletions and insertions can be visualized by high resolution gel electrophoresis. DNA fragments of different sequences may be distinguished on denaturing formamidine gradient gels in which the mobilities of different DNA fragments are retarded in the gel at different positions according to their specific melting or partial melting temperatures. See, *e.g.*, Myers et al., Science, 230: 1242 (1985).

Sequence changes at specific locations may also be revealed by nuclease protection assays, such as RNase and S1 protection or the chemical cleavage method, for example, Cotton et al.; Proc. Natl. Acad. Sci. USA, 85: 4397-4401 (1985).

Thus, the detection of a specific DNA sequence may be achieved by methods such as hybridization, RNase protection, chemical cleavage, direct DNA sequencing, or the use of restriction enzymes, *e.g.*, restriction fragment length polymorphisms (RFLP), and Southern blotting of genomic DNA.

### vii. Use to Detect PRO364 or PRO175 Polypeptide Levels

In addition to more conventional gel-electrophoresis and DNA sequencing, mutations can also be detected by in *situ* analysis.

Expression of nucleic acid encoding PR0364 or PRO175 polypeptide may be linked to vascular disease or neovascularization associated with tumor formation. If the PR0364 or PRO175 polypeptide has a signal sequence and the mRNA is highly expressed in endothelial cells and to a lesser extent in smooth muscle cells, this indicates that the PR0364 or PRO175 polypeptide is present in serum. Accordingly, an anti- PR0364 or - PRO175 polypeptide antibody could be used to diagnose vascular disease or neovascularization associated with tumor formation, since an altered level of this PR0364 or PRO175 polypeptide may be indicative of such disorders.

A competition assay may be employed wherein antibodies specific to the PR0364 or PRO175 polypeptide are attached to a solid support and labeled PR0364 or PRO175 polypeptide and a sample derived from the host are passed over the solid support and the amount of label detected attached to the solid support can be correlated to a quantity of PR0364 or PRO175 polypeptide in the sample.

### viii. Chromosome Mapping

The sequences of the present invention are also valuable for chromosome identification. The sequence is specifically targeted to and can hybridize with a particular location on an individual human chromosome. Moreover, there is a current need for identifying particular sites on the chromosome. Few chromosome marking reagents based on actual sequence data (repeat polymorphisms) are presently available for marking chromosomal location. The mapping of DNAs to chromosomes according to the present invention is an important first step in correlating those sequences with genes associated with disease.

Briefly, sequences can be mapped to chromosomes by preparing PCR primers (preferably 15-25 bp) from the cDNA. Computer analysis for the 3- untranslated region is used to rapidly select primers that do not span more than one exon in the genomic DNA. thus complicating the amplification process. These primers are then used for PCR screening of somatic cell hybrids containing individual human chromosomes. Only those hybrids containing the human gene corresponding to the primer will yield an amplified fragment.

PCR mapping of somatic cell hybrids is a rapid procedure for assigning a particular DNA to a particular chromosome. Using the present invention with the same oligonucleotide primers, sublocalization can be achieved with panels of fragments from specific chromosomes or pools of large genomic clones in an analogous manner. Other mapping strategies that can similarly be used to map to its chromosome include *in situ* hybridization, prescreening with labeled flow-sorted chromosomes, and preselection by hybridization to construct chromosome-specific cDNA libraries.

Fluorescence *in situ* hybridization (FISH) of a cDNA clone to a metaphase chromosomal spread can be used to provide a precise chromosomal location in one step. This technique can be used with cDNA as short as 500 or 600 bases; however, clones larger than 2,000 bp have a higher likelihood of binding to a unique chromosomal location with sufficient signal intensity for simple detection. FISH requires use of the clones from which the gene encoding PR0364 or PRO175 polypeptide was derived, and the longer the better. For example, 2.000 bp is good, 4,000 bp is better, and more than 4,000 is probably not necessary to get good results a reasonable percentage of the time. For a review of this technique, see Verma et al., Human Chromosomes: a Manual of Basic Technique (Pergamon Press, New York, 1988).

Once a sequence has been mapped to a precise chromosomal location, the physical position of the sequence on the chromosome can be correlated with genetic map data. Such data are found, for example, in V. McKusick, Mendelian Inheritance in Man (available on line through Johns Hopkins University Welch Medical Library). The relationship between genes and diseases that have been mapped to the same chromosomal region is then identified through linkage analysis (coinheritance of physically adjacent genes).

Next, it is necessary to determine the differences in the cDNA or genomic sequence between affected and unaffected individuals. If a mutation is observed in some or all of the affected individuals but not in any normal individuals, then the mutation is likely to be the causative agent of the disease.

With current resolution of physical mapping and genetic mapping techniques, a cDNA precisely localized to a chromosomal region associated with the disease could be one of between 50 and 500 potential causative genes. (This assumes 1 megabase mapping resolution and one gene per 20 kb).

### ix: Screening Assays for Drug Candidates

This invention encompasses methods of screening compounds to identify those that mimic the PR0364 or PRO175 polypeptide (agonists) or prevent the effect of the PR0364 or PRO175 polypeptide (antagonists). Screening assays for antagonist drug candidates are designed to identify compounds that bind or complex with the PR0364 or PRO175 polypeptides encoded by the genes identified herein, or otherwise interfere with the interaction of the encoded polypeptides with other cellular proteins. Such screening assays will include assays amenable to high-throughput screening of chemical libraries, making them particularly suitable for identifying small molecule drug candidates.

The assays can be performed in a variety of formats, including protein-protein binding assays, biochemical screening assays, immunoassays, and cell- based assays, which are well characterized in the art. All assays for antagonists are common in that they call for contacting the drug candidate with a PR0364 or PRO175 polypeptide encoded by a nucleic acid identified herein under conditions and for a time sufficient to allow these two components to interact.

In binding assays, the interaction is binding and the complex formed can be isolated or detected in the reaction mixture. In a particular embodiment, the PR0364 or PRO175 polypeptide encoded by the gene identified herein or the drug candidate is immobilized on a solid phase, *e.g.,* on a microtiter plate, by covalent or non-covalent attachments. Non-covalent attachment generally is accomplished by coating the solid surface with a solution of the PRO364 or PRO175 polypeptide and drying. Alternatively, an immobilized antibody, *e.g.*, a monoclonal antibody, specific for PRO364 or PRO175 polypeptide to be immobilized can be used to anchor it to a solid surface. The assay is performed by adding the non-immobilized component, which may be labeled by a detectable label, to the immobilized component, *e.g.*, the coated surface containing the anchored component. When the reaction is complete, the non-reacted components are removed, *e.g.*, by washing, and complexes anchored on the solid surface are detected. When the originally non-immobilized component carries a detectable label, the detection of label immobilized on the surface indicates that complexing occurred. Where the originally non-immobilized component does not carry a label, complexing can be detected, for example, by using a labeled antibody specifically binding the immobilized complex.

If the candidate compound interacts with but does not bind to a particular PR0364 or PRO175 polypeptide encoded by a gene identified herein, its interaction with that polypeptide can be assayed by methods well known for detecting protein-protein interactions. Such assays include traditional approaches, such as; *e.g.*, cross-linking, co-immunoprecipitation, and co-purification through gradients or chromatographic columns. In addition, protein-protein interactions can be monitored by using a yeast-based genetic system described by Fields and co-workers (Fields and Song, Nature (London), 340: 245-246 (1989); Chien et al., Proc. Natl. Acad. Sci. USA, 88: 9578-9582 (1991)) as disclosed by Chevray and Nathans, Proc. Natl. Acad. Sci. USA, 89: 5789-5793 (1991). Many transcriptional activators, such as yeast GAL4, consist of two physically discrete modular domains, one acting as the DNA-binding domain, the other one functioning as the transcription-activation domain. The yeast expression system described in the foregoing publications (generally referred to as the "two-hybrid system") takes advantage of this property, and employs two hybrid proteins, one in which the target protein is fused to the DNA-binding domain of GAL4, and another, in which candidate activating proteins are fused to the activation domain. The expression of a GAL1-*lac*Z reporter gene under control of a GAL4-activated promoter depends on reconstitution of GAL4 activity via protein-protein interaction. Colonies containing interacting polypeptides are detected with a chromogenic substrate for β-galactosidase. A complete kit (MATCHMAKER^{™}) for identifying protein-protein interactions between two specific proteins using the two-hybrid technique is commercially available from Clontech. This system can also be extended to map protein domains involved in specific protein interactions as well as to pinpoint amino acid residues that are crucial for these interactions.

Compounds that interfere with the interaction of a gene encoding a PR0364 or PRO175 polypeptide identified herein and other intra- or extracellular components can be tested as follows: usually a reaction mixture is prepared containing the product of the gene and the intra- or extracellular component under conditions and for a time allowing for the interaction and binding of the two products. To test the ability of a candidate compound to inhibit binding, the reaction is run in the absence and in the presence of the test compound. In addition, a placebo may be added to a third reaction mixture, to serve as positive control. The binding (complex formation) between the test compound and the intra- or extracellular component present in the mixture is monitored as described hereinabove. The formation of a complex in the control reaction(s) but not in the reaction mixture containing the test compound indicates that the test compound interferes with the interaction of the test compound and its reaction partner.

If the PRO364 or PRO175 polypeptide has the ability to stimulate the proliferation of endothelial cells in the presence of the co-mitogen ConA, then one example of a screening method takes advantage of this ability. Specifically, in the proliferation assay, human umbilical vein endothelial cells are obtained and cultured in 96-well flat-bottomed culture plates (Costar, Cambridge, MA) and supplemented with a reaction mixture appropriate for facilitating proliferation of the cells, the mixture containing Con-A (Calbiochem, La Jolla, CA). Con-A and the compound to be screened are added and after incubation at 37°C, cultures are pulsed with ³⁻H-thymidine and harvested onto glass fiber filters (phD; Cambridge Technology, Watertown, MA). Mean ³⁻(H)thymidine incorporation (cpm) of triplicate cultures is determined using a liquid scintillation counter (Beckman Instruments, Irvine, CA). Significant ³⁻(H)thymidine incorporation indicates stimulation of endothelial cell proliferation.

To assay for antagonists, the assay described above is performed; however, in this assay the PR0364 or PRO175 polypeptide is added along with the compound to be screened and the ability of the compound to inhibit ³⁻(H)thymidine incorporation in the presence of the PR0364 or PRO175 polypeptide indicates that the compound is an antagonist to the PR0364 or PRO175 polypeptide. Alternatively, antagonists may be detected by combining the PR0364 or PRO175 polypeptide and a potential antagonist with membrane-bound PR0364 or PRO175 polypeptide receptors or recombinant receptors under appropriate conditions for a competitive inhibition assay. The PR0364 or PRO175 polypeptide can be labeled, such as by radioactivity, such that the number of PR0364 or PRO175 polypeptide molecules bound to the receptor can be used to determine the effectiveness of the potential antagonist. The gene encoding the receptor can be identified by numerous methods known to those of skill in the art, for example, ligand panning and FACS sorting. Coligan et al., Current Protocols in Immun., 1(2): Chapter 5 (1991). Preferably, expression cloning is employed wherein polyadenylated RNA is prepared from a cell responsive to the PRO364 or PRO175 polypeptide and a cDNA library created from this RNA is divided into pools and used to transfect COS cells or other cells that are not responsive to'the PR0364 or PRO175 polypeptide. Transfected cells that are grown on glass slides are exposed to labeled PR0364 or PRO175 polypeptide. The PR0364 or PRO175 polypeptide can be labeled by a variety of means including iodination or inclusion of a recognition site for a site-specific protein kinase. Following fixation and incubation, the slides are subjected to autoradiographic analysis. Positive pools are identified and sub-pools are prepared and re-transfected using an interactive sub-pooling and re-screening process, eventually yielding a single clone that encodes the putative receptor.

As an alternative approach for receptor identification, labeled PR0364 or PRO175 polypeptide can be photoaffiniry-linked with cell membrane or extract preparations that express the receptor molecule. Cross-linked material is resolved by PAGE and exposed to X-ray film. The labeled complex containing the receptor can be excised, resolved into peptide fragments, and subjected to protein micro-sequencing. The amino acid sequence obtained from micro- sequencing would be used to design a set of degenerate oligonucleotide probes to screen a cDNA library to identify the gene encoding the putative receptor.

In another assay for antagonists, mammalian cells or a membrane preparation expressing the receptor would be incubated with labeled PR0364 or PRO175 polypeptide in the presence of the candidate compound. The ability of the compound to enhance or block this interaction could then be measured.

The compositions useful in the treatment of cardiovascular, endothelial, and angiogenic disorders include, without limitation, antibodies, small organic and inorganic molecules, peptides, phosphopeptides, antisense and ribozyme molecules, triple-helix molecules, etc., that inhibit the expression and/or activity of the target gene product. ,

More specific examples of potential antagonists include an oligonucleotide that binds to the fusions of immunoglobulin with PR0364 polypeptide or PRO175 polypeptide and, in particular, antibodies including, without limitation, poly- and monoclonal antibodies and antibody fragments, single-chain antibodies, anti-idiotypic antibodies, and chimeric or humanized versions of such antibodies or fragments, as well as human antibodies and antibody fragments. Alternatively, a potential antagonist may be a closely related protein, for example, a mutated form of the PR0364 or PRO175 polypeptide that recognizes the receptor but imparts no effect, thereby competitively inhibiting the action of the PRO364 or PRO175 polypeptide.

Another potential PR0364 or PRO175 polypeptide antagonist is an antisense RNA or DNA construct prepared using antisense technology, where, *e.g.*, an antisense RNA or DNA molecule acts to block directly the translation of mRNA by hybridizing to targeted mRNA and preventing protein translation. Antisense technology can be used to control gene expression through triple-helix formation or antisense DNA or RNA, both of which methods are based on binding of a polynucleotide to DNA or RNA. For example, the 5' coding portion of the polynucleotide sequence, which encodes the mature PR0364 or PRO175 polypeptides herein, is used to design an antisense RNA oligonucleotide of from about 10 to 40 base pairs in length. A DNA oligonucleotide is designed to be complementary to a region of the gene involved in transcription (triple helix - see Lee et al., Nucl. Acids Res., 6: 3073 (1979); Cooney et al., Science, 241: 456 (1988); Dervan et al., Science, 251: 1360 (1991)), thereby preventing transcription and the production of the PR0364 or PRO175 polypeptide. The antisense RNA oligonucleotide hybridizes to the mRNA *in vivo* and blocks translation of the mRNA molecule into the PR0364 or PRO175 polypeptide (antisense - Okano, Neurochem., 56: 560 (1991); Oligodeoxynucleotides as Antisense Inhibitors of Gene Expression (CRC Press: Boca Raton, FL, 1988). The oligonucleotides described above can also be delivered to cells such that the antisense RNA or DNA may be expressed *in vivo* to inhibit production of the PR0364 or PRO175 polypeptide. When antisense DNA is used, oligodeoxyribonucleotides derived from the translation-initiation site, *e.g.*, between about -10 and +10 positions of the target gene nucleotide sequence, are preferred.

Potential antagonists include small molecules that bind to the active site, the receptor binding site, or growth factor or other relevant binding site of the PR0364 or PRO175 polypeptide, thereby blocking the normal biological activity of the PR0364 or PRO175 polypeptide. Examples of small molecules include, but are not limited to, small peptides or peptide-like molecules, preferably soluble peptides, and synthetic non-peptidyl organic or inorganic compounds.

Ribozymes are enzymatic RNA molecules capable of catalyzing the specific cleavage of RNA. Ribozymes act by sequence-specific hybridization to the complementary target RNA, followed by endonucleolytic cleavage. Specific ribozyme cleavage sites within a potential RNA target can be identified by known techniques. For further details see, *e.g.*, Rossi, Current Biology, 4: 469-471 (1994), and PCT publication No. WO 97/33551 (published September 18, 1997).

Nucleic acid molecules in triple-helix formation used to inhibit transcription should be single-stranded and composed of deoxynucleotides. The base composition of these oligonucleotides is designed such that it promotes triple-helix formation via Hoogsteen base-pairing rules, which generally require sizeable stretches of purines or pyrimidines on one strand of a duplex. For further details see, *e.g.*, PCT publication No. WO 97/33551, *supra.*

These small molecules can be identified by any one or more of the screening assays discussed hereinabove and/or by any other screening techniques well known for those skilled in the art.

### x. Types of Cardiovascular, Endothelial, and Angiogenic Disorders to be Treated

The PRO364 or PRO175 polypeptides or agonists or antagonists thereto, that have activity in the cardiovascular, angiogenic, and endothelial assays described herein, and/or whose gene product has been found to be localized to the cardiovascular system, are likely to have therapeutic uses in a variety of cardiovascular, endothelial, and angiogenic disorders, including systemic disorders that affect vessels, such as diabetes mellitus. Their therapeutic utility could include diseases of the arteries, capillaries, veins, and/or lymphatics. Examples of treatments hereunder include treating muscle wasting disease, treating osteoporosis, aiding in implant fixation to stimulate the growth of cells around the implant and therefore facilitate its attachment to its intended site, increasing IGF stability in tissues or in serum, if applicable, and increasing binding to the IGP receptor (since IGF has been shown in *vitro* to enhance human marrow erythroid and granulocytic progenitor cell growth).

The PR0364 or PRO175 polypeptides or agonists or antagonists thereto may also be employed to stimulate erythropoiesis or granulopolesis, to stimulate wound healing or tissue regeneration and associated therapies concerned with re-growth of tissue, such as connective tissue, skin, bone, cartilage, muscle, lung, or kidney, to promote angiogenesis, to stimulate or inhibit migration of endothelial cells, and to proliferate the growth of vascular smooth muscle and endothelial cell production. The increase in angiogenesis mediated by PR0364 or PRO175 polypeptide or antagonist would be beneficial to ischemic tissues and to collateral coronary development in the heart subsequent to coronary stenosis. Antagonists are used to inhibit the action of such polypeptides, for example, to limit the production of excess connective tissue during wound healing or pulmonary fibrosis if the PRO364 or PRO175 polypeptide promotes such production. This would include treatment of acute myocardial infarction and heart failure.

Moreover, the present invention concerns the treatment of cardiac hypertrophy, regardless of the underlying cause, by administering a therapeutically effective dose of PR0364 or PRO175 polypeptide.

If the objective is the treatment of human patients, the PRO364 or PRO175 polypeptide preferably is recombinant human PR0364 or PRO175 polypeptide (rhPR0364 or rhPRO175 polypeplide). The treatment for cardiac hypertrophy can be performed at any of its various stages, which may result from a variety of diverse pathologic conditions, including myocardial infarction, hypertension, hypertrophic cardiomyopathy, and valvular regurgitation. The treatment extends to all stages of the progression of cardiac hypertrophy, with or without structural damage of the heart muscle, regardless of the underlying cardiac disorder.

The decision of whether to use the molecule itself or an agonist thereof for any particular indication, as opposed to an antagonist to the molecule, would depend mainly on whether the molecule herein promotes cardiovascularization, genesis of endothelial cells, or angiogenesis or inhibits these conditions. For example, if the molecule promotes angiogenesis, an antagonist thereof would be useful for treatment of disorders where it is desired to limit or prevent angiogenesis. Examples of such disorders include vascular tumors such as haemangioma, tumor angiogenesis, neovascularization in the retina, choroid, or cornea, associated with diabetic retinopathy or premature infant retinopathy or macular degeneration and proliferative vitreoretinopathy, rheumatoid arthritis, Crohn's disease, atherosclerosis, ovarian hyperstimulation, psoriasis, endometriosis associated with neovascularization, restenosis subsequent to balloon angioplasty, scar tissue overproduction, for example, that seen in a keloid that forms after surgery, fibrosis after myocardial infarction, or fibrotic lesions associated with pulmonary fibrosis.

If, however, the molecule inhibits angiogenesis, it would be expected to be used directly for treatment of the above conditions.

On the other hand, if the molecule stimulates angiogenesis it would be used itself (or an agonist thereof) for indications where angiogenesis is desired such as peripheral vascular disease, hypertension, inflammatory vasculitides, Reynaud's disease and Reynaud's phenomenon, aneurysms, arterial restenosis, thrombophlebitis, lymphangitis, lymphedema, wound healing and tissue repair, ischemia reperfusion injury, angina, myocardial infarctions such as acute myocardial infarctions, chronic heart conditions, heart failure such as congestive heart failure, and osteoporosis.

If, however, the molecule inhibits angiogenesis, an antagonist thereof would be used for treatment of those conditions where angiogenesis is desired.

Specific types of diseases are described below, where the PR0364 or PRO175 polypeptide herein or antagonists thereof may serve as useful for vascular-related drug targeting or as therapeutic targets for the treatment or prevention of the disorders. Atherosclerosis is a disease characterized by accumulation of plaques of intimal thickening in arteries, due to accumulation of lipids, proliferation of smooth muscle cells, and formation of fibrous tissue within the arterial wall. The disease can affect large, medium, and small arteries in any organ. Changes in endothelial and vascular smooth muscle cell function are known to play an important role in modulating the accumulation and regression of these plaques.

Hypertension is characterized by raised vascular pressure in the systemic arterial, pulmonary arterial, or portal venous systems. Elevated pressure may result from or result in impaired endothelial function and/or vascular disease.

Inflammatory vasculitides include giant cell arteritis, Takayasu's arteritis, polyarteritis nodosa (including the microangiopathic form), Kawasaki's disease, microscopic polyangiitis, Wegener's granulomatosis, and a variety of infectious-related vascular disorders (including Henoch-Schonlein prupura). Altered endothelial cell function has been shown to be important in these diseases.

Reynaud's disease and Reynaud's phenomenon are characterized by intermittent abnormal impairment of the circulation through the extremities on exposure to cold. Altered endothelial cell function has been shown to be important in this disease.

Aneurysms are saccular or fusiform dilatations of the arterial or venous tree that are associated with altered endothelial cell and/or vascular smooth muscle cells.

Arterial restenosis (restenosis of the arterial wall) may occur following angioplasty as a result of alteration in the function and proliferation of endothelial and vascular smooth muscle cells.

Thrombophlebitis and lymphangitis are inflammatory disorders of veins and lymphatics, respectively, that may result from, and/or in, altered endothelial cell function. Similarly, lymphedema is a condition involving impaired lymphatic vessels resulting from endothelial cell function. ,

The family of benign and malignant vascular tumors are characterized by abnormal proliferation and growth of cellular elements of the vascular system. For example, lymphangiomas are benign tumors of the lymphatic system that are congenital, often cystic, malformations of the lymphatics that usually occur in newborns. Cystic tumors tend to grow into the adjacent tissue. Cystic tumors usually occur in the cervical and axillary region. They can also occur in the soft tissue of the extremities. The main symptoms are dilated, sometimes reticular, structured lymphatics and lymphocysts surrounded by connective tissue. Lymphangiomas are assumed to be caused by improperly connected embryonic lymphatics or their deficiency. The result is impaired local lymph drainage. Griener et al., Lymphology, 4: 140-144 (1971).

Another use for the PRO364 or PRO175 polypeptides herein or antagonists thereto is in the prevention of tumor angiogenesis, which involves vascularization of a tumor to enable it to growth and/or metastasize. This process is dependent on the growth of new blood vessels. Examples of neoplasms and related conditions that involve tumor angiogenesis include breast carcinomas, lung carcinomas, gastric carcinomas, esophageal carcinomas, colorectal carcinomas, liver carcinomas, ovarian carcinomas, thecomas, arrhenoblastomas, cervical carcinomas, endometrial carcinoma, endometrial hyperplasia, endometriosis, fibrosarcomas, choriocarcinoma, head and neck cancer, nasopharyngeal carcinoma, laryngeal carcinomas, hepatoblastoma, Kaposi's sarcoma, melanoma, skin carcinomas, hemangioma, cavernous hemangioma, hemangioblastoma, pancreas carcinomas, retinoblastoma, astrocytoma, glioblastoma, Schwannoma, oligodendroglioma; medulloblastoma, neuroblastomas, rhabdomyosarcoma, osteogenic sarcoma, leiomyosarcomas, urinary tract carcinomas, thyroid carcinomas, Wilm's tumor, renal cell carcinoma, prostate carcinoma, abnormal vascular proliferation associated with phakomatoses, edema (such as that associated with brain tumors), and Meigs' syndrome.

Age-related macular degeneration (AMD) is a leading cause of severe visual loss in the elderly population. The exudative form of AMD is characterized by choroidal neovascularization and retinal pigment epithelial cell detachment. Because choroidal neovascularization is associated with a dramatic worsening in prognosis, the PR0364 or PRO175 polypeptides or antagonist thereto is expected to be useful in reducing the severity of AMD.

Healing of trauma such as wound healing and tissue repair is also a targeted use for the PR0364 or PRO175 polypeptides herein or their antagonists. Formation and regression of new blood vessels is essential for tissue healing and repair. This category includes bone, cartilage, tendon, ligament, and/or nerve tissue growth or regeneration, as well as wound healing and tissue repair and replacement, and in the treatment of burns, incisions, and ulcers. A PR0364 or PRO175 polypeptide or antagonist thereof that induces cartilage and/or bone growth in circumstances where bone is not normally formed has application in the healing of bone fractures and cartilage damage or defects in humans and other animals. Such a preparation employing a PRO364 or PRO175 polypeptide or antagonist thereof may have prophylactic use in closed as well as open fracture reduction and also in the improved fixation of artificial joints. *De novo* bone formation induced by an osteogenic agent contributes to the repair of congenital, trauma-induced, or oncologic, resection-induced craniofacial defects, and also is useful in cosmetic plastic surgery.

PR0364 or PRO175 polypeptides or antagonists thereto may also be useful to promote better or faster closure of non-healing wounds, including without limitation pressure ulcers, ulcers associated with vascular insufficiency, surgical and traumatic wounds, and the like.

It is expected that a PR0364 or PRO175 polypeptide or antagonist thereto may also exhibit activity for generation or regeneration of other tissues, such as organs (including, for example, pancreas, liver, intestine, kidney, skin, or endothelium), muscle (smooth, skeletal, or cardiac), and vascular (including vascular endothelium) tissue, or for promoting the growth of cells comprising such tissues. Part of.the desired effects may be by inhibition or modulation of fibrotic scarring to allow normal tissue to regenerate.

A PR0364 or PRO175 polypeptide herein or antagonist thereto may also be useful for gut protection or regeneration and treatment of lung or liver fibrosis, reperfusion injury in various tissues, and conditions resulting from systemic cytokine damage. Also, the PR0364 or PRO175 polypeptide or antagonist thereto may be useful for promoting or inhibiting differentiation of tissues described above from precursor tissues or cells, or for inhibiting the growth of tissues described above.

A PR0364 or PRO175 polypeptide or antagonist thereto may also be used in the treatment of periodontal diseases and in other tooth-repair processes. Such agents may provide an environment to attract bone-forming cells, stimulate growth of bone-forming cells, or induce differentiation of progenitors of bone-forming cells. A PR0364 or PRO175 polypeptide herein or an antagonist thereto may also be useful in the treatment of osteoporosis or osteoarthritis, such as through stimulation of bone and/or cartilage repair or by blocking inflammation or processes of tissue destruction (collagenase activity, osteoclast activity, etc.) mediated by inflammatory processes, since blood vessels play an important role in the regulation of bone turnover and growth.

Another category of tissue regeneration activity that may be attributable to the PR0364 or PRO175 polypeptide herein or antagonist thereto is tendon/ligament formation. A protein that induces tendon/ligament-like tissue or other tissue formation in circumstances where such tissue is not normally formed has application in the healing of tendon or ligament tears, deformities, and other tendon or ligament defects in humans and other animals. Such a preparation may have prophylactic use in preventing damage to tendon or ligament tissue, as well as use in the improved fixation of tendon or ligament to bone or other tissues, and in repairing defects to tendon or ligament tissue. *De novo* tendon/ligament-like tissue formation induced by a composition of the PR0364 or PRO175 polypeptide herein or antagonist thereto contributes to the repair of congenital, trauma-induced, or other tendon or ligament defects of other origin, and is also useful in cosmetic plastic surgery for attachment or repair of tendons or ligaments. The compositions herein may provide an environment to attract tendon- or ligament-forming cells, stimulate growth of tendon- or ligament-forming cells, induce differentiation of progenitors of tendon- or ligament-forming cells, or induce growth of tendon/ligament cells or progenitors ex *vivo* for return in *vivo* to effect tissue repair. The compositions herein may also be useful in the treatment of tendonitis, carpal tunnel syndrome, and other tendon or ligament defects. The compositions may also include an appropriate matrix and/or sequestering agent as a carrier as is well known in the art.

The PR0364 or PRO175 polypeptide or its antagonist may also be useful for proliferation of neural cells and for regeneration of nerve and brain tissue, *i.e.,* for the treatment of central and peripheral nervous system disease and neuropathies, as well as mechanical and traumatic disorders, that involve degeneration, death, or trauma to neural cells or nerve tissue. More specifically, a PRO364 or PRO175 polypeptide or its antagonist may be used in the treatment of diseases of the peripheral nervous system, such as peripheral nerve injuries, peripheral neuropathy and localized neuropathies, and central nervous system diseases, such as Alzheimer's, Parkinson's disease, Huntington's disease, amyotrophic lateral sclerosis, and Shy-Drager syndrome. Further conditions that may be treated in accordance with the present invention include mechanical and traumatic disorders, such as spinal cord disorders, head trauma, and cerebrovascular diseases such as stroke. Peripheral neuropathies resulting from chemotherapy or other medical therapies may also be treatable using a PR0364 or PRO175 polypeptide herein or antagonist thereto.

Ischemia-reperfusion injury is another indication. Endothelial cell dysfunction may be important in both the initiation of, and in regulation of the sequelae of events that occur following ischemia-reperfusion injury.

Rheumatoid arthritis is a further indication. Blood vessel growth and targeting of inflammatory cells through the vasculature is an important component in the pathogenesis of rheumatoid and sero-negative forms of arthritis.

PR0364 or PRO175 polypeptide or its antagonist may also be administered prophylactically to patients with cardiac hypertrophy, to prevent the progression of the condition, and avoid sudden death, including death of asymptomatic patients. Such preventative therapy is particularly warranted in the case of patients diagnosed with massive left ventricular cardiac hypertrophy (a maximal wall thickness of 35 mm or more in adults, or a comparable value in children), or in instances when the hemodynamic burden on the heart is particularly strong.

PR0364 or PRO175 polypeptide or its antagonist may also be useful in the management of atrial fibrillation, which develops in a substantial portion of patients diagnosed with hypertrophic cardiomyopathy. Further indications include angina, myocardial infarctions such as acute myocardial infarctions, and heart failure such as congestive heart failure. Additional non-neoplastic conditions include psoriasis, diabetic and other proliferative retinopathies including retinopathy of prematurity, retrolental fibroplasia, neovascular glaucoma, thyroid hyperplasias (including Grave's disease), corneal and other tissue transplantation, chronic inflammation, lung inflammation, nephrotic syndrome, preeclampsia, ascites, pericardial effusion (such as that associated with pericarditis), and pleural effusion.

In view of the above, the PR0364 or PRO175 polypeptides or agonists or antagonists thereof described herein, which are shown to alter or impact endothelial cell function, proliferation, and/or form, are likely to play an important role in the etiology and pathogenesis of many or all of the disorders noted above, and as such can serve as therapeutic targets to augment or inhibit these processes or for vascular-related drug targeting in these disorders.

### xi. Administration Protocols, Schedules, Doses, and Formulations

The molecules herein and agonists and antagonists thereto are pharmaceutically useful as a prophylactic and therapeutic agent for various disorders and diseases as set forth above.

Therapeutic compositions of the PRO364 or PRO175 polypeptides or agonists or antagonists are prepared for storage by mixing the desired molecule having the appropriate degree of purity with optional pharmaceutically acceptable carriers, excipients, or stabilizers (Remington's Pharmaceutical Sciences, 16th edition, Osol, A. ed. (1980)), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (*e.g.*, Zn-protein complexes); and/or non-ionic surfactants such as TWEEN^{™}, PLURONICS^{™} or polyethylene glycol (PEG).

Additional examples of such carriers include ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts, or electrolytes such as protamine sulfate, disodium hydrogen phosphate, potassium hydrogen phosphate, sodium chloride, zinc salts, colloidal silica, magnesium trisilicate, polyvinyl pyrrolidone, cellulose-based substances, and polyethylene glycol. Carriers for topical or gel-based forms of antagonist include polysaccharides such as sodium carboxymethylcellulose or methylcellulose, polyvinylpyrrolidone, polyacrylates, polyoxyethylene-polyoxypropylene-block polymers, polyethylene glycol, and wood wax alcohols. For all administrations, conventional depot forms are suitably used. Such forms include, for example, microcapsules, nano-capsules, liposomes, plasters, inhalation forms, nose sprays, sublingual tablets, and sustained-release preparations. The PR0230, PR0216, or PR0302 polypeptides or agonists or antagonists will typically be formulated in such vehicles at a concentration of about 0.1 mg/ml to 100 mg/ml.

Another formulation comprises incorporating a PRO364 or PRO175 polypeptide or antagonist thereof into formed articles. Such articles can be used in modulating endothelial cell growth and angiogenesis. In addition, tumor invasion and metastasis may be modulated with these articles.

PRO364 or PRO175 polypeptide or antagonist to be used for *in vivo* administration must be sterile. This is readily accomplished by filtration through sterile filtration membranes, prior to or following lyophilization and reconstitution. PRO364 or PRO175 polypeptide ordinarily will be stored in lyophilized form or in solution if administered systemically. If in lyophilized form, PRO364 or PRO175 polypeptide or antagonist thereto is typically formulated in combination with other ingredients for reconstitution with an appropriate diluent at the time for use. An example of a liquid formulation of PRO364 or PRO175 polypeptide or antagonist is a sterile, clear, colorless unpreserved solution filled in a single-dose vial for subcutaneous injection. Preserved pharmaceutical compositions suitable for repeated use may contain, for example, depending mainly on the indication and type of polypeptide:
a) PR0364 or PRO175 polypeptide or agonist or antagonist thereto;
b) a buffer capable of maintaining the pH in a range of maximum stability of the polypeptide or other molecule in solution, preferably about 4-8;
c) a detergent/surfactant primarily to stabilize the polypeptide or molecule against agitation-induced aggregation;
d) an isotonifier;
e) a preservative selected from the group of phenol, benzyl alcohol and a benzethonium halide, *e.g.,* chloride; and
f) water.

If the detergent employed is non-ionic, it may, for example, be polysorbates (*e.g.,* POLYSORBATE^{™} (TWEEN^{™}) 20, 80, etc.) or poloxamers (*e.g.*, POLOXAMER^{™} 188). The use of non-ionic surfactants permits the formulation to be exposed to shear surface stresses without causing denaturation of the polypeptide. Further, such surfactant-containing formulations may be employed in aerosol devices such as those used in a pulmonary dosing, and needleless jet injector guns (see, *e.g.*, EP 257,956).

An isotonifier may be present to ensure isotonicity of a liquid composition of the PR0364 or PRO175 polypeptide or antagonist thereto, and includes polyhydric sugar alcohols, preferably trihydric or higher sugar alcohols, such as glycerin, erythritol, arabitol, xylitol, sorbitol, and mannitol. These sugar alcohols can be used alone or in combination. Alternatively, sodium chloride or other appropriate inorganic salts may be used to render the solutions isotonic.

The buffer may, for example, be an acetate, citrate, succinate, or phosphate buffer depending on the pH desired. The pH of one type of liquid formulation of this invention is buffered in the range of about 4 to 8, preferably about physiological pH.

The preservatives phenol, benzyl alcohol and benzethonium halides, *e.g.*, chloride, are known antimicrobial agents that may be employed.

Therapeutic PR0364 or PRO175 polypeptide compositions generally are placed into a container having a sterile access port, for example, an intravenous solution bag or vial having a stopper pierceable by a hypodermic injection needle. The formulations are preferably administered as repeated intravenous (i.v.), subcutaneous (s.c.), or intramuscular (i.m.) injections, or as aerosol formulations suitable for intranasal or intrapulmonary delivery (for intrapulmonary delivery see, *e.g.,* EP 257,956).

PR0364 or PRO175 polypeptide can also be administered in the form of sustained-released preparations. Suitable examples of sustained-release preparations include semipermeable matrices of solid hydrophobic polymers containing the protein, which matrices are in the form of shaped articles, *e.g.*, films, or microcapsules. Examples of sustained-release matrices include polyesters, hydrogels (*e.g.*, poly(2-hydroxyethyl-methacrylate) as described by Langer et al., J. Biomed. Mater. Res., 15: 167-277 (1981) and Langer, Chem. Tech., 12: 98-105 (1982) or poly(vinylalcohol)), polylactides (U.S. Patent No. 3,773,919, EP 58.481), copolymers of L-glutamic acid and gamma ethyl-L-glutamate (Sidman et al., Biopolymers, 22: 547-556 (1983)), non-degradable ethylene-vinyl acetate (Langer *et al., supra),* degradable lactic acid-glycolic acid copolymers such as the Lupron Depot^{™} (injectable microspheres composed of lactic acid-glycolic acid copolymer and leuprolide acetate), and poly-D-(-)-3-hydroxybutyric acid (EP 133,988).

While polymers such as ethylene-vinyl acetate and lactic acid-glycolic acid enable release of molecules for over 100 days, certain hydrogels release proteins for shorter time periods. When encapsulated proteins remain in the body for a long time, they may denature or aggregate as a result of exposure to moisture at 37°C, resulting in a loss of biological activity and possible changes in immunogenicity. Rational strategies can be devised for protein stabilization depending on the mechanism involved. For example, if the aggregation mechanism is discovered to be intermolecular S-S bond formation through thio-disulfide interchange, stabilization may be achieved by modifying sulfhydryl residues, lyophilizing from acidic solutions, controlling moisture content, using appropriate additives, and developing specific polymer matrix compositions.

Sustained-release PR0364 or PRO175 polypeptide compositions also include liposomally entrapped PR0364 or PRO175 polypeptide. Liposomes containing PR0364 or PRO175 polypeptide are prepared by methods known *per se:* DE 3,218,121; Epstein et al., Proc. Natl. Acad. Sci. USA, 82: 3688-3692 (1985); Hwang et al., Proc. Natl. Acad. Sci. USA, 77: 4030-4034 (1980); EP 52,322; EP 36,676; EP 88,046; EP 143,949; EP 142,641; Japanese patent application 83-118008; U.S. Patent Nos. 4,485,045 and 4,544,545; and EP 102,324. Ordinarily the liposomes are of the small (about 200-800 Angstroms) unilamellar type in which the lipid content is greater than about 30 mol. % cholesterol, the selected proportion being adjusted for the optimal therapy.

The therapeutically effective dose of PR0364 or PRO175 polypeptide or antagonist thereto will, of course, vary depending on such factors as the pathological condition to be treated (including prevention), the method of administration, the type of compound being used for treatment, any co-therapy involved, the patient's age, weight, general medical condition, medical history, etc., and its determination is well within the skill of a practicing physician. Accordingly, it will be necessary for the therapist to titer the dosage and modify the route of administration as required to obtain the maximal therapeutic effect. If the PR0364 or PRO175 polypeptide has a narrow host range, for the treatment of human patients formulations comprising human PRO364, or human PRO175 polypeptide, more preferably native-sequence human PR0364, or human PRO175 polypeptide, are preferred. The clinician will administer PR0364 or PRO175 polypeptide until a dosage is reached that achieves the desired effect for treatment of the condition in question. For example, if the objective is the treatment of CHF, the amount would be one that inhibits the progressive cardiac hypertrophy associated with this condition. The progress of this therapy is easily monitored by echo cardiography. Similarly, in patients with hypertrophic cardiomyopathy, PR0364 or PRO175 polypeptide can be administered on an empirical basis.

With the above guidelines, the effective dose generally is within the range of from about 0.001 to about 1.0 mg/kg, more preferably about 0.01-1 mg/kg, most preferably about 0.01-0.1 mg/kg.

For non-oral use in treating human adult hypertension, it is advantageous to administer PR0364 or PRO175 polypeptide in the form of an injection at about 0.01 to 50 mg, preferably about 0.05 to 20 mg, most preferably 1 to 20 mg, per kg body weight. 1 to 3 times daily by intravenous injection. For oral administration, a molecule based on the PR0364 or PRO175 polypeptide is preferably, administered at about 5 mg to 1 g, preferably about 10 to 100 mg, per kg body weight, 1 to 3 times daily. It should be appreciated that endotoxin contamination should be kept minimally at a safe level, for example, less than 0.5 ng/mg protein. Moreover, for human administration, the formulations preferably meet sterility, pyrogenicity, general safety, and purity as required by FDA Office and Biologics standards.

The dosage regimen of a pharmaceutical composition containing PR0364 or PRO175 polypeptide to be used in tissue regeneration will be determined by the attending physician considering various factors that modify the action of the polypeptides, *e.g.*, amount of tissue weight desired to be formed, the site of damage, the condition of the damaged tissue, the size of a wound, type of damaged tissue (*e.g.*, bone), the patient's age, sex, and diet, the severity of any infection, time of administration, and other clinical factors. The dosage may vary with the type of matrix used in the reconstitution and with inclusion of other proteins in the pharmaceutical composition. For example, the addition of other known growth factors, such as IGF-I, to the final composition may also affect the dosage. Progress can be monitored by periodic assessment of tissue/bone growth and/or repair, for example, X-rays, histomorphometric determinations, and tetracycline labeling.

The route of PRO364 or PRO175 polypeptide or antagonist or agonist administration is in accord with known methods, *e.g.*, by injection or infusion by intravenous, intramuscular, intracerebral, intraperitoneal, intracerobrospinal, subcutaneous, intraocular, intraarticular, intrasynovial, intrathecal, oral, topical, or inhalation routes, or by sustained-release systems as noted below. The PR0364 or PRO175 polypeptide or antagonists thereof also are suitably administered by intratumoral, peritumoral, intralesional, or perilesional routes, to exert local as well as systemic therapeutic effects. The intraperitoneal route is expected to be particularly useful, for example, in the treatment of ovarian tumors.

If a peptide or small molecule is employed as an antagonist or agonist, it is preferably administered orally or non-orally in the form of a liquid or solid to mammals.

Examples of pharmacologically acceptable salts of molecules that form salts and are useful hereunder include alkali metal salts (*e.g.*, sodium salt, potassium salt), alkaline earth metal salts (*e.g.*, calcium salt, magnesium salt), ammonium salts, organic base salts (*e.g.*, pyridine salt, triethylamine salt), inorganic acid salts (*e.g.*, hydrochloride, sulfate, nitrate), and salts of organic acid *(e.g.,* acetate, oxalate, p-toluenesulfonate).

For compositions herein that are useful for bone, cartilage, tendon, or ligament regeneration, the therapeutic method includes administering the composition topically, systemically, or locally as an implant or device. When administered, the therapeutic composition for use is in a pyrogen-free, physiologically acceptable form. Further, the composition may desirably be encapsulated or injected in a viscous form for delivery to the site of bone, cartilage, or tissue damage. Topical administration may be suitable for wound healing and tissue repair. Preferably, for bone and/or cartilage formation, the composition would include a matrix capable of delivering the protein-containing composition to the site of bone and/or cartilage damage, providing a structure for the developing bone and cartilage and preferably capable of being resorbed into the body. Such matrices may be formed of materials presently in use for other implanted medical applications.

The choice of matrix material is based on biocompatibility, biodegradabiity, mechanical properties, cosmetic appearance, and interface properties. The particular application of the compositions will define the appropriate formulation. Potential matrices for the compositions may be biodegradable and chemically defined calcium sulfate, tricalcium phosphate, hydroxyapatite, polylactic acid, polyglycolic acid, and polyanhydrides. Other potential materials are biodegradable and biologically well-defined, such as bone or dermal collagen. Further matrices are comprised of pure proteins or extracellular matrix components. Other potential matrices are nonbiodegradable and chemically defined, such as sintered hydroxyapatite, bioglass, aluminates, or other ceramics. Matrices may be comprised of combinations of any of the above-mentioned types of material, such as polylactic acid and hydroxyapatite or collagen and tricalcium phosphate. The bioceramics may be altered in composition, such as in calcium-aluminate-phosphate and processing to alter pore size, particle size, particle shape, and biodegradability.

One specific embodiment is a 50:50 (mole weight) copolymer of lactic acid and glycolic acid in the form of porous particles having diameters ranging from 150 to 800 microns. In some applications, it will be useful to utilize a sequestering agent, such as carboxymethyl cellulose or autologous blood clot, to prevent the polypeptide compositions from disassociating from the matrix.

One suitable family of sequestering agents is cellulosic materials such as alkylcelluloses (including hydroxyalkylcelluloses), including methylcellulose, ethylcellulose, hydoxyethylcellulose, hydroxypropylcellulose, hydroxypropylmethylcellulose, and carboxymethylcellulose, one preferred being cationic salts of carboxymethylcellulose (CMC). Other preferred sequestering agents include hyaluronic acid, sodium alginate, poly(ethylene glycol), polyoxyethylene oxide, carboxyvinyl polymer, and poly(vinyl alcohol). The amount of sequestering agent useful herein is 0.5-20 wt%, preferably 1-10 wt%, based on total formulation weight, which represents the amount necessary to prevent desorption of the polypeptide (or its antagonist) from the polymer matrix and to provide appropriate handling of the composition, yet not so much that the progenitor cells are prevented from infiltrating the matrix, thereby providing the polypeptide (or its antagonist) the opportunity to assist the osteogenic activity of the progenitor cells.

### xii. Combination Therapies

The effectiveness of the PR0364 or PRO175 polypeptide or an agonist or antagonist thereof in preventing or treating the disorder in question may be improved by administering the active agent serially or in combination with another agent that is effective for those purposes, either in the same composition or as separate compositions.

For example, for treatment of cardiac hypertrophy, PR0364 or PRO175 polypeptide therapy can be combined with the administration of inhibitors of known cardiac myocyte hypertrophy factors, *e.g.*, inhibitors of α-adrenergic agonists such as phenylephrine; endothelin-1 inhibitors such as BOSENTAN^{™} and MOXONODIN^{™}; inhibitors to CT-1 (US Pat. No. 5,679,545); inhibitors to LIF; ACE inhibitors; des-aspartate-angiotensin I inhibitors (U.S. Pat. No. 5,773,415), and angiotensin II inhibitors.

For treatment of cardiac hypertrophy associated with hypertension, PR0364 or PRO175 polypeptide can be administered in combination with β-adrenergic receptor blocking agents, *e.g.*, propranolol, timolol, tertalolol, carteolol, nadolol, betaxolol, penbutolol, acetobutolol, atenolol, metoprolol, or carvedilol; ACE inhibitors, *e.g*., quinapril, captopril, enalapril, ramipril, benazepril, fosinopril, or lisinopril: diuretics, *e.g.*, chorothiazide, hydrochlorothiazide, hydroflumethazide, methylchlothiazide, benzthiazide, dichlorphenamide, acetazolamide, or indapamide; and/or calcium channel blockers, *e.g.*, diltiazem, nifedipine, verapamil, or nicardipine. Pharmaceutical compositions comprising the therapeutic agents identified herein by their generic names are commercially available, and are to be administered following the manufacturers' instructions for dosage, administration, adverse effects, contraindications, etc. See, *e.g.*, Physicians' Desk Reference (Medical Economics Data Production Co.: Montvale, N.J., 1997), 51th Edition.

Preferred candidates for combination therapy in the treatment of hypertrophic cardiomyopathy are β-adrenergic-blocking drugs (*e.g.*, propranolol, timolol, tertalolol, carteolol, nadolol, betaxolol, penbutolol, acetobutolol, atenolol, metoprolol, or carvedilol), verapamil, difedipine, or diltiazem. Treatment of hypertrophy associated with high blood pressure may require the use of antihypertensive drug therapy, using calcium channel blockers. *e.g.*, diltiazem, nifedipine, verapamil, or nicardipine: β-adrenergic blocking agents; diuretics, *e.g.*, chorothiazide, hydrochlorothiazide, hydroflumethazide, methylehlothiazide, benzthiazide, dichlorphenamide, acetazolamide, or indapamide; and/or ACE-inhibitors, *e.g.*, quinapril, captopril, enalapril, ramipril, benazepril, fosinopril, or lisinopril.

For other indications, PR0364 or PRO175 polypeptides or their antagonists may be combined with other agents beneficial to the treatment of the bone and/or cartilage defect, wound, or tissue in question. These agents include various growth factors such as EGF, PDGF, TGF-α or TGF-β, IGF, FGF, and CTGF.

In addition, PR0364 or PRO175 polypeptides or their antagonists used to treat cancer may be combined with cytotoxic, chemotherapeutic, or growth-inhibitory agents as identified above. Also, for cancer treatment, the PRO364 or PRO175 polypeptide or antagonist thereof is suitably administered serially or in combination with radiological treatments, whether involving irradiation or administration of radioactive substances.

The effective amounts of the therapeutic agents administered in combination with PR0364 or PRO175 polypeptide or antagonist thereof will be at the physician's or veterinarian's discretion. Dosage administration and adjustment is done to achieve maximal management of the conditions to be treated. For example, for treating hypertension, these amounts ideally take into account use of diuretics or digitalis, and conditions such as hyper-or hypotension, renal impairment, etc. The dose will additionally depend on such factors as the type of the therapeutic agent to be used and the specific patient being treated. Typically, the amount employed will be the same dose as that used, if the given therapeutic agent is administered without PR0364 or PRO175 polypeptide.

### xiii. Articles of Manufacture

An article of manufacture such as a kit containing PR0364 or PRO175 polypeptide or antagonists thereof useful for the diagnosis or treatment of the disorders described above comprises at least a container and a label. Suitable containers include, for example, bottles, vials, syringes, and test tubes. The containers may be formed from a variety of materials such as glass or plastic. The container holds a composition that is effective for diagnosing or treating the condition and may have a sterile access port (for example, the container may be an intravenous solution bag or a vial having a stopper pierceable by a hypodermic injection needle). The active agent in the composition is the PR0364 or PRO175 polypeptide or an agonist or antagonist thereto. The label on, or associated with, the container indicates that the composition is used for diagnosing or treating the condition of choice. The article of manufacture may further comprise a second container comprising a pharmaceutically-acceptable buffer, such as phosphate-buffered saline, Ringer's solution, and dextrose solution. It may further include other materials desirable from a commercial and user standpoint, including other buffers, diluents, filters, needles, syringes, and package inserts with instructions for use. The article of manufacture may also comprise a second or third container with another active agent as described above.

### C. Antibodies

Some of the most promising drug candidates according to the present invention are antibodies and antibody fragments that may inhibit the production or the gene product of the genes identified herein and/or reduce the activity of the gene products.

### i. Polyclonal Antibodies

Methods of preparing polyclonal antibodies are known to the skilled artisan. Polyclonal antibodies can be raised in a mammal, for example, by one or more injections of an immunizing agent and, if desired, an adjuvant. Typically, the immunizing agent and/or adjuvant will be injected in the mammal by multiple subcutaneous or intraperitoneal injections. The immunizing agent may include the PR0364 or PRO175 polypeptide or a fusion protein thereof. It may be useful to conjugate the immunizing agent to a protein known to be immunogenic in the mammal being immunized. Examples of such immunogenic proteins include, but are not limited to keyhole limpet hemocyanin, serum albumin. bovine thyroglobulin, and soybean trypsin inhibitor. Examples of adjuvants that may be employed include Freund's complete adjuvant and MPL-TDM adjuvant (monophosphoryl Lipid A or synthetic trehalose dicorynomycolate). The immunization protocol may be selected by one skilled in the art without undue experimentation.

### ii. Monoclonal Antibodies

The anti- PR0364 or -PRO175 antibodies may, alternatively, be monoclonal antibodies. Monoclonal antibodies may be prepared using hybridoma methods, such as those described by Kohler and Milstein, Nature, 256:495 (1975). In a hybridoma method, a mouse, hamster, or other appropriate host animal is typically immunized with an immunizing agent to elicit lymphocytes that produce or are capable of producing antibodies that will specifically bind to the immunizing agent. Alternatively, the lymphocytes may be immunized *in vitro.*

The immunizing agent will typically include the PR0364 or PRO175 polypeptide or a fusion protein thereof. Generally, either peripheral blood lymphocytes ("PBLs") are used if cells of human origin are desired, or spleen cells or lymph node cells are used if non-human mammalian sources are desired. The lymphocytes are then fused with an immortalized cell line using a suitable fusing agent, such as polyethylene glycol, to form a hybridoma cell. Goding, Monoclonal Antibodies: Principles and Practice (New York: Academic Press, 1986), pp. 59-103. Immortalized cell lines are usually transformed mammalian cells, particularly myeloma cells of rodent, bovine, and human origin. Usually, rat or mouse myeloma cell lines are employed. The hybridoma cells may be cultured in a suitable culture medium that preferably contains one or more substances that inhibit the growth or survival of the unfused, immortalized cells. For example, if the parental cells lack the enzyme hypoxanthine guanine phosphoribosyl transferase (HGPRT or HPRT), the culture medium for the hybridomas typically will include hypoxanthine, aminopterin, and thymidine ("HAT medium"), which substances prevent the growth of HGPRT-deficient cells.

Preferred immortalized cell lines are those that fuse efficiently, support stable high-level expression of antibody by the selected antibody-producing cells, and are sensitive to a medium such as HAT medium. More preferred immortalized cell lines are murine myeloma lines, which can be obtained, for instance, from the Salk Institute Cell Distribution Center, San Diego, California and the American Type Culture Collection, Manassas, Virginia. Human myeloma and mouse-human heteromyeloma cell lines also have been described for the production of human monoclonal antibodies. Kozbor, J. lmmunol., 133:3001 (1984); Brodeur et al., Monoclonal Antibody Production Techniques and Applications (Marcel Dekker, Inc.: New York, 1987) pp. 51-63.

The culture medium in which the hybridoma cells are cultured can then be assayed for the presence of monoclonal antibodies directed against PRO364 or PRO175. Preferably, the binding specificity of monoclonal antibodies produced by the hybridoma cells is determined by immunoprecipitation or by an *in vitro* binding assay, such as radioimmunoassay (RIA) or enzyme-linked immunoabsorbent assay (ELISA). Such techniques and assays are known in the art. The binding affinity of the monoclonal antibody can, for example, be determined by the Scatchard analysis of Munson and Pollard, Anal. Biochem., 107:220 (1980).

After the desired hybridoma cells are identified, the clones may be subcloned by limiting dilution procedures and grown by standard methods. Goding, *supra.* Suitable culture media for this purpose include, for example, Dulbecco's Modified Eagle's Medium and RPMI-1640 medium. Alternatively, the hybridoma cells may be grown *in vivo* as ascites in a mammal.

The monoclonal antibodies secreted by the subclones may be isolated or purified from the culture medium or ascites fluid by conventional immunoglobulin purification procedures such as, for example, protein A-Sepharose, hydroxylapatite chromatography, gel electrophoresis, dialysis, or affinity chromatography.

The monoclonal antibodies may also be made by recombinant DNA methods, such as those described in U.S. Patent No. 4,816,567. DNA encoding the monoclonal antibodies of the invention can be readily isolated and sequenced using conventional procedures (*e*.*g*., by using oligonucleotide probes that are capable of binding specifically to genes encoding the heavy and light chains of murine antibodies). The hybridoma cells of the invention serve as a preferred source of such DNA. Once isolated, the DNA may be placed into expression vectors, which are then transfected into host cells such as simian COS cells, Chinese hamster ovary (CHO) cells, or myeloma cells that do not otherwise produce immunoglobulin protein, to obtain the synthesis of monoclonal antibodies in the recombinant host cells. The DNA also may be modified, for example, by substituting the coding sequence for human heavy- and light-chain constant domains in place of the homologous murine sequences (U.S. Patent No. 4,816,567; Morrison *et al., supra)* or by covalently joining to the immunoglobulin coding sequence all or part of the coding sequence for a non-immunoglobulin polypeptide. Such a non-immunoglobulin polypeptide can be substituted for the constant domains of an antibody of the invention, or can be substituted for the variable domains of one antigen-combining site of an antibody of the invention to create a chimeric bivalent antibody.

The antibodies may be monovalent antibodies. Methods for preparing monovalent antibodies are well known in the art. For example, one method involves recombinant expression of immunoglobulin light chain and modified heavy chain. The heavy chain is truncated generally at any point in the Fc region so as to prevent heavy-chain crosslinking. Alternatively, the relevant cysteine residues are substituted with another amino acid residue or are deleted so as to prevent crosslinking.

*In vitro* methods are also suitable for preparing monovalent antibodies. Digestion of antibodies to produce fragments thereof, particularly Fab fragments, can be accomplished using routine techniques known in the art.

### iii. Human and Humanized Antibodies

The anti- PR0364 or -PRO175 antibodies may further comprise humanized antibodies or human antibodies. Humanized forms of non-human (*e.g.*, murine) antibodies are chimeric immunoglobulins, immunoglobulin chains, or fragments thereof (such as Fv, Fab, Fab', F(ab')_{2.} or other antigen-binding subsequences of antibodies) that contain minimal sequence derived from non-human immunoglobulin. Humanized antibodies include human immunoglobulins (recipient antibody) in which residues from a CDR of the recipient are replaced by residues from a CDR of a non-human species (donor antibody) such as mouse, rat, or rabbit having the desired specificity, affinity, and capacity. In some instances, Fv framework residues of the human immunoglobulin are replaced by corresponding non-human residues. Humanized antibodies may also comprise residues that are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin, and all or substantially all of the FR regions are those of a human immunoglobulin consensus sequence. The humanized antibody preferably also will comprise at least a portion of an immunoglobulin constant region (Fc), typically that of a human immunoglobulin. Jones et al., Nature, 321: 522-525 (1986); Riechmann et al., Nature, 332: 323-329 (1988); Presta, Curr. Op. Struct. Biol., 2:593-596 (1992).

Methods for humanizing non-human antibodies are well known in the art. Generally, a humanized antibody has one or more amino acid residues introduced into it from a source that is non-human. These non-human amino acid residues are often referred to as "import" residues, which are typically taken from an "import" variable domain, Humanization can be essentially performed following the method of Winter and co-workers (Jones et al.. Nature, 321: 522-525 (1986): Riechmann et al., Nature, 332: 323-327 (1988): Verhoeyen et al., Science, 239: 1534-1536 (1988)), by substituting rodent CDRs or CDR sequences for the corresponding sequences of a human antibody. Accordingly, such "humanized" antibodies are chimeric antibodies (U.S. Patent No. 4,816.567), wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

Human antibodies can also be produced using various techniques known in the art, including phage display libraries. Hoogenboom and Winter, J. Mol. Biol., 227: 381 (1991); Marks et al., J. Mol. Biol., 222: 581 (1991). The techniques of Cole *et al.* and Boerner *et al.* are also available for the preparation of human monoclonal antibodies. Cole et al., Monoclonal Antibodies and Cancer Therapy, Alan R. Liss, p. 77 (1985) and Boerner et al., J. Immunol., 147(1): 86-95 (1991). Similarly, human antibodies can be made by introducing human immunoglobulin loci into transgenic animals, *e.g.*, mice in which the endogenous immunoglobulin genes have been partially or completely inactivated. Upon challenge, human antibody production is observed that closely resembles that seen in humans in all respects, including gene rearrangement, assembly, and antibody repertoire. This approach is described, for example, in U.S. Patent Nos. 5,545,807; 5,545,806; 5,569,825 ; 5,625,126; 5,633,425; and 5,661,016, and in the following scientific publications: Marks et al., Bio/Technology, 10: 779-783 (1992); Lonberg et al., Nature, 368: 856-859 (1994); Morrison, Nature, 368: 812-813 (1994); Fishwild et al., Nature Biotechnology, 14: 845-851 (1996); Neuberger, Nature Biotechnology, 14: 826 (1996); Lonberg and Huszar, Intern. Rev. Immunol., 13: 65-93 (1995).

### iv. Bispecific Antibodies

Bispecific antibodies are monoclonal, preferably human or humanized, antibodies that have binding specificities for at least two different antigens. In the present case, one of the binding specificities is for the PR0364 or PRO175, the other one is for any other antigen, and preferably for a cell-surface protein or receptor or receptor subunit.

Methods for making bispecific antibodies are known in the art. Traditionally, the recombinant production of bispecific antibodies is based on the co-expression of two immunoglobulin heavy-chain/light-chain pairs, where the two heavy chains have different specificities. Milstein and Cuello, Nature, 305: 537-539 (1983). Because of the random assortment of immunoglobulin heavy and light chains, these hybridomas (quadromas) produce a potential mixture of ten different antibody molecules, of which only one has the correct bispecific structure. The purification of the correct molecule is usually accomplished by affinity chromatography steps. Similar procedures are disclosed in WO 93/08829, published 13 May 1993, and in Traunecker et al., EMBO J., 10: 3655-3659 (1991).

Antibody variable domains with the desired binding specificities (antibody-antigen combining sites) can be fused to immunoglobulin constant- domain sequences. The fusion preferably is with an immunoglobulin heavy-chain constant domain, comprising at least part of the hinge, CH2, and CH3 regions. It is preferred to have the first heavy-chain constant region (CH1) containing the site necessary for light-chain binding present in at least one of the fusions. DNAs encoding the immunoglobulin heavy-chain fusions and, if desired, the immunoglobulin light chain, are inserted into separate expression vectors, and are co-transfected into a suitable host organism. For further details of generating bispecific antibodies, see, for example, Suresh et al., Methods in Enzymology, 121: 210 (1986).

### v. Heteroconjugate Antibodies

Heteroconjugate antibodies are composed of two covalently joined antibodies. Such antibodies have, for example, been proposed to target immune-system cells to unwanted cells (U.S. Patent No. 4.676.980), and for treatment of HIV infection. WO 91/00360; WO 92/200373; EP 03089. It is contemplated that the antibodies may be prepared *in vitro* using known methods in synthetic protein chemistry, including those involving crosslinking agents. For example, immunotoxins may be constructed using a disulfide-exchange reaction or by forming a thioether bond. Examples of suitable reagents for this purpose include iminothiolate and methyl-4-mercaptobutyrimidate and those disclosed, for example, in U.S. Patent No. 4.676,980.

### vi. Effector Function Engineering

lt may be desirable to modify the antibody of the invention with respect to effector function, so as to enhance, *e.g.*, the effectiveness of the antibody in treating cancer. For example, cysteine residue(s) may be introduced into the Fc region, thereby allowing interchain disulfide bond formation in this region. The homodimeric antibody thus generated may have improved internalization capability and/or increased complement-mediated cell killing and antibody-dependent cellular cytotoxicity (ADCC). See Caron et al., J. Exp Med. 176: 1191-1195 (1992) and Shopes, J. Immunol., 148: 2918-2922 (1992). Homodimeric antibodies with enhanced anti-tumor activity may also be prepared using heterobifunctional cross-linkers as described in Wolff et al. Cancer Research, 53: 2560-2565 (1993). Alternatively, an antibody can be engineered that has dual Fc regions and may thereby have enhanced complement lysis and ADCC capabilities. See Stevenson et al., AntiCancer Drug Design 3: 219-230 (1989).

### vii. Immunoconjugates

The invention also pertains to immunoconjugates comprising an antibody conjugated to a cytotoxic agent such as a chemotherapeutic agent, toxin (*e.g.*, an enzymatically active toxin of bacterial, fungal, plant, or animal origin, or fragments thereof), or a radioactive isotope (i.e., a radioconjugate).

Chemotherapeutic agents useful in the generation of such immunoconjugates have been described above. Enzymatically active toxins and fragments thereof that can be used include diphtheria A chain, nonbinding active fragments of diphtheria toxin, exotoxin A chain (from *Pseudomonas aeruginosa),* ricin A chain, abrin A chain, modeccin A chain, alpha-sarcin, *Aleurites fordii* proteins, dianthin proteins, *Phytolaca americana* proteins (PAPI, PAPII, and PAP-S), momordica charantia inhibitor, curcin, crotin, sapaonaria officinalis inhibitor, gelonin, mitogellin, restrictocin, phenomycin, enomycin, and the tricothecenes. A variety of radionuclides are available for the production of radioconjugated antibodies. Examples include ²¹²Bi, ¹³¹I, ¹³¹In, ⁹⁰Y, and ¹⁸⁶Re.

Conjugates of the antibody and cytotoxic agent are made using a variety of bifunctional protein-coupling agents such as N-succinimidyl-3-(2-pyridyldithiol) propionate (SPDP), iminothiolane (IT), bifunctional derivatives of imidoesters (such as dimethyl adipimidate HCL), active esters (such as disuccinimidyl suberate), aldehydes (such as glutareldehyde), bis-azido compounds (such as bis (p-azidobenzoyl) hexanediamine), bis-diazonium derivatives (such as bis-(p-diazoniumbenzoyl)-ethylenediamine), diisocyanates (such as tolyene 2,6-diisocyanate), and bis-active fluorine compounds (such as 1,5-difluoro-2,4-dinitrobenzene). For example, a ricin immunotoxin can be prepared as described in Vitetta et al., Science, 238: 1098 (1987). Carbon-14-labeled 1-isothiocyanatobenzyl-3-methyldiethylene triaminepentaacetic acid (MX-DTPA) is an exemplary chelating agent for conjugation of radionucleotide to the antibody. See WO94/11026.

In another embodiment, the antibody may be conjugated to a "receptor" (such streptavidin) for utilization in tumor pretargeting wherein the antibody-receptor conjugate is administered to the patient, followed by removal of unbound conjugate from the circulation using a clearing agent and then administration of a "ligand" (*e.g.*, avidin) that is conjugated to a cytotoxic agent (*e.g.*, a radionucleotide).

### viii. Immunoliposomes

The antibodies disclosed herein may also be formulated as immunoliposomes. Liposomes containing the antibody are prepared by methods known in the art, such as described in Epstein et al.. Proc. Natl. Acad. Sci. USA, 82: 3688 (1985); Hwang et al.. Proc. Natl Acad. Sci. USA, 77: 4030 (1980); and U.S. Pat. Nos. 4,485,045 and 4.544,545. Liposomes with enhanced circulation time are disclosed in U.S. Patent No. 5.013,556.

Particularly useful liposomes can be generated by the reverse-phase evaporation method with a lipid composition comprising phosphatidylcholine, cholesterol, and PEG-derivatized phosphatidylethanolamine (PEG-PE). Liposomes are extruded through filters of defined pore size to yield liposomes with the desired diameter. Fab' fragments of the antibody of the present invention can be conjugated to the liposomes as described in Martin et al., J. Biol. Chem. 257: 286-288 (1982) via a disulfide-interchange reaction. A chemotherapeutic agent (such as Doxorubicin) is optionally contained within the liposome. See Gabizon et al., J. National Cancer Inst. 81(19): 1484 (1989).

### ix. Pharmaceutical Compositions of Antibodies

Antibodies specifically binding a PR0364 or PRO175 polypeptide identified herein, as well as other molecules identified by the screening assays disclosed hereinbefore, can be administered for the treatment of various disorders as noted above and below in the form of pharmaceutical compositions.

If the PR0364 or PRO175 polypeptide is intracellular and whole antibodies are used as inhibitors, internalizing antibodies are preferred. However, lipofections or liposomes can also be used to deliver the antibody, or an antibody fragment, into cells. Where antibody fragments are used, the smallest inhibitory fragment that specifically binds to the binding domain of the target protein is preferred. For example, based upon the variable-region sequences of an antibody, peptide molecules can be designed that retain the ability to bind the target protein sequence. Such peptides can be synthesized chemically and/or produced by recombinant DNA technology. See, *e.g.*, Marasco et al., Proc. Natl. Acad. Sci. USA, 90: 7889-7893 (1993).

The formulation herein may also contain more than one active compound as necessary for the particular indication being treated, preferably those with complementary activities that do not adversely affect each other. Alternatively, or in addition, the composition may comprise an agent that enhances its function, such as, for example, a cytotoxic agent, cytokine, chemotherapeutic agent, or growth-inhibitory agent. Such molecules are suitably present in combination in amounts that are effective for the purpose intended.

The active ingredients may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles, and nanocapsules) or in macroemulsions. Such techniques are disclosed in Remington's Pharmaceutical Sciences, *supra.*

The formulations to be used for *in vivo* administration must be sterile. This is readily accomplished by filtration through sterile filtration membranes.

Sustained-release preparations may be prepared. Suitable examples of sustained-release preparations include semipermeable matrices of solid hydrophobic polymers containing the antibody, which matrices are in the form of shaped articles, *e.g.,* films, or microcapsules. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and γ ethyl-L-glutamate, non-degradable ethylene-vinyl acetate, degradable lactic acid-glycolic acid copolymers such as the LUPRON DEPOT ^{™} (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods. When encapsulated antibodies remain in the body for a long time, they may denature or aggregate as a result of exposure to moisture at 37°C, resulting in a loss of biological activity and possible changes in immunogenicity. Rational strategies can be devised for stabilization depending on the mechanism involved. For example, if the aggregation mechanism is discovered to be intermolecular S-S bond formation through thio-disulfide interchange, stabilization may be achieved by modifying sulfhydryl residues, lyophilizing from acidic solutions, controlling moisture content, using appropriate additives, and developing specific polymer matrix compositions.

### x. Methods of Treatment using the Antibody

It is contemplated that the antibodies to PR0364 or PRO175 polypeptide may be used to treat various cardiovascular, endothelial, and angiogenic conditions as noted above.

The antibodies are administered to a mammal, preferably a human, in accord with known methods, such as intravenous administration as a bolus or by continuous infusion over a period of time, by intramuscular, intraperitoneal, intracerobrospinal, subcutaneous, intra-articular, intrasynovial, intrathecal, oral, topical, or inhalation routes. Intravenous administration of the antibody is preferred.

Other therapeutic regimens may be combined with the administration of the antibodies of the instant invention as noted above. For example, if the antibodies are to treat cancer, the patient to be treated with such antibodies may also receive radiation therapy. Alternatively, or in addition, a chemotherapeutic agent may be administered to the patient. Preparation and dosing schedules for such chemotherapeutic agents may be used according to manufacturers' instructions or as determined empirically by the skilled practitioner. Preparation and dosing schedules for such chemotherapy are also described in Chemotherapy Service, Ed., M.C. Perry (Williams & Wilkins: Baltimore, MD, 1992). The chemotherapeutic agent may precede, or follow administration of the antibody, or may be given simultaneously therewith. The antibody may be combined with an anti-oestrogen compound such as tamoxifen or EVISTA^{™} or an anti-progesterone such as onapristone (see, EP 616812) in dosages known for such molecules.

If the antibodies are used for treating cancer it may be desirable also to administer antibodies against other tumor-associated antigens, such as antibodies that bind to one or more of the ErbB2, EGFR, ErbB3, ErbB4, or VEGF receptor(s). These also include the agents set forth above. Also, the antibody is suitably administered serially or in combination with radiological treatments, whether involving irradiation or administration of radioactive substances. Alternatively, or in addition, two or more antibodies binding the same or two or more different antigens disclosed herein may be co-administered to the patient. Sometimes, it may be beneficial also to administer one or more cytokines to the patient. In a preferred embodiment, the antibodies herein are co-administered with a growth-inhibitory agent. For example, the growth-inhibitory agent may be administered first, followed by an antibody of the present invention. However, simultaneous administration or administration of the antibody of the present invention first is also contemplated. Suitable dosages for the growth-inhibitory agent are those presently used and may be lowered due to the combined action (synergy) of the growth-inhibitory agent and the antibody herein.

In one embodiment, vascularization of rumors is attacked in combination therapy. The anti- PR0364 or - PRO175 polypeptide and another antibody (*e.g.,* anti-VEGF) are administered to rumor-bearing patients at therapeutically effective doses as determined, for example, by observing necrosis of the rumor or its metastatic foci, if any. This therapy is continued until such time as no further beneficial effect is observed or clinical examination shows no trace of the tumor or any metastatic foci. Then TNF is administered, alone or in combination with an auxiliary agent such as alpha-, beta- or gamma-interferon, anti-HER2 antibody, heregulin, anti-heregulin antibody, D-factor, interleukin-1 (IL-1), interleukin-2 (IL-2), granulocyte-macrophage colony stimulating factor (GM-CSF), or agents that promote microvascular coagulation in tumors, such as anti-protein C antibody, anti-protein S antibody, or C4b binding protein (see WO 91/01753, published 21 February 1991), or heat or radiation.

Since the auxiliary agents will vary in their effectiveness, it is desirable to compare their impact on the tumor by matrix screening in conventional fashion. The administration of anti- PR0364 or anti-PRO175 polypeptide antibody and TNF is repeated until the desired clinical effect is achieved. Alternatively, the of anti-PR0364 or anti-PRO175 polypeptide antibody is administered together with TNF and, optionally, auxiliary agent(s). In instances where solid tumors are found in the limbs or in other locations susceptible to isolation from the general circulation, the therapeutic agents described herein are administered to the isolated tumor or organ. In other embodiments, a FGF or PDGF antagonist, such as an anti-FGF or an anti-PDGF neutralizing antibody, is administered to the patient in conjunction with the of anti- PR0364 or anti-PRO175 polypeptide antibody. Treatment with anti- PR0364 or anti-PRO175 polypeptide antibodies preferably may be suspended during periods of wound healing or desirable neovascularization.

For the prevention or treatment of cardiovascular, endothelial, and angiogenic disorder, the appropriate dosage of an antibody herein will depend on the type of disorder to be treated, as defined above, the severity and course of the disease, whether the antibody is administered for preventive or therapeutic purposes, previous therapy, the patient's clinical history and response to the antibody, and the discretion of the attending physician. The antibody is suitably administered to the patient at one time or over a series of treatments.

For example, depending on the type and severity of the disorder, about 1 µg/kg to 50 mg/kg (*e.g.*, 0.1-20 mg/kg) of antibody is an initial candidate dosage for administration to the patient, whether, for example, by one or more separate administrations, or by continuous infusion. A typical daily or weekly dosage might range from about 1 µg/kg to 100 mg/kg or more, depending on the factors mentioned above. For repeated administrations over several days or longer, depending on the condition, the treatment is repeated or sustained until a desired suppression of disorder symptoms occurs. However, other dosage regimens may be useful. The progress of this therapy is easily monitored by conventional techniques and assays, including, for example, radiographic tumor imaging.

### xi. Articles of Manufacture with Antibodies

An article of manufacture containing a container with the antibody and a label is also provided. Such articles are described above, wherein the active agent is an anti-PR0364 or -PRO 175 antibody.

### xii. Diagnosis and Prognosis of Tumors using Antibodies

If the indication for which the antibodies are used is cancer, while cell- surface proteins, such as growth receptors overexpressed in certain tumors, are excellent targets for drug candidates or tumor (*e.g.*, cancer) treatment, the same proteins along with PR0364 or PRO175 polypeptides find additional use in the diagnosis and prognosis of tumors. For example, antibodies directed against the PR0364 or PRO175 polypeptides may be used as tumor diagnostics or prognostics.

For example, antibodies, including antibody fragments, can be used qualitatively or quantitatively to detect the expression of genes including the gene encoding the PRO364 or PRO175 polypeptide. The antibody preferably is equipped with a detectable. *e.g.*. fluorescent label, and binding can be monitored by light microscopy, flow cytometry, fluorimetry, or other techniques known in the art. Such binding assays are performed essentially as described above.

*In situ* detection of antibody binding to the marker gene products can be performed, for example, by immunofluorescence or immunoelectron microscopy. For this purpose, a histological specimen is removed from the patient, and a labeled antibody is applied to it, preferably by overlaying the antibody on a biological sample. This procedure also allows for determining the distribution of the marker gene product in the tissue examined. It will be apparent to those skilled in the art that a wide variety of histological methods are readily available for in *situ* detection.

The following Examples are offered for illustrative purposes only, and are not intended to limit the scope of the present invention in any way.

The disclosures of all patent and literature references cited in the present specification are hereby incorporated by reference in their entirety.

### EXAMPLES

Commercially available reagents referred to in the Examples were used according to manufacturer's instructions unless otherwise indicated. The source of those cells identified in the following Examples, and throughout the specification, by ATCC accession numbers is the American Type Culture Collection, Manassas, VA. Unless otherwise noted, the present invention uses standard procedures of recombinant DNA technology, such as those described hereinabove and in the following textbooks: Sambrook *et al., supra;* Ausubel et al., Current Protocols in Molecular Biology (Green Publishing Associates and Wiley Interscience, N.Y., 1989); Innis et al., PCR Protocols: A Guide to Methods and Applications (Academic Press. Inc.: N.Y., 1990); Harlow et al., Antibodies: A Laboratory Manual (Cold Spring Harbor Press: Cold Spring Harbor, 1988); Gait, Oligonucleotide Synthesis (IRL Press: Oxford, 1984); Freshney, Animal Cell Culture, 1987; Coligan et al., Current Protocols in Immunology, 1991.

### EXAMPLE 1

### Isolation of cDNA Clones Encoding Human PRO364

An expressed sequence tag (EST) DNA database (LIFESEQ^{™}, Incyte Pharmaceuticals, Palo Alto, CA) was searched and an EST (Incyte EST No. 3003460) was identified that showed homology to members of the tumor necrosis factor receptor (TNFR) family of polypeptides.

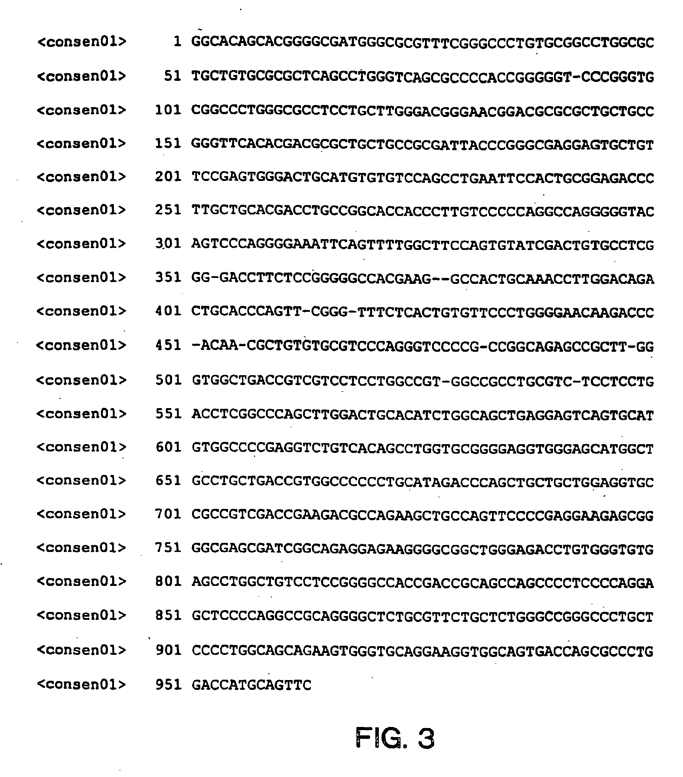

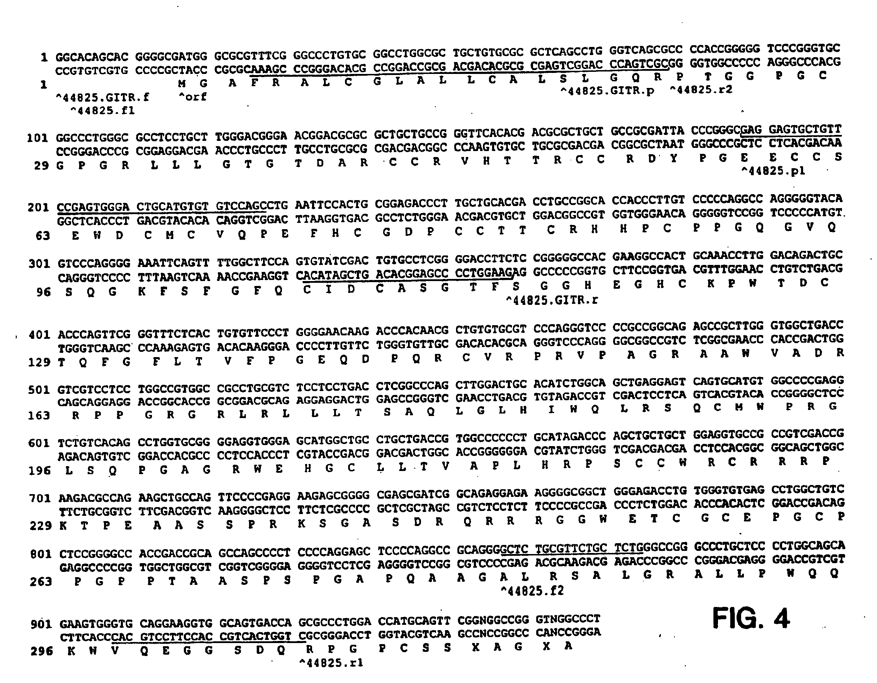

A consensus DNA sequence was then assembled relative to the Incyte 3003460 EST and other EST sequences using repeated cycles of BLAST (Altshul et al., Methods in Enzymology 266:460-480 (1996)) and "phrap" (Phil Green, University of Washington, Seattle, http://bozeman.mbt.washington.edu/phrap.docs/phrap.html). This consensus sequence is herein designated "<consen01>" in Figures 3A-C. The "<consen01>" consensus sequence (SEQ ID NO:4) shown in Figures 3A-C is also herein designated as "DNA44825" (see Figure 4; SEQ ID NO:4).

Based upon the DNA44825 and "<consen1>" consensus sequences shown in Figures 3-4, oligonucleotides were synthesized: 1) to identify by PCR a cDNA library that contained the sequence of interest, and 2) for use as probes to isolate a clone of the full-length coding sequence for PR0364. Forward and reverse PCR primers generally range from 20 to 30 nucleotides and are often designed to give a PCR product of about 100-1000 bp in length. The probe sequences are typically 40-55 bp in length. In some cases, additional oligonucleotides are synthesized when the consensus sequence is greater than about 1-1.5kbp. In order to screen several libraries for a full-length clone. DNA from the libraries was screened by PCR amplification, as per Ausubel et al., Current Protocols in Molecular Biology, with the PCR primer pair. A positive library was then used to isolate clones encoding the gene of interest using the probe oligonucleotide and one of the primer pairs.

Pairs of PCR primers (forward and reverse) were synthesized:
forward PCR primer (44825.f1) 5'-CACAGCACGGGGCGATGGG-3' (SEQ ID NO:6)
forward PCR primer (44825.f2) 5'-GCTCTGCGTTCTGCTCTG-3' (SEQ ID NO:11)
forward PCR primer (44825.GITR.f) 5'-GGCACAGCACGGGGCGATGGGCGCGTTT-3' (SEQ ID NO:5)
reverse PCR primer (44825.r1) 5'-CTGGTCACTGCCACCTTCCTGCAC-3' (SEQ ID NO:12)
reverse PCR primer (44825.r2) 5'-CGCTGACCCAGGCTGAG-3' (SEQ ID NO:8)
reverse PCR primer (44825.GITR.r) 5'-GAAGGTCCCCGAGGCACAGTCGATACA-3' (SEQ ID NO:10)
Additionally, synthetic oligonucleotide hybridization probes were constructed from the consensus DNA44825 sequence which had the following nucleotide sequences
hybridization probe (44825.p1)
   5'-GAGGAGTGCTGTTCCGAGTGGGACTGCATGTGTGTCCAGC-3' (SEQ ID NO:9)
hybridization probe (44825.GITR.p)
   5'-AGCCTGGGTCAGCGCCCCACCGGGGGTCCCGGGTGCGGCC-3' (SEQ ID NO:7)

In order to screen several libraries for a source of a full-length clone, DNA from the libraries was screened by PCR amplification with the PCR primer pairs identified above. A positive library was then used to isolate clones encoding the PRO364 gene using the probe oligonucleotides and one of the PCR primers.

RNA for construction of the cDNA libraries was isolated from human bone marrow tissue. The cDNA libraries used to isolate the cDNA clones were constructed by standard methods using commercially available reagents such as those from Invitrogen, San Diego, CA. The cDNA was primed with oligo dT containing a NotI site, linked with blunt to SalI hemikinased adaptors, cleaved with NotI, sized appropriately by gel electrophoresis, and cloned in a defined orientation into a suitable cloning vector (such as pRKB or pRKD; pRK5B is a precursor of pRK5D that does not contain the SfiI site; see, Holmes et al., Science, 253:1278-1280 (1991) in the unique XhoI and NotI sites.

DNA sequencing of the clones isolated as described above gave the full-length DNA sequence for PR0364 [herein designated as UNQ319 (DNA47365-1206)] (SEQ ID NO:1) and the derived protein sequence for PR0364.

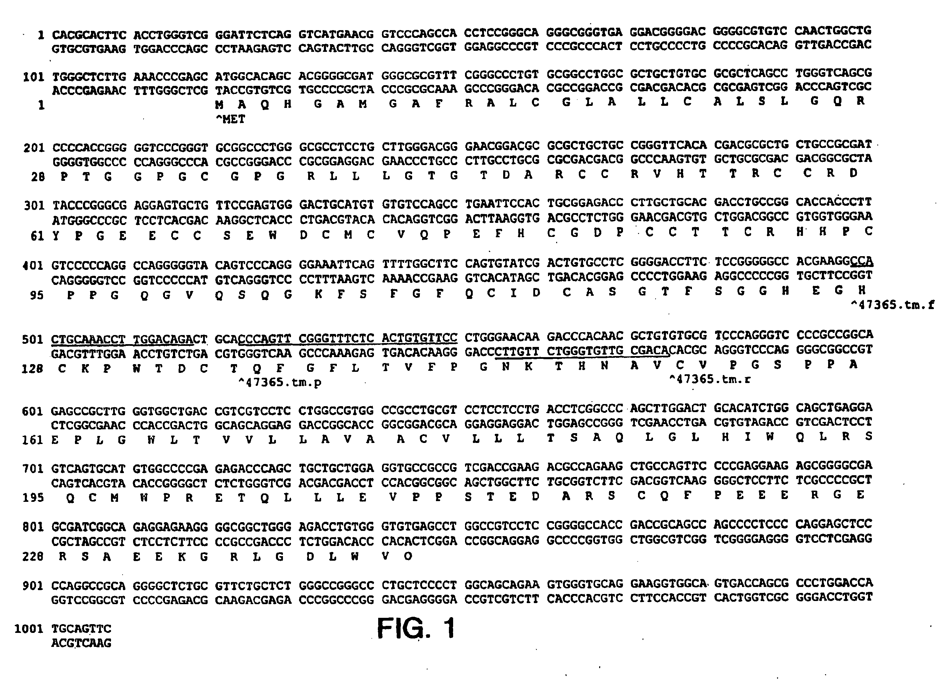

The entire nucleotide sequence of UNQ319 (DNA47365-1206) is shown in Figure 1 (SEQ ID NO:1). Clone UNQ319 (DNA47365-1206) has been deposited with ATCC and is assigned ATCC Deposit No. ATCC 209436. Clone UNQ319 (DNA47365-1206) contains a single open reading frame with an apparent translational initiation site at nucleotide positions 121-123 [Kozak et al., supra] and ending at the stop codon at nucleotide positions 844-846 (Figure 1 ; SEQ ID NO:1). The predicted polypeptide precursor is 241 amino acids long (Figure 2: SEQ ID NO:3). The full-length PR0364 protein (SEQ ID NO:3) shown in Figure 2 has an estimated molecular weight of about 26.000 daltons and a pl of about 6.34. A potential N-glycosylation site exists between amino acids 146 and 149 of the amino acid sequence shown in Figure 2 (SEQ ID NO:3). Hydropathy analysis (not shown) suggested a Type 1 transmembrane typology; a putative signal sequence is from amino acids 1 to 25 and a potential transmembrane domain exists between amino acids 162 to 180 of the sequence shown in Figure 2 (SEQ ID NO:3).

Analysis of the amino acid sequence of the full-length PR0364 polypeptide suggests that portions of it possess homology to members of the tumor necrosis factor receptor family, thereby indicating that PR0364 may be a novel member of the tumor necrosis factor receptor family. The intracellular domain of PR0364 contains a motif (in the region of amino acids 207-214 of Figure 2: SEQ ID NO:3) similar to the minimal domain within the CD30 receptor shown to be required for TRAF2 binding and which is also present within TNFR2 [Lee et al., supra, (1996)]. There are three apparent extracellular cysteine-rich domains characteristic of the TNFR family [see, Naismith and Sprang, Trends Biochem. Sci., 23:74-79 (1998)], of which the third CRD has 3 rather than the more typical 4 or 6 cysteines of the TNFR family, As compared to the mouse GITR (described below) the PR0364 amino acid sequence has 8 cysteines in CRD1 relative to 5 cysteines in CRD1 of mouse GITR, and the presence of one potential N-linked glycosylation site in the ECD as compared to 4 potential N-linked glycosylation sites in mouse GITR.

A detailed review of the putative amino acid sequence of the full-length native PR0364 polypeptide and the nucleotide sequence that encodes it evidences sequence homology with the mouse GITR (mGITR) protein reported by Nocentini et al., Proc. Natl. Acad. Sci. USA 94:6216-6221 (1997). It is possible, therefore, that PR0364 represents the human counterpart or ortholog to the mouse GITR protein reported by Nocentini et al.

### EXAMPLE 2

### Isolation of Human PRO175

Methods described in Klein et al., PNAS, 93:7108-7113 (1996) were employed with the following modifications. Yeast transformation was performed with limiting amounts of transforming DNA in order to reduce the number of multiple transformed yeast cells. Instead of plasmid isolation from the yeast followed by transformation of *E*. *coli* as described in Klein et al., supra, PCR analysis was performed on single yeast colonies. This was accomplished by restreaking the original sucrose positive colony onto fresh sucrose medium to purify the positive clone. A single purified colony was then used for PCR using the following primers:
TGTAAAACGACGGCCAGTTTCTCTCAGAGAAACAAGCAAAAC (SEQ ID NO:15) and CAGGAAACAGCTATGACCGAAGTGGACCAAAGGTCTATCGCTA (SEQ ID NO:16). The PCR primers are bipartite in order to amplify the insert and a small portion of the invertase gene (allowing to determine that the insert was in frame with invertase) arid to add on universal sequencing primer sites.

A library of cDNA fragments derived from human HUVEC cells fused to invertase was transformed into yeast and transformants were selected on SC-URA media. URA and transformants were replica plated onto sucrose medium in order to identify clones secreting invertase. Positive clones were re-tested and PCR products were sequenced. The sequence of one clone, DNA1840, was determined to contain a signal peptide coding sequence. Oligonucleotide primers and probes were designed using the nucleotide sequence of DNA 1840. A full length plasmid library of cDNAs from human umbilical vein endothelial cells (HUVEC) was titered and approximately 100,000 cfu were plated in 192 pools of 500 cfu/pool into 96-well round bottom plates. The pools were grown overnight at 37°C with shaking (200rpm). PCR was performed on the individual cultures using primers specific to DNA 1840. Agarose gel electrophoresis was performed and positive wells were identified by visualization of a band of the expected size. Individual positive clones were obtained by colony lift followed by hybridization with ³²P-labeled oligonucleotide. These clones were characterized by PCR, restriction digest, and southern blot analyses.

A cDNA clone (DNA 119355) was sequenced in entirety. A nucleotide sequence of PRO175 is shown in Figure 5A-B (SEQ ID NO:13). Clone PRO175-1150 contains a single open reading frame with an apparent translational initiation site at nucleotide positions 21-23 [Kozak et al., supra] (Figure 5 ; SEQ ID NO:13). The predicted polypeptide precursor is 177 amino acids long and has a calculated molecular weight of approximately 20,308 daltons. Hydropathy analysis suggests a type 11 transmembrane protein typology, with a putative cytoplasmic region (amino acids 1-25); transmembrane region (amino acids 26-51); and extracellular region (amino acids 52-177). Two potential N-linked glycosylation sites have been identified at position 129 (Asn) and position 161 (Asn) of the sequence shown in Figure 5 (SEQ ID NO: 14). Clone PRO175-1150 has been deposited with ATCC and is assigned ATCC deposit no. 209466. PRO175 polypeptide is obtained or obtainable by expressing the molecule encoded by the cDNA insert of the deposited ATCC 209466 vector. Digestion of the vector with XbaI and NotI restriction enzymes will yield a 1411 bp fragment and 668 bp fragment.

Based on a BLAST and FastA sequence alignment analysis (using the ALIGN computer program) of extracellular sequence, PRO175 shows amino acid sequence identity to several members of the TNF cytokine family, and particularly, to human Apo-2L (19.8%), Fas/Apo1-ligand (19.0%), TNF-alpha (20.6%) and Lymphotoxin-α(17.5%). Most of the amino acid sequence identity is found in the regions corresponding to the beta-strands in the crystal structure of TNF-alpha [Banner et al., Cell, 73:431-435 (1993); Eck et al., J. Biol. Chem., 264:17595-605 (1989); Lewit-Bentley et al., J. Mol. Biol., 199:389-92 (1988)]. The sequence of strand C is especially conserved in all members of the family. The sequence between the putative transmembrane domain and the first beta-strand of the PRO175 polypeptide is relatively short, including 5 residues, as compared to about 30 to about 80 residues in TNF-alpha, CD95L or Apo-2 ligand.

### EXAMPLE 3

### Northern Blot Analysis of PRO175

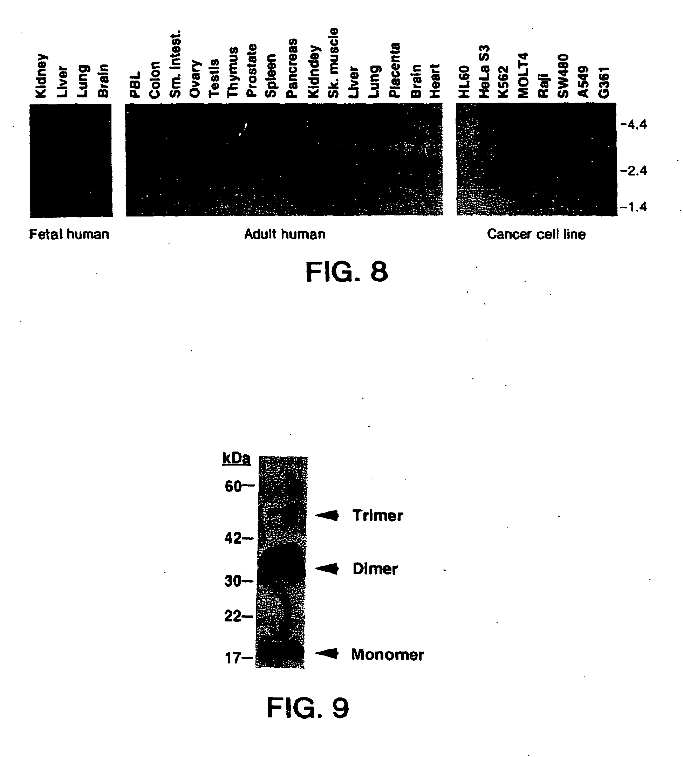

Expression of PRO175 mRNA in human tissues and tumor cell lines was examined by Northern blot analysis (see Figure 8). Human RNA blots were hybridized to an approximately 700 bp-long ³²P-labeled DNA probe generated by digestion of the pRK5 plasmid encoding full-length PRO175 cDNA with Xba-I: this probe corresponds to the entire coding sequence plus some flanking 5' and 3' sequences.

Human fetal, adult, or cancer cell line mRNA blots (Clontech) were incubated with the DNA probe in hybridization buffer (5X SSPE; 2X Denhardt's solution; 100 mg/mL denatured sheared salmon sperm DNA; 50% formamide; 2% SDS) for 60 hours at 42°C. The blots were washed several times in 2X SSC: 0.05% SDS for 1 hour at room temperature, followed by a 30 minute wash in 0.1X SSC; 0.1% SDS at 50°C. The blots were developed after overnight exposure by phosphorimager analysis (Fuji).

As shown in Figure 8 a predominant mRNA transcript of about 3.2 kB was detected in fetal kidney and lung, and in adult small intestine. Expression was also detected in 6 out of 8 human tumor cell lines tested, which showed about the same 3.2 kB transcript, as well as weaker expression of about 1.5 and about 5 kB transcripts.

The results indicate that the mRNA expression of the PRO175 polypeptide is relatively restricted in normal tissues, but is markedly elevated in tumor cell lines from lymphoid as well as non-lymphoid origin.

### EXAMPLE 4: Assays to Detect Expression of PRO364 mRNA in Human Cells and Tissues

Assays were conducted to examine expression of PRO364 mRNA in normal human tissues and in cancer cells lines.

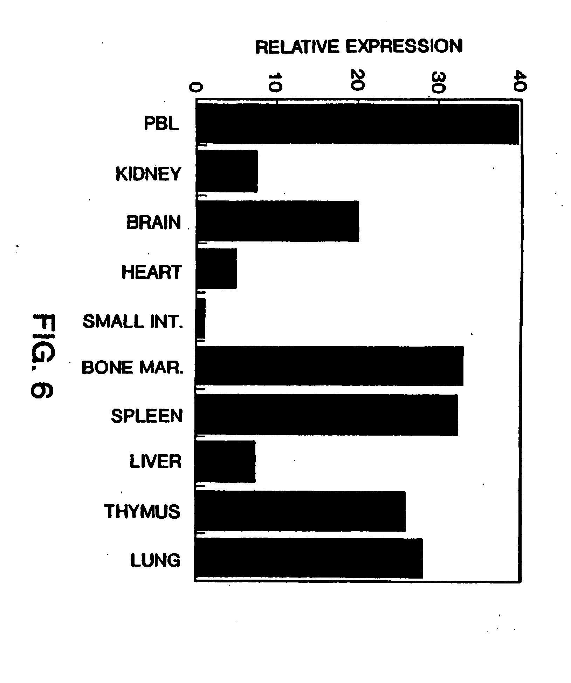

Various human tissues and cancer cell lines (Clontech) were tested by Northern blot hybridization for detection of PRO364 transcripts, but none were detected. Using quantitative reverse-transcriptase PCR, PRO364 mRNA was detected in PBL, brain, bone marrow, spleen, thymus and lung, and at relatively lower levels, in kidney, heart, small intestine and liver tissues (see Figure 6). The relative mRNA expression levels were determined by quantitative PCR using a Taqman instrument (ABI) essentially as described in Heid et al., Genome Res., 6:986-94 (1996) using PRO364 specific primers and fluorogenic probes:
DNA47365.tm.f- CCACTGAAACCTTGGACAGA (SEQ ID NO:17)
DNA47365.tm.p - CCCAGTTCGGGTTTCTCACTGTGTTCC (SEQ ID NO: 18)
DNA47365.tm.r - ACAGCGTTGTGGGTCTTGTTC (SEQ ID NO: 19)
The authenticity of the PCR product was confirmed by Southern blot hybridization to the corresponding cDNA. Expression levels were normalized relative to small intestine tissue.

In a separate assay, primary human T cells (isolated from donor whole blood using a T cell enrichment column (R & D Systems)) and monocytes/macrophages (isolated from donor whole blood by adherence to tissue culture flasks) were maintained in RPMI supplemented with 10% FBS and 2 mM glutamine. The cells were then treated for 24 hours with PHA (1 microgram/ml; Sigma), anti-CD3 antibody (1 microgram/ml; Pharmingen), LPS (1 microgram/ml; Sigma), TNF-alpha (1 microgram/ml; prepared essentially as described in Pennica et al., Nature, 312:724-729 (1984)), or the soluble PRO175 ligand (5 microgram/ml). The relative mRNA expression levels were then analyzed by the Taqman procedure described above. The expression levels were normalized relative to buffer-treated T cells.

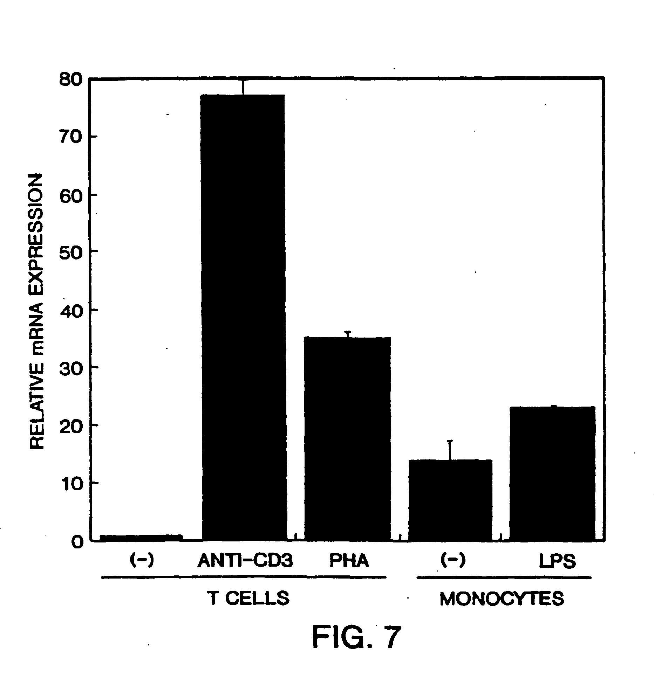

The results are shown in Figure 7. Substantial up-regulation of PRO364 mRNA was observed in isolated peripheral blood T cells after stimulation by phytohemagglutinin (PHA) or by anti-CD3 antibody. High levels of expression were observed in isolated monocytes/macrophages and this expression was further increased by LPS. (See Figure 7).

### EXAMPLE 5

### Expression of PRO175 in E. coli

The DNA sequence (Figure 5A-B; SEQ ID NO:13) encoding an extracellular region of the PRO175 polypeptide (amino acids 52 to 177 of Figure 5; SEQ ID NO:14) was amplified with PCR primers containing flanking Ndel and Xbal restriction sites, respectively: forward: 5'-GAC GAC AAG CAT ATG TTA GAG ACT GCT AAG GAG CCC TG-3' (SEQ ID NO:20); reverse: 5'-TAG CAG CCG GAT CCT AGG AGA TGA ATT . GGG GATT-3' (SEQ ID NO:21). The PCR was digested and cloned into the Ndel and Xbal sites of plasmid pET19B (Novagen) downstream and in frame of a Met Gly His₁₀ sequence followed by a 12 amino acid enterokinase cleavage site (derived from the plasmid):
Met Gly His His His His His His His His His His Ser Ser Gly His Ile Asp Asp Asp Asp Lys His Met (SEQ ID NO:22).

The resulting plasmid was used to transform *E*. *coli* strain JM109 (ATCC 53323) using the methods described in Sambrook et al., supra. Transfor mants were identified by PCR. Plasmid DNA was isolated and confirmed by restriction analysis and DNA sequencing.

Selected clones were grown overnight in liquid culture medium LB supplemented with antibiotics. The overnight culture was subsequently used to inoculate a larger scale culture. The cells were grown to a desired optical density, during which the expression promoter is turned on.

After culturing the cells for several more hours, the cells were harvested by centrifugation. The cell pellet obtained by the centrifugation was solubilized using a microfluidizer in a buffer containing 0.1M Tris, 0.2M NaCl, 50 mM EDTA, pH 8.0. The solubilized PRO175 protein was purified using Nickel-sepharose affinity chromatography.

The PRO175 protein was analyzed by SDS-PAGE followed by Western Blot with nickel-conjugated horseradish peroxidase followed by ECL detection (Boehringer Mannheim). Three predominant protein bands were detected, which corresponded in size to monomeric, homodimeric, and homotrimeric forms of the protein (Figure 9). It is believed based on this result that in its native form, in the absence of SDS denaturation, the soluble PRO175 protein is capable of forming homotrimers.

### EXAMPLE 6

### Binding Specificity of PRO175 for the PRO364 Receptor

Assays were conducted to determine whether the PRO175 polypeptide (described in Example 2,3 and 5 above) interacts and specifically binds with PRO364, which is believed to be a human ortholog of the murine GITR (mGITR) polypeptide described in Nocentini et al., Proc. Natl. Acad. Sci., 94:6216-6221 (1997).

To test for binding, a soluble immunoglobulin fusion protein (immunoadhesin) which included a PRO364 extracellular domain (see amino acids 1-161 of Figure 2; SEQ ID NO:3) was expressed in insect cells. The PRO364 ECD was expressed as a C-terminus IgG-Fc tagged form in insect cells using Baculovirus. A soluble PRO175 polypeptide was prepared by expressing an ECD as described above in Example 5.

The soluble PRO175 ECD molecule was abeled with ¹²⁵I, for testing its ability to interact with the PRO364 immunoadhesin. For comparison, immunoadhesin constructs were also made of the following TNF receptor family members: CD95, DR4, DR5, TNFR1, TNFR2, and Apo-3. CD95, DR4, DR5, TNFR1, TNFR2, and Apo-3 immunoadhesins were prepared by fusing each receptor's ECD to the hinge and Fc portion of human IgG, as described previously for TNFR1 [Ashkenazi et al., Proc. Natl. Acad. Sci., 88:10535-10539 (1991)]. The respective TNF receptor family members are described (and relevant references cited) in the Background of the Invention section.

For the co-precipitation assay, each immunoadhesin (5 microgram) was incubated with ¹²⁵I-labeled soluble PRO175 polypeptide (1 microgram) for 1 hour at 24°C, followed by protein A-sepharose for 30 minutes on ice. The reaction mixtures were spun down and washed several times in PBS, boiled in SDS-PAGE buffer containing 20 mM dithiothreitol and then resolved by SDS-PAGE and autoradiography.

The results are shown in Figure 9. The position of the molecular weight markers (kDa) are indicated in the figure. The PRO364-IgG bound to the radioiodinated soluble PRO175 polypeptide. However, the PRO364-IgG did not bind to the immunoadhesin constructs of CD95, DR4, DR5, TNFR1, TNFR2, or Apo-3.

In another assay, human 293 cells were transiently transfected with full-length PRO175 and the ability of receptor immunoadhesin constructs for PRO364, TNFR1, HVEM, and DcR1 to bind to those transfected cells was determined by FACS analysis. The 293 cells were maintained in high glucose DMEM media supplemented with 10% fetal bovine serum (FBS), 2mM glutamine. 100 microgram/ml penicillin, and 100 microgram/ml streptomycin. The transfected cells (1 x 10⁵) were incubated for 60 minutes at 4°C in 200 microliters 2% FBS/PBS with 1 microgram of the respective receptor or ligand immunoadhesin. The cells were then washed with 2% FBS/PBS, stained with R-phycoerythrin-conjugated goat anti-human antibody (Jackson Immunoresearch, West Grove, PA). Next, the cells were analyzed by FACS. To test the binding of the respective immunoadhesins to the transiently transfected cells, an expression vector (pRK5-CD4; Smith et al., Science, 328:1704-1707 (1987)) for CD4 was co-transfected with PRO175 expression vector (see above). FITC-conjugated anti-CD4 (Pharmingen, San Diego, CA) was then used to identify and gate the transfected cell population in the FACS analysis.

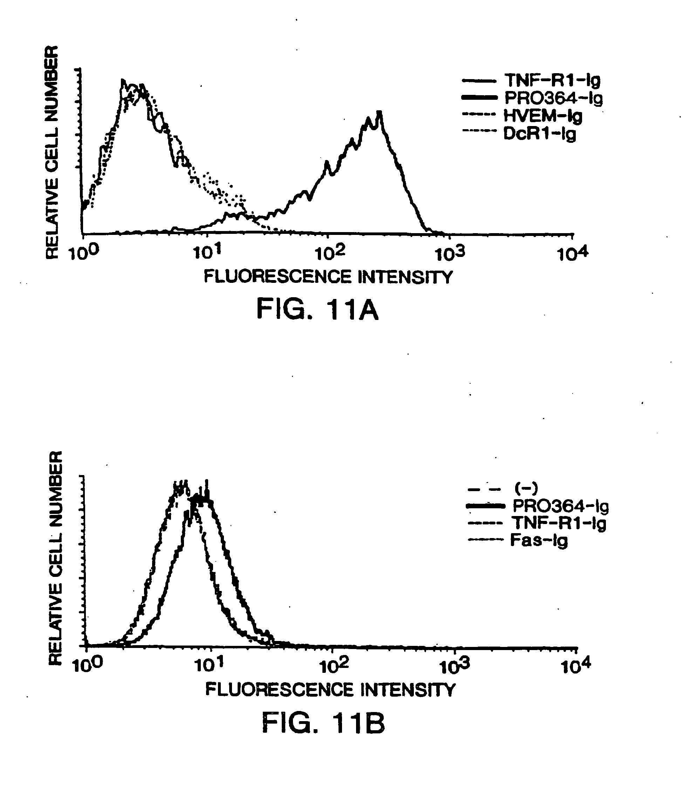

As shown in Figure 11A, the PRO364-IgG bound specifically to the surface of cells transfected with the expression plasmid encoding the full length PRO175. No such binding was observed for the TNFR1. HVEM or DcR1 immunoadhesins. The PRO364-IgG did not bind to the cells transfected with a control plasmid (data not shown).

The results demonstrate a specific binding interaction of the PRO175 polypeptide with PRO364 and that the PRO175 polypeptide does not interact with any of the other TNF receptor family members tested.

The PRO175 polypeptide was identified in a human umbilical vein endothelial cell (HUVEC) library, and the PRO175 polypeptide transcripts are readily detectable in HUVEC by RT-PCR (data not shown). A FACS analysis assay was conducted to examine whether specific binding of PRO364-IgG could be demonstrated with HUVEC by FACS analysis. HUVEC were purchased from Cell Systems (Kirkland, WA) and grown in a 50:50 mix of Ham's F12 and Low Glucose DMEM media containing 10% fetal bovine serum, 2 mM L-glutamine, 10 mM Hepes, and 10 ng/ml basic FGF. Cells were FACS sorted with PBS, PRO364-IgG, TNFR1-IgG or Fas-IgG as a primary antibody and goat anti-human F(ab')2 conjugated to phycoerythrin (CalTag, Burlingame, CA).

It was found that PRO364-IgG specifically bound to HUVEC (See Figure 11B). Neither the Fas-IgG nor the TNFR1-IgG exhibited specific binding to the endothelial cells.

### EXAMPLE 7

### Chromosomal Mapping

Chromosomal localization of the human PRO175 gene was examined by radiation hybrid (RH) panel analysis. RH mapping was performed by PCR using a mouse-human cell radiation hybrid panel (Research Genetics) and primers based on the coding region of the PRO175 cDNA [Gelb et al., Hum. Genet., 98:141 (1996)]. Analysis of the PCR data using the Stanford Human Genome Center Database indicated that PRO175 is linked to the STS marker D1S2790 and to Genethon marker AFMb352xe9, and maps to the human chromosome 1q23. Notably, CD95L also maps to chromosome lq23 [Takahashi et al., Int. Immunol., 6:1567-1574 (1994), whereas OX40 ligand maps to chromosome 1q25 [Baum et al., EMBO J., 13:3992-4001 (1994)]. Accordingly, these TNF family members may have arisen by duplication and divergence of a common ancestral gene.

### EXAMPLE 8

### Induction of c-fos in Endothelial Cells

This assay was performed to determine whether PRO364 (human GITR) showed the ability to induce c-fos in endothelial cells.

Human venous umbilical vein endothelial cells (HUVEC, Cell Systems) in growth media (50% Ham's F12 w/o GHT: low glucose, and 50% DMEM without glycine: with NaHCO₃, 1% glutamine, 10 mM HEPES, 10% FBS, 10 ng/ml bFGF) were plated on 96-well microtiter plates at a cell density of 1 x 10⁴ cells/well. The day after plating, the cells were starved by removing the growth media and adding 100 µl/well serum-free media (growth media without FBS or bFGF). After 24 hrs, the serum-free media was removed. The cells were treated with 100 µl/well test samples and controls (positive control: growth media; negative control: 10 mM HEPES, 140 mM NaCl, 4% (w/v) mannitol, pH 6.8). The cells were incubated for 30 minutes at 37°C, in 5% CO₂, The samples were removed, and the first part of the bDNA kit protocol (Chiron Diagnostics, cat. #6005-037) is followed, where each capitalized reagent/buffer listed below is available from the kit.

Briefly, the amounts of the TM Lysis Buffer and Probes needed for the tests were calculated based on information provided by the manufacturer. The appropriate amounts of thawed Probes were added to the TM Lysis Buffer. The Capture Hybridization Buffer was warmed to room temperature. The bDNA strips were set up in the metal strip holders, and 100 µl of Capture Hybridization Buffer were added to each b-DNA well needed, followed by incubation for at least 30 minutes. The test plates with the cells were removed from the incubator, and the media were gently removed using the vacuum manifold. 100 µl of Lysis Hybridization Buffer with Probes were quickly pipetted into each well of the microtiter plates. The plates were then incubated at 55°C for 15 minutes. Upon removal from the incubator, the plates were placed on the vortex mixer with the microtiter adapter head and vortex on the #2 setting for one minute. 80 µl of the lysate was removed and added to the bDNA wells containing the Capture Hybridization Buffer, and pipetted up and down to mix. The plates were incubated at 53 °C for at least 16 hours.

On the next day, the second part of the bDNA kit protocol was followed. Specifically, the plates were removed from the incubator and placed on the bench to cool for 10 minutes. The volumes of additions needed were calculated based upon information provided by the manufacturer. An Amplifier Working Solution was prepared by making a 1:100 dilution of the Amplifier Concentrate (20 fm/µl) in AL Hybridization Buffer. The hybridization mixture was removed from the plates and washed twice with Wash A. 50 µl of Amplifier Working Solution were added to each well and the wells were incubated at 53 °C for 30 minutes. The plates were then removed from the incubator and allowed to cool for 10 minutes. The Label Probe Working Solution was prepared by making a 1:100 dilution of Label Concentrate (40 pmoles/µl) in AL Hybridization Buffer. After the 10-minute cool-down period, the amplifier hybridization mixture was removed and the plates were washed twice with Wash A. 50 µl of Label Probe Working Solution were added to each well and the wells were incubated at 53°C for 15 minutes. The Substrate Solution was prepared by making a 1:100 dilution of Substrate Enhancer in Substrate Buffer. The plates were allowed to cool for 10 minutes, the label hybridization mixture was removed, and the plates were washed twice with Wash A and three times with Wash D. 50 µl of the Substrate Solution with Enhancer were added to each well. The plates were incubated for 30 minutes at 37°C and RLU was read in an appropriate luminometer.

The replicates were averaged and the coefficient of variation was determined. The measure of activity of the fold increase over the negative control (HEPES buffer described above) value was indicated by chemiluminescence units (RLU). Samples that show an at least two-fold value over the negative control value were considered positive.

PRO364 assay results:
1. Negative control = 2.57 RLU
   Positive control = 29.57 RLU
   PRO364 at 0.01 % = 8.60 RLU
   PRO364 at 0.1% = 9.66 RLU
   PRO364 at 1% = 3.44 RLU
2. Negative control = 3.19 RLU
   Positive control = 18.22 RLU
   PRO364 at 0.01% = 3.51 RLU
   PRO364 at 0.1% = 4.54 RLU
   PRO364 at 1% = 6.98 RLU

As shown above, PRO364 assayed positive twice, indicating its ability to induce c-fos expression in endothelial cells.

### EXAMPLE 9

This assay was performed to determine whether PRO175 (hGLITTER) showed the ability to induce c-fos in pericytes.

On Day 1, 4 T-25 tissue culture flasks of bovine pericytes (VEC Technologies) in growth media were received. All but 5 ml media was removed, and the flasks were placed in an incubator at 37°C in 5%CO₂. On Day 2, the pericytes were trypsinized, spun down, resuspended in growth media (low glucose DMEM with 20% FBS, 1X penicillin and streptomycin, and 1X fungizone) and plated onto 4 96-well microtiter plates. On Day 7, the media was removed, and the pericytes were treated with 100µl of test samples and controls (assay diluent: DMEM + 5% FBS; positive control: DMEM+5%serum +/- PDGF@50 ng/ml; negative control: protein 32). The cells were incubated for 30 minutes at 37°C in 5%CO₂. The samples were removed and the first part of the bDNA Kit (Chiron) protocol was followed.

Probes for c-fos were added to the Lysis Buffer included in the bDNA Kit. After the 30 minute incubation, the media was aspirated from the cells and 100 µl of the Lysis Buffer containing the c-fos probes was added to each well. The plates were then incubated for 15 minutes at 55°C. During this time, 100 µl of the Capture Hybridization Buffer was added to the bDNA Plate included in the kit. At the end of the 15 minutes, the cells are removed from the incubator, and vortexed on a microtiter plate shaker for 1 minute. 85 µl of the cell lysate was removed and added to the 100 µl of Capture Hybridization Buffer in the bDNA Plate. The plates were then incubated at 53°C for at least 16 hours.

The next day, the second part of the bDNA Kit protocol was followed. All the reagents were removed from the kit and allowed to come to room temperature. The plates were removed from the incubator and placed on the bench to cool for 10 minutes. The Amplifier was diluted to 1:100 in Amplifier/Label Probe Diluent. After 10 minutes, the hybridization mixture was aspirated from the plates and the wells were washed twice with Wash Buffer A. 50 µl of diluted amplifier was added to each of the wells, and the plates were incubated for 30 minutes at 53°C. The plates were then removed from the incubator and allowed to cool for 10 minutes. The Label Probe was diluted 1:100 in Amplifier/Label Probe Diluent. After 10 minutes, the amplifier was aspirated off. and the plates were again washed twice with Wash Buffer A. 50 µl of the diluted Label Probe was added to each of the wells and the plates were incubated for 15 minutes at 53°C. The plates were then removed from the incubator, and allowed to cool for 10 minutes. At this point, the substrate solution was prepared by adding 3 µl of Substrate Enhancer to each ml of Substrate needed for the assay. The label probe mixture was then aspirated from the wells and the plates are washed twice with Wash Buffer A and three times with Wash Buffer B. 50 µl of substrate solution was added to each well, and the plates were incubated at 37°C for 30 minutes. The plates were then read for chemiluminescence using a luminometer.

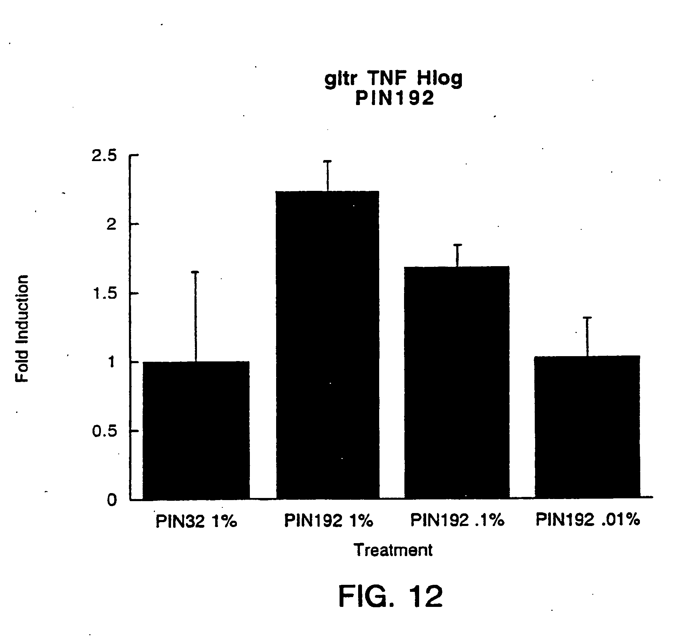

The replicates were averaged and the standard deviation/coefficient of variation was determined. The measure of activity of the fold increase over negative control (Protein 32) value was indicated by chemiluminescence units (RLU). Samples that show at least two fold value over the negative control value were considered a positive. The results from these analyses are shown in Figure 12.

As shown in Figure 12. PRO175 assayed positive for induction of c-fos expression in bovine pericytes.

### Deposit of Material

The following materials have been deposited with the American Type Culture Collection, 10801 University Blvd., Manassas, Virginia USA (ATCC):

| Material | ATCC Dep. No. | Deposit Date |
|---|---|---|
| DNA47365-1206 | ATCC 209436 | November 7, 1997 |
| DNA19355-1150 | ATCC 209466 | November 7, 1997 |

This deposit was made under the provisions of the Budapest Treaty on the International Recognition of the Deposit of Microorganisms for the Purpose of Patent Procedure and the Regulations thereunder (Budapest Treaty). This assures maintenance of a viable culture of the deposit for 30 years from the date of deposit. The deposit will be made available by ATCC under the terms of the Budapest Treaty, and subject to an agreement between Genentech, Inc. and ATCC, which assures permanent and unrestricted availability of the progeny of the culture of the deposit to the public upon issuance of the pertinent U.S. patent or upon laying open to the public of any U.S. or foreign patent application, whichever comes first, and assures availability of the progeny to one determined by the U.S. Commissioner of Patents and Trademarks to be entitled thereto according to 35 USC Section 122 and the Commissioner's rules pursuant thereto (including 37 CFR Section 1.14 with particular reference to 886 OG 638).

The assignee of the present application has agreed that if a culture of the materials on deposit should die or be lost or destroyed when cultivated under suitable conditions, the materials will be promptly replaced on notification with another of the same. Availability of the deposited material is not to be construed as a license to practice the invention in contravention of the rights granted under the authority of any government in accordance with its patent laws.

The foregoing written specification is considered to be sufficient to enable one skilled in the art to practice the invention. The present invention is not to be limited in scope by the construct(s) deposited, since the deposited embodiment(s) is/are intended as single illustration(s) of certain aspects of the invention and any constructs that are functionally equivalent are within the scope of this invention. The deposit of material(s) herein does not constitute an admission that the written description herein contained is inadequate to enable the practice of any aspect of the invention, including the best mode thereof, nor is it to be construed as limiting the scope of the claims to the specific illustrations that it represents.

## Claims

1. A composition comprising a PRO364 polypeptide in admixture with a pharmaceutically acceptable carrier and cardiovascular, endothelial or angiogenic agent or an angiostatic agent, wherein the PRO364 polypeptide:
(1) comprises amino acids 1 to 241 as set out In SEQ ID NO: 3; or
(2) comprises amino acids 26 to 241 as set out in SEQ ID NO: 3; or
(3) comprises amino acid residues Y to X as set out in SEQ ID NO: 3,
wherein Y is any one of amino acid residues 1 to 26 and X is any one of amino acids residues 157 to 167; or
(4) has at least 80% sequence identity with either a full length PRO364 polypeptide having amino acid residues 1 to 241 as set out in SEQ ID NO: 3 or an extracellular domain of PRO364 polypeptide having amino acid residues Y to X as set out in SEQ ID NO: 3, wherein Y is any one of amino acid residues 1 to 26 and X is any one of amino acids residues 157 to 167.

2. A composition comprising a PRO175 polypeptide in admixture with a pharmaceutically acceptable carrier, wherein the PRO176 polypeptide:
(1) comprises amino acids 1 to 177 as set out in SEQ ID NO: 14; or
(2) comprises amino acids 52 to 177 as set out SEQ ID NO: 14; or
(3) comprises amino acid residues X to 177 as set out in SEQ ID NO: 3,
wherein X is any one of amino acid residues 48 to 57; or
(4) has at least 80% sequence identity with either a full length PRO175 polypeptide having amino acid residues 1 to 177 as set out in SEQ ID NO: 14 or an extracellular domain of PRO175 polypeptide having amino acid residues X to 177 as set out in SEQ ID NO: 3, wherein X is any one of amino acid residues 48 to 57.

3. The composition of claim 1 or 2 comprising a therapeutically effective amount of the polypeptide.

4. The composition of claim 2, wherein the PRO175 polypeptide is in admixture with a pharmaceutically acceptable carrier and cardiovascular, endothelial or angiogenic agent or an angiostatic agent.

5. The composition of claim 4, wherein the polypeptide and agent are present in therapeutically effective amounts.

6. A method for preparing a composition of any one of the preceding claims for the treatment of a cardiovascular or endothelial or angiogenic disorder comprising admixing a therapeutically effective amount of PRO364 or PRO175 polypeptide with a pharmaceutically acceptable carrier.

7. A pharmaceutical product comprising:
(a) a composition comprising a therapeutically effective amount of PRO364 polypeptide in admixture with a cardiovascular, endothelial or angiogenic agent or an angiostatic agent, or of PRO175 polypeptide, in admixture with a pharmaceutically acceptable carrier;
(b) a container containing said composition; and
(c) a label affixed to said container, or a package insert included in said pharmaceutical product referring to the use of said PRO364 or PRO175 polypeptide composition in the treatment of a cardiovascular or endothelial or angiogenic disorder;
wherein the PRO384 polypeptide:
(1) comprises amino acids 1 to 241 as set out in SEQ ID NO: 3; or
(2) comprises amino acids 26 to 241 as set out in SEQ ID NO: 3; or
(3) comprises amino acid residues Y to X as set out in SEQ ID NO: 3,
wherein Y is any one of amino acid residues 1 to 26 and X is any one of amino acids residues 157 to 167; or
(4) has at least 80% sequence identity with either a full length PRO364 polypeptide having amino acid residues 1 to 241 as set out in SEQ ID NO: 3 or an extracellular domain of PRO364 polypeptide having amino acid residues Y to X as set out in SEQ ID NO: 3, wherein Y is any one of amino acid residues 1 to 26 and X is any one of amino acids residues 157 to 167; or
wherein the PRO175 polypeptide:
(1) comprises amino acids 1 to 177 as set out in SEQ ID NO: 14; or
(2) comprises amino acids 52 to 177 as set out SEQ ID NO: 14; or
(3) comprises amino acid residues X to 177 as set out in SEQ ID NO: 3,
wherein X is any one of amino acid residues 48 to 57; or
(4) has at least 80% sequence identity with either a full length PRO175 polypeptide having amino acid residues 1 to 177 as set out in SEQ ID NO: 14 or an extracellular domain of PRO175 polypeptide having amino acid residues X to 177 as set out in SEQ ID NO: 3, wherein X is any one of amino acid residues 48 to 57.

8. A process for diagnosing a disease or a susceptibility to a disease related to a mutation in a nucleic acid sequence encoding PRO364 or PRO175 polypeptide having an amino acid sequence as set out in SEQ ID NO: 3 or 14 comprising:
(a) isolating a nucleic acid sequence encoding PRO364 or PRO175 polypeptide from a sample derived from a host; and
(b) determining a mutation in the nucleic acid sequence encoding the PRO364 or PRO175 polypeptide.

9. A method of diagnosing cardiovascular or endothelial or angiogenic disorders in a mammal comprising detecting the level of expression of a gene encoding a PRO364 or PRO175 polypeptide having an amino acid sequence as set out in SEQ ID NO: 3 or 14 (a) in a test sample of tissue cells obtained from the mammal, and (b) in a control sample of known normal tissue cells of the same cell type, wherein a higher or lower expression level in the test sample indicates the presence of a cardiovascular or endothelial or angiogenic dysfunction in the mammal from which the test tissue cells were obtained.

10. Use of a PRO364 or PRO175 polypeptide for the preparation of a medicament for the treatment of a cardiovascular or endothelial or angiogenic disorder in a mammal;
wherein the PRO364 polypeptide:
(1) comprises amino acids 1 to 241 as set out in SEQ ID NO: 3; or
(2) comprises amino acids 26 to 241 as set out in SEQ ID NO: 3; or
(3) comprises amino acid residues Y to X as set out in SEQ ID NO: 3,
wherein Y is any one of amino acid residues 1 to 26 and X is any one of amino acids residues 157 to 167; or
(4) has at least 80% sequence identity with either a full length PRO364 polypeptide having amino acid residues 1 to 241 as set out in SEQ ID NO: 3 or an extracellular domain of PRO364 polypeptide having amino acid residues Y to X as set out in SEQ ID NO: 3, wherein Y is any one of amino acid residues 1 to 26 and X is any one of amino acids residues 157 to 167; or
wherein the PRO175 polypeptide:
(1) comprises amino acids 1 to 177 as set out in SEQ ID NO: 14; or
(2) comprises amino acids 52 to 177 as set out SEQ ID NO: 14; or
(3) comprises amino acid residues X to 177 as set out in SEQ ID NO: 3,
wherein X is any one of amino acid residues 48 to 57; or
(4) has at least 80% sequence identity with either a full length PRO175 polypeptide having amino acid residues 1 to 177 as set out in SEQ ID NO: 14 or an extracellular domain of PRO175 polypeptide having amino acid residues X to 177 as set out in SEQ ID NO: 3, wherein X is any one of amino acid residues 48 to 57.

11. The use of claim 10, wherein the cardiovascular or endothelial or angiogenic disorder is cardiac hypertrophy or trauma or cancer.

12. The use of claim 10, wherein said mammal is human.

13. The use of claim 11, wherein said cardiac hypertrophy is **characterized by** the presence of an elevated level of PGF_{2α}.

14. The use of claim 11, wherein said cardiac hypertrophy has been induced by myocardial infraction.

15. The use of claim 14, wherein the administration of said PRO364 or PRO175 polypeptide is initiated within 48 hours following myocardial infarction.

16. The use of claim 11, wherein the cardiovascular or endothelial or angiogenic disorder is cardiac hypertrophy and said PRO364 or PRO175 polypeptide is administered together with a cardiovascular, endothelial, or angiogenic agent.

17. The use of claim 16, wherein said cardiovascular, endothelial, or angiogenic agent is selected from the group consisting of an antihypertensive drug, an ACE-inhibitor, an endothelin receptor antagonist, and a thrombolytic agent.

18. The use of claim 11, wherein the cardiovascular or endothelial or angiogenic disorder is a cancer and the PRO364 or PRO175 polypeptide is administered in combination with a chemotherapeutic agent, a growth inhibitory agent, or a cytotoxic agent.

19. A process for identifying agonists to PRO364 or PRO175 polypeptide comprising;
(a) contacting cells and a compound to be screened under conditions suitable for the stimulation of cell proliferation by PRO364 or PRO175 polypeptide; and
(b) measuring the proliferation of the cells to determine if the compound is an effective agonist;
wherein the PRO364 polypeptide:
(1) comprises amino acids 1 to 241 as set out in SEQ ID NO: 3; or
(2) comprises amino acids 26 to 241 as set out in SEQ ID NO: 3; or
(3) comprises amino acid residues Y to X as set out in SEQ ID NO: 3,
wherein Y is any one of amino acid residues 1 to 26 and X is any one of amino acids residues 157 to 167; or
(4) has at least 80% sequence identity with either a full length PRO364 polypeptide having amino acid residues 1 to 241 as set out in SEQ ID NO: 3 or an extracellular domain of PRO364 polypeptide having amino acid residues Y to X as set out in SEQ ID NO: 3, wherein Y is any one of amino acid residues 1 to 26 and X is any one of amino acids residues 157 to 167; or
wherein the PRO175 polypeptide:
(1) comprises amino acids 1 to 177 as set out in SEQ ID NO: 14; or
(2) comprises amino acids 52 to 177 as set out SEQ ID NO: 14; or
(3) comprises amino acid residues X to 177 as set out in SEQ ID NO: 3,
wherein X is any one of amino acid residues 48 to 57; or
(4) has at least 80% sequence identity with either a full length PRO175 polypeptide having amino acid residues 1 to 177 as set out in SEQ ID NO: 14 or an extracellular domain of PRO175 polypeptide having amino acid residues X to 177 as set out in SEQ ID NO: 3, wherein X is any one of amino acid residues 48 to 57.

20. A method for identifying a compound that inhibits the expression or activity of a PRO364 or PRO175 polypeptide comprising contacting a candidate compound with a PRO364 or PRO175 polypeptide under conditions and for a time sufficient to allow the compound and polypeptide to interact;
wherein the PRO364 polypeptide:
(1) comprises amino acids 1 to 241 as set out in SEQ ID NO: 3; or
(2) comprises amino acids 26 to 241 as set out in SEQ ID NO: 3; or
(3) comprises amino acid residues Y to X as set out in SEQ ID NO: 3,
wherein Y is any one of amino acid residues 1 to 26 and X is any one of amino acids residues 157 to 167; or
(4) has at least 80% sequence identity with either a full length PRO364 polypeptide having amino acid residues 1 to 241 as set out in SEQ ID NO: 3 or an extracellular domain of PRO364 polypeptide having amino acid residues Y to X as set out in SEQ ID NO: 3, wherein Y is any one of amino acid residues 1 to 26 and X is any one of amino acids residues 157 to 167; or
wherein the PRO175 polypeptide:
(1) comprises amino acids 1 to 177 as set out in SEQ ID NO: 14; or
(2) comprises amino acids 52 to 177 as set out SEQ ID NO: 14; or
(3) comprises amino acid residues X to 177 as set out in SEQ ID NO: 3,
wherein X is any one of amino acid residues 48 to 57; or
(4) has at least 80% sequence identity with either a full length PRO175 polypeptide having amino acid residues 1 to 177 as set out in SEQ ID NO: 14 or an extracellular domain of PRO175 polypeptide having amino acid residues X to 177 as set out in SEQ ID NO: 3, wherein X is any one of amino acid residues 48 to 57.

21. The process of claim 20 comprising the steps of:
(a) contacting cells and a compound to be screened in the presence of a PRO364 or PRO175 polypeptide under conditions suitable of the stimulation of cell proliferation by a PRO364 or PRO175 polypeptide; and
(b) measuring the proliferation of the cells to determine if the compound is an effective antagonist.

22. An in vitro method for determining the presence of a PRO364 or PRO175 polypeptide comprising exposing a cell suspected of containing the PRO364 or PRO175 polypeptide to an anti -PRO364 or -PRO175 antibody and determining binding of said antibody to said cell;
wherein the PRO364 polypeptide:
(1) comprises amino acids 1 to 241 as set out in SEQ ID NO: 3; or
(2) comprises amino acids 26 to 241 as set out in SEQ ID NO: 3; or
(3) comprises amino acid residues Y to X as set out in SEQ ID NO: 3,
wherein Y is any one of amino acid residues 1 to 26 and X is any one of amino acids residues 157 to 167; or
(4) has at least 80% sequence identity with either a full length PRO364 polypeptide having amino acid residues 1 to 241 as set out in SEQ ID NO: 3 or an extracellular domain of PRO364 polypeptide having amino acid residues Y to X as set out in SEQ ID NO: 3, wherein Y is any one of amino acid residues 1 to 26 and X is any one of amino acids residues 157 to 167; or
wherein the PRO175 polypeptide:
(1) comprises amino acids 1 to 177 as set out in SEQ ID NO: 14; or
(2) comprises amino acids 52 to 177 as set out SEQ ID NO: 14; or
(3) comprises amino acid residues X to 177 as set out in SEQ ID NO: 3,
wherein X is any one of amino acid residues 48 to 57; or
(4) has at least 80% sequence identity with either a full length PRO175 polypeptide having amino acid residues 1 to 177 as set out in SEQ ID NO: 14 or an extracellular domain of PRO175 polypeptide having amino acid residues X to 177 as set out in SEQ ID NO: 3, wherein X is any one of amino acid residues 48 to 57.

23. A method of diagnosing cardiovascular or endothelial or angiogenic disorders in a mammal comprising (a) contacting an anti- PRO364 or -PRO175 antibody with a test sample of tissue cells obtained from the mammal, and (b) detecting the formation of a complex between the anti- PRO364 or -PRO175 antibody and the PRO364 or PRO175 polypeptide in the test sample.

24. A cancer diagnostic kit comprising an anti- PRO364 or -PRO175 antibody and a carrier in suitable packaging.

25. The kit of claim 24 further comprising instructions for using said antibody to detect the PRO364 or PRO175 polypeptide.

26. An article of manufacture, comprising:
a container;
a label on the container; and
a composition comprising an anti- PRO364 or -PRO175 antibody contained within the container; wherein the label on the container indicates that the composition can be used for treating cardiovascular or endothelial or angiogenic disorders.

27. Use of an antibody that binds a PRO364 or PRO175 polypeptide for the preparation of a medicament inhibiting angiogenesis induced by PRO364 or PRO175 polypeptide in a mammal.

28. The use of claim 27 wherein the mammal is a human.

29. The use of claim 27 wherein the mammal has a tumor or a retinal disorder.

## Patentansprüche

1. Zusammensetzung, umfassend ein PRO364-Polypeptid in Beimischung mit einem pharmazeutisch annehmbaren Träger und einem kardiovaskulären, endothelialen oder angiogenen Mittel oder einem angiostatischen Mittel, worin das PRO364-Polypeptid:
(1) die Aminosäuren 1 bis 241 umfasst, wie in Seq.-ID Nr. 3 dargelegt; oder
(2) die Aminosäuren 26 bis 241 umfasst, wie in Seq.-ID Nr. 3 dargelegt; oder
(3) die Aminosäurereste Y bis X umfasst, wie in Seq.-ID Nr. 3 dargelegt, worin Y ein beliebiger der Aminosäurereste 1 bis 26 ist und X ein beliebiger der Aminosäurereste 157 bis 167 ist; oder
(4) eine Sequenzidentität von zumindest 80 % entweder mit einem PRO364-Polypeptid voller Länge mit Aminosäureresten 1 bis 241, wie in Seq.-ID Nr. 3 dargelegt, oder einer extrazellulären Domäne des PRO364-Polypeptids mit Aminosäureresten Y bis X, wie in Seq.-ID Nr. 3 dargelegt, aufweist, worin Y ein beliebiger der Aminosäurereste 1 bis 26 und X ein beliebiger der Aminosäurereste 157 bis 167 ist.

2. Zusammensetzung, umfassend ein PRO175-Polypeptid in Beimischung mit einem pharmazeutisch annehmbaren Träger, worin das PRO175-Polypeptid:
(1) die Aminosäuren 1 bis 177 umfasst, wie in Seq.-ID Nr. 14 dargelegt; oder
(2) die Aminosäuren 52 bis 177 umfasst, wie in Seq.-ID Nr. 14 dargelegt; oder
(3) die Aminosäurereste X bis 177 umfasst, wie in Seq.-ID Nr. 3 dargelegt, worin X ein beliebiger der Aminosäurereste 48 bis 57 ist; oder
(4) eine Sequenzidentität von zumindest 80 % entweder mit einem PRO175-Polypeptid voller Länge mit Aminosäureresten 1 bis 177, wie in Seq.-ID Nr. 14 dargelegt, oder einer extrazellulären Domäne des PRO175-Polypeptids mit Aminosäureresten X bis 177, wie in Seq.-ID Nr. 3 dargelegt, aufweist, worin X ein beliebiger der Aminosäurereste 48 bis 57 ist.

3. Zusammensetzung nach Anspruch 1 oder 2, umfassend eine therapeutisch wirksame Menge des Polypeptids.

4. Zusammensetzung nach Anspruch 2, worin das PRO175-Polypeptid in Beimischung mit einem pharmazeutisch annehmbaren Träger und einem kardiovaskulären, endothelialen oder angiogenen Mittel oder einem angiostatischen Mittel vorliegt.

5. Zusammensetzung nach Anspruch 4, worin das Polypeptid und das Mittel in therapeutisch wirksamen Mengen vorhanden sind.

6. Verfahren zur Herstellung einer Zusammensetzung eines beliebigen der vorangehenden Ansprüche zur Behandlung einer kardiovaskulären oder endothelialen oder angiogenen Erkrankung, umfassend die Beimischung einer therapeutisch wirksamen Menge eines PRO364- oder PRO175-Polypeptids zu einem pharmazeutisch annehmbaren Träger.

7. Pharmazeutisches Produkt, umfassend:
(a) eine Zusammensetzung, umfassend eine therapeutisch wirksame Menge an PRO364-Polypeptid in Beimischung mit einem kardiovaskulären, endothelialen oder angiogenen Mittel oder einem angiostatischen Mittel oder an PRO175-Polypeptid in Beimischung mit einem pharmazeutisch annehmbaren Träger;
(b) einen Behälter, der die Zusammensetzung enthält; sowie
(c) eine Markierung, die an dem Behälter angebracht ist, oder eine Packungsbeilage, die in dem pharmazeutischen Produkt inkludiert ist, die sich auf die Verwendung der PRO364- oder PRO175-Polypeptidzusammensetzung in der Behandlung einer kardiovaskulären oder endothelialen oder angiogenen Erkrankung bezieht;
worin das PRO364-Polypeptid:
(1) die Aminosäuren 1 bis 241 umfasst, wie in Seq.-ID Nr. 3 dargelegt; oder
(2) die Aminosäuren 26 bis 241 umfasst, wie in Seq.-ID Nr. 3 dargelegt; oder
(3) die Aminosäurereste Y bis X umfasst, wie in Seq.-ID Nr. 3 dargelegt, worin Y ein beliebiger der Aminosäurereste 1 bis 26 ist und X ein beliebiger der Aminosäurereste 157 bis 167 ist; oder
(4) eine Sequenzidentität von zumindest 80 % entweder mit einem PRO364-Polypeptid voller Länge mit Aminosäureresten 1 bis 241, wie in Seq.-ID Nr. 3 dargelegt, oder einer extrazellulären Domäne des PRO364-Polypeptids mit Aminosäureresten Y bis X, wie in Seq.-ID Nr. 3 dargelegt, aufweist, worin Y ein beliebiger der Aminosäurereste 1 bis 26 und X ein beliebiger der Aminosäurereste 157 bis 167 ist; oder
worin das PRO175-Polypeptid :
(1) die Aminosäuren 1 bis 177 umfasst, wie in Seq.-ID Nr. 14 dargelegt; oder
(2) die Aminosäuren 52 bis 177 umfasst, wie in Seq.-ID Nr. 14 dargelegt; oder
(3) die Aminosäurereste X bis 177 umfasst, wie in Seq.-ID Nr. 3 dargelegt, worin X ein beliebiger der Aminosäurereste 48 bis 57 ist; oder
(4) eine Sequenzidentität von zumindest 80 % entweder mit einem PRO175-Polypeptid voller Länge mit Aminosäureresten 1 bis 177, wie in Seq.-ID Nr. 14 dargelegt, oder einer extrazellulären Domäne des PRO175-Polypeptids mit Aminosäureresten X bis 177, wie in Seq.-ID Nr. 3 dargelegt, aufweist, worin X ein beliebiger der Aminosäurereste 48 bis 57 ist.

8. Verfahren zur Diagnose einer Erkrankung oder einer Anfälligkeit für eine Erkrankung, die mit einer Mutation in einer Nucleinsäuresequenz in Zusammenhang steht, die für das PRO364- oder PRO175-Polypeptid mit einer Aminosäuresequenz kodiert, wie in Seq.-ID Nr. 3 oder 14 dargelegt ist, umfassend:
(a) das Isolieren einer Nucleinsäuresequenz, die für das PRO364- oder PRO175-Polypeptid kodiert, aus einer Probe, die von einem Wirt stammt; sowie
(b) das Bestimmen einer Mutation in der Nucleinsäuresequenz, die für das PRO364- oder PRO175-Polypeptid kodiert.

9. Verfahren zur Diagnose kardiovaskulärer oder endothelialer oder angiogener Erkrankungen bei einem Säugetier, umfassend die Detektion der Expressionsmenge eines Gens, das für ein PRO364- oder PRO175-Polypeptid mit einer Aminosäuresequenz kodiert, wie in Seq.-ID Nr. 3 oder 14 dargelegt, und zwar (a) in einer Testprobe an Gewebezellen, die von dem Säugetier erhalten wurden, und (b) in einer Kontrollprobe bekannter normaler Gewebezellen desselben Zelltyps, worin eine höhere oder geringere Expressionsmenge in der Testprobe die Gegenwart einer kardiovaskulären oder endothelialen oder angiogenen Dysfunktion bei dem Säugetier zeigt, von dem die Testgewebe-Zellen erhalten wurden.

10. Verwendung eines PRO364- oder PRO175-Polypeptids zur Herstellung eines Medikaments zur Behandlung einer kardiovaskulären oder endothelialen oder angiogenen Erkrankung in einem Säugetier;
worin das PRO364-Polypeptid:
(1) die Aminosäuren 1 bis 241 umfasst, wie in Seq.-ID Nr. 3 dargelegt; oder
(2) die Aminosäuren 26 bis 241 umfasst, wie in Seq.-ID Nr. 3 dargelegt; oder
(3) die Aminosäurereste Y bis X umfasst, wie in Seq.-ID Nr. 3 dargelegt, worin Y ein beliebiger der Aminosäurereste 1 bis 26 ist und X ein beliebiger der Aminosäurereste 157 bis 167 ist; oder
(4) eine Sequenzidentität von zumindest 80 % entweder mit einem PRO364-Polypeptid voller Länge mit Aminosäureresten 1 bis 241, wie in Seq.-ID Nr. 3 dargelegt, oder einer extrazellulären Domäne des PRO364-Polypeptids mit Aminosäureresten Y bis X, wie in Seq.-ID Nr. 3 dargelegt, aufweist, worin Y ein beliebiger der Aminosäurereste 1 bis 26 und X ein beliebiger der Aminosäurereste 157 bis 167 ist; oder
worin das PRO175-Polypeptid:
(1) die Aminosäuren 1 bis 177 umfasst, wie in Seq.-ID Nr. 14 dargelegt; oder
(2) die Aminosäuren 52 bis 177 umfasst, wie in Seq.-ID Nr. 14 dargelegt; oder
(3) die Aminosäurereste X bis 177 umfasst, wie in Seq.-ID Nr. 3 dargelegt, worin X ein beliebiger der Aminosäurereste 48 bis 57 ist; oder
(4) eine Sequenzidentität von zumindest 80 % entweder mit einem PRO175-Polypeptid voller Länge mit Aminosäureresten 1 bis 177, wie in Seq.-ID Nr. 14 dargelegt, oder einer extrazellulären Domäne des PRO175-Polypeptids mit Aminosäureresten X bis 177, wie in Seq.-ID Nr. 3 dargelegt, aufweist, worin X ein beliebiger der Aminosäurereste 48 bis 57 ist.

11. Verwendung nach Anspruch 10, worin es sich bei der kardiovaskulären oder endothelialen oder angiogenen Erkrankung um Herzhypertrophie oder Trauma oder Krebs handelt.

12. Verwendung nach Anspruch 10, worin das Säugetier ein Mensch ist.

13. Verwendung nach Anspruch 11, worin die Herzhypertrophie durch die Gegenwart einer erhöhten Menge an PGF_{2α} **gekennzeichnet** ist.

14. Verwendung nach Anspruch 11, worin die Herzhypertrophie durch Myocardinfarkt induziert wurde.

15. Verwendung nach Anspruch 14, worin die Verabreichung des PRO364- oder PRO175-Polypeptids innerhalb von 48 Stunden nach dem Myocardinfarkt initiiert wird.

16. Verwendung nach Anspruch 11, worin es sich bei der kardiovaskulären oder endothelialen oder angiogenen Erkrankung um Herzhypertrophie handelt und das PRO364- oder PRO175-Polypeptid zusammen mit einem kardiovaskulären, endothelialen oder angiogenen Mittel verabreicht wird.

17. Verwendung nach Anspruch 16, worin das kardiovaskuläre, endotheliale oder angiogene Mittel aus der aus einem blutdrucksenkenden Arzneimittel, einem ACE-Hemmer, einem Endothelin-Rezeptorantagonisten und einem thrombolytischen Mittel bestehenden Gruppe ausgewählt ist.

18. Verwendung nach Anspruch 11, worin die kardiovaskuläre, endotheliale oder angiogene Erkrankung eine Krebsart ist und das PRO364- oder PRO175-Polypeptid in Kombination mit einem chemotherapeutischen Mittel, einem wachstumshemmenden Mittel oder einem zytotoxischen Mittel verabreicht wird.

19. Verfahren zur Identifikation von Agonisten für das PRO364- oder PRO175-Polypeptid, umfassend:
(a) das Kontaktieren von Zellen und einer Verbindung, die gescreent werden soll, unter Bedingungen, die für die Stimulierung der Zellproliferation durch das PRO364- oder PRO175-Polypeptid geeignet sind; und
(b) das Messen der Zellproliferation, um zu bestimmen, ob die Verbindung ein wirksamer Agonist ist;
worin das PRO364-Polypeptid:
(1) die Aminosäuren 1 bis 241 umfasst, wie in Seq.-ID Nr. 3 dargelegt; oder
(2) die Aminosäuren 26 bis 241 umfasst, wie in Seq.-ID Nr. 3 dargelegt; oder
(3) die Aminosäurereste Y bis X umfasst, wie in Seq.-ID Nr. 3 dargelegt, worin Y ein beliebiger der Aminosäurereste 1 bis 26 ist und X ein beliebiger der Aminosäurereste 157 bis 167 ist; oder
(4) eine Sequenzidentität von zumindest 80 % entweder mit einem PRO364-Polypeptid voller Länge mit Aminosäureresten 1 bis 241, wie in Seq.-ID Nr. 3 dargelegt, oder einer extrazellulären Domäne des PRO364-Polypeptids mit Aminosäureresten Y bis X, wie in Seq.-ID Nr. 3 dargelegt, aufweist, worin Y ein beliebiger der Aminosäurereste 1 bis 26 und X ein beliebiger der Aminosäurereste 157 bis 167 ist; oder
worin das PRO175-Polypeptid:
(1) die Aminosäuren 1 bis 177 umfasst, wie in Seq.-ID Nr. 14 dargelegt; oder
(2) die Aminosäuren 52 bis 177 umfasst, wie in Seq.-ID Nr. 14 dargelegt; oder
(3) die Aminosäurereste X bis 177 umfasst, wie in Seq.-ID Nr. 3 dargelegt, worin X ein beliebiger der Aminosäurereste 48 bis 57 ist; oder
(4) eine Sequenzidentität von zumindest 80 % entweder mit einem PRO175-Polypeptid voller Länge mit Aminosäureresten 1 bis 177, wie in Seq.-ID Nr. 14 dargelegt, oder einer extrazellulären Domäne des PRO175-Polypeptids mit Aminosäureresten X bis 177, wie in Seq.-ID Nr. 3 dargelegt, aufweist, worin X ein beliebiger der Aminosäurereste 48 bis 57 ist.

20. Verfahren zur Identifikation einer Verbindung, welche die Expression oder Aktivität eines PRO364- oder PRO175-Polypeptids inhibiert, umfassend das Kontaktieren einer Kandidatenverbindung mit einem PRO364- oder PRO175-Polypeptid unter Bedingungen und für eine Zeitspanne, die ausreichen/ausreicht, um eine Wechselwirkung zwischen der Verbindung und dem Polypeptid zu ermöglichen;
worin das PRO364-Polypeptid:
(1) die Aminosäuren 1 bis 241 umfasst, wie in Seq.-ID Nr. 3 dargelegt; oder
(2) die Aminosäuren 26 bis 241 umfasst, wie in Seq.-ID Nr. 3 dargelegt; oder
(3) die Aminosäurereste Y bis X umfasst, wie in Seq.-ID Nr. 3 dargelegt, worin Y ein beliebiger der Aminosäurereste 1 bis 26 ist und X ein beliebiger der Aminosäurereste 157 bis 167 ist; oder
(4) eine Sequenzidentität von zumindest 80 % entweder mit einem PRO364-Polypeptid voller Länge mit Aminosäureresten 1 bis 241, wie in Seq.-ID Nr. 3 dargelegt, oder einer extrazellulären Domäne des PRO364-Polypeptids mit Aminosäureresten Y bis X, wie in Seq.-ID Nr. 3 dargelegt, aufweist, worin Y ein beliebiger der Aminosäurereste 1 bis 26 und X ein beliebiger der Aminosäurereste 157 bis 167 ist; oder
worin das PRO175-Polypeptid:
(1) die Aminosäuren 1 bis 177 umfasst, wie in Seq.-ID Nr. 14 dargelegt; oder
(2) die Aminosäuren 52 bis 177 umfasst, wie in Seq.-ID Nr. 14 dargelegt; oder
(3) die Aminosäurereste X bis 177 umfasst, wie in Seq.-ID Nr. 3 dargelegt, worin X ein beliebiger der Aminosäurereste 48 bis 57 ist; oder
(4) eine Sequenzidentität von zumindest 80 % entweder mit einem PRO175-Polypeptid voller Länge mit Aminosäureresten 1 bis 177, wie in Seq.-ID Nr. 14 dargelegt, oder einer extrazellulären Domäne des PRO175-Polypeptids mit Aminosäureresten X bis 177, wie in Seq.-ID Nr. 3 dargelegt, aufweist, worin X ein beliebiger der Aminosäurereste 48 bis 57 ist.

21. Verfahren nach Anspruch 20, umfassend folgende Schritte:
(a) das Kontaktieren von Zellen und einer Verbindung, die gescreent werden soll, in Gegenwart eines PRO364- oder PRO175-Polypeptids unter Bedingungen, die für die Stimulierung der Zellproliferation durch ein PRO364- oder PRO175-Polypeptid geeignet sind; und
(b) das Messen der Zellproliferation, um zu bestimmen, ob die Verbindung ein wirksamer Antagonist ist.

22. In-vitro-Verfahren zur Bestimmung der Gegenwart eines PRO364- oder PRO175-Polypeptids, umfassend das Aussetzen einer Zelle, von der angenommen wird, dass sie das PRO364- oder PRO175-Polypeptid enthält, gegenüber einem Anti-PRO364- oder -PRO175-Antikörper sowie das Bestimmen der Bindung des Antikörpers an die Zelle;
worin das PRO364-Polypeptid:
(1) die Aminosäuren 1 bis 241 umfasst, wie in Seq.-ID Nr. 3 dargelegt; oder
(2) die Aminosäuren 26 bis 241 umfasst, wie in Seq.-ID Nr. 3 dargelegt; oder
(3) die Aminosäurereste Y bis X umfasst, wie in Seq.-ID Nr. 3 dargelegt, worin Y ein beliebiger der Aminosäurereste 1 bis 26 ist und X ein beliebiger der Aminosäurereste 157 bis 167 ist; oder
(4) eine Sequenzidentität von zumindest 80 % entweder mit einem PRO364-Polypeptid voller Länge mit Aminosäureresten 1 bis 241, wie in Seq.-ID Nr. 3 dargelegt, oder einer extrazellulären Domäne des PRO364-Polypeptids mit Aminosäureresten Y bis X, wie in Seq.-ID Nr. 3 dargelegt, aufweist, worin Y ein beliebiger der Aminosäurereste 1 bis 26 und X ein beliebiger der Aminosäurereste 157 bis 167 ist; oder
worin das PRO175-Polypeptid:
(1) die Aminosäuren 1 bis 177 umfasst, wie in Seq.-ID Nr. 14 dargelegt; oder
(2) die Aminosäuren 52 bis 177 umfasst, wie in Seq.-ID Nr. 14 dargelegt; oder
(3) die Aminosäurereste X bis 177 umfasst, wie in Seq.-ID Nr. 3 dargelegt, worin X ein beliebiger der Aminosäurereste 48 bis 57 ist; oder
(4) eine Sequenzidentität von zumindest 80 % entweder mit einem PRO175-Polypeptid voller Länge mit Aminosäureresten 1 bis 177, wie in Seq.-ID Nr. 14 dargelegt, oder einer extrazellulären Domäne des PRO175-Polypeptids mit Aminosäureresten X bis 177, wie in Seq.-ID Nr. 3 dargelegt, aufweist, worin X ein beliebiger der Aminosäurereste 48 bis 57 ist.

23. Verfahren zur Diagnose kardiovaskulärer oder endothelialer oder angiogener Erkrankungen bei einem Säugetier, umfassend (a) das Kontaktieren eines Anti-PRO364- oder -PRO175-Antikörpers mit einer Testprobe an Gewebezellen, die von dem Säugetier erhalten wurden, und (b) das Detektieren der Bildung eines Komplexes zwischen dem Anti-PRO364- oder -PRO175-Antikörper und dem PRO364- oder PRO175-Polypeptid in der Testprobe.

24. Krebs-Diagnose-Set, umfassend einen Anti-PRO364- oder -PRO175-Antikörper und einen Träger in einer geeigneten Verpackung.

25. Set nach Anspruch 24, weiters umfassend Anweisungen zur Verwendung des Antikörpers zur Detektion des PRO364- oder PRO75-Polypeptids.

26. Artikel, umfassend:
einen Behälter;
eine Markierung auf dem Behälter; sowie
eine Zusammensetzung, umfassend einen Anti-PRO364- oder -PRO175-Antikörper, die im Behälter enthalten ist; worin die Markierung auf dem Behälter angibt, dass die Zusammensetzung zur Behandlung von kardiovaskulären oder endothelialen oder angiogenen Erkrankungen verwendet werden kann.

27. Verwendung eines Antikörpers, der ein PRO364- oder -PRO175-Polypeptid bindet, zur Herstellung eines Medikaments, das die Angiogenese in einem Säugetier inhibiert, die durch das PRO364- oder -PRO175-Polypeptid induziert wird.

28. Verwendung nach Anspruch 27, worin das Säugetier ein Mensch ist.

29. Verwendung nach Anspruch 27, worin das Säugetier unter einem Tumor oder einer Netzhauterkrankung leidet.

## Revendications

1. Composition comprenant un polypeptide PRO364 en mélange avec un support pharmaceutiquement acceptable et un agent cardio-vasculaire, endothélial ou angiogénique ou un agent angiostatique, dans laquelle le polypeptide PRO364 :
(1) comprend les aminoacides 1 à 241 indiqués dans la SEQ ID N° 3 ; ou
(2) comprend les aminoacides 26 à 241 indiqués dans la SEQ ID N° 3 ; ou
(3) comprend les résidus d'aminoacides Y à X indiqués dans la SEQ ID N° 3, où Y représente n'importe lequel des résidus d'aminoacides 1 à 26 et X représente n'importe lequel des résidus d'aminoacides 157 à 167 ; ou
(4) a une identité de séquence d'au moins 80 % avec un polypeptide PRO364 complet comprenant les résidus d'aminoacides 1 à 241 indiqués dans la SEQ ID N° 3 ou un domaine extracellulaire du polypeptide PRO364 comprenant les résidus d'aminoacides Y à X indiqués dans la SEQ ID N° 3, où Y représente n'importe lequel des résidus d'aminoacides 1 à 26 et X représente n'importe lequel des résidus d'aminoacides 157 à 167.

2. Composition comprenant un polypeptide PRO175 en mélange avec un support pharmaceutiquement acceptable, dans laquelle le polypeptide PRO175 :
(1) comprend les aminoacides 1 à 177 indiqués dans la SEQ ID N° 14 ; ou
(2) comprend les aminoacides 52 à 177 indiqués dans la SEQ ID N° 14 ; ou
(3) comprend les résidus d'aminoacides X à 177 indiqués dans la SEQ ID N° 3, où X représente n'importe lequel des résidus d'aminoacides 48 à 57 ; ou
(4) a une identité de séquence d'au moins 80 % avec un polypeptide PRO175 complet comprenant les résidus d'aminoacides 1 à 177 indiqués dans la SEQ ID N° 14 ou un domaine extracellulaire du polypeptide PRO175 comprenant les résidus d'aminoacides X à 177 indiqués dans la SEQ ID N° 3, où X représente n'importe lequel des résidus d'aminoacides 48 à 57.

3. Composition suivant la revendication 1 ou 2, comprenant une quantité thérapeutiquement efficace du polypeptide.

4. Composition suivant la revendication 2, dans laquelle le polypeptide PRO175 est en mélange avec un support pharmaceutiquement acceptable et un agent cardio-vasculaire, endothélial ou angiogénique ou un agent angiostatique.

5. Composition suivant la revendication 4, dans laquelle le polypeptide et l'agent sont présents en des quantités thérapeutiquement efficaces.

6. Procédé pour la préparation d'une composition de l'une quelconque des revendications précédentes pour le traitement d'un trouble cardio-vasculaire, endothélial ou angiogénique, comprenant le mélange d'une quantité thérapeutiquement efficace du polypeptide PRO364 ou PRO175 à un support pharmaceutiquement acceptable.

7. Produit pharmaceutique comprenant :
(a) une composition comprenant une quantité thérapeutiquement efficace du polypeptide PRO364 en mélange avec un agent cardio-vasculaire, endothélial ou angiogénique ou un agent angiostatique, ou du polypeptide PRO175, en mélange avec un support pharmaceutiquement acceptable ;
(b) un récipient contenant ladite composition ; et
(c) une étiquette fixée audit récipient, ou à un emballage incorporé audit produit pharmaceutique mentionnant l'utilisation de ladite composition de polypeptide PRO364 ou PRO175 dans le traitement d'un trouble cardio-vasculaire, endothélial ou angiogénique ;
dans lequel le polypeptide PRO364 :
(1) comprend les aminoacides 1 à 241 indiqués dans la SEQ ID N° 3 ; ou
(2) comprend les aminoacides 26 à 241 indiqués dans la SEQ ID N° 3 ; ou
(3) comprend les résidus d'aminoacides Y à X indiqués dans la SEQ ID N° 3, où Y représente n'importe lequel des résidus d'aminoacides 1 à 26 et X représente n'importe lequel des résidus d'aminoacides 157 à 167 ; ou
(4) a une identité de séquence d'au moins 80 % avec un polypeptide PRO364 complet comprenant les résidus d'aminoacides 1 à 241 indiqués dans la SEQ ID N° 3 ou un domaine extracellulaire du polypeptide PRO364 comprenant les résidus d'aminoacides Y à X indiqués dans la SEQ ID N°3, où Y représente n'importe lequel des résidus d'aminoacides 1 à 26 et X représente n'importe lequel des résidus d'aminoacides 157 à 167 ; ou
dans lequel le polypeptide PRO175 :
(1) comprend les aminoacides 1 à 177 indiqués dans la SEQ ID N° 14 ; ou
(2) comprend les aminoacides 52 à 177 indiqués dans la SEQ ID N° 14 ; ou
(3) comprend les résidus d'aminoacides X à 177 indiqués dans la SEQ ID N° 3, où X représente n'importe lequel des résidus d'aminoacides 48 à 57 ; ou
(4) a une identité de séquence d'au moins 80 % avec un polypeptide PRO175 complet comprenant les résidus d'aminoacides 1 à 177 indiqués dans la SEQ ID N° 14 ou un domaine extracellulaire du polypeptide PRO175 comprenant les résidus d'aminoacides X à 177 indiqués dans la SEQ ID N° 3, où X représente n'importe lequel des résidus d'aminoacides 48 à 57.

8. Procédé pour diagnostiquer une maladie ou une prédisposition à une maladie en rapport avec une mutation dans une séquence d'acide nucléique codant pour le polypeptide PRO364 ou PRO175 ayant une séquence d'aminoacides indiquée dans la SEQ ID N° 3 ou 14, comprenant :
(a) l'isolement d'une séquence d'acide nucléique codant pour le polypeptide PRO364 ou PRO175 à partir d'un échantillon dérivé d'un hôte ; et
(b) la détermination d'une mutation dans la séquence d'acide nucléique codant pour le polypeptide PRO364 ou PRO175.

9. Méthode pour le diagnostic de troubles cardio-vasculaires, endothéliaux ou angiogéniques chez un mammifère, comprenant la détection du degré d'expression d'un gène codant pour un polypeptide PRO364 ou PRO175 ayant une séquence d'aminoacides indiquée dans la SEQ ID N° 3 ou 14 (a) dans un échantillon d'essai de cellules d'un tissu obtenues à partir du mammifère, et (b) dans un échantillon témoin de cellules d'un tissu normal connues du même type cellulaire, dans laquelle un degré d'expression supérieur ou inférieur dans l'échantillon d'essai indique la présence d'un dysfonctionnement cardio-vasculaire, endothélial ou angiogénique chez le mammifère à partir duquel les cellules du tissu d'essai ont été obtenues.

10. Utilisation d'un polypeptide PRO364 ou PRO175 pour la préparation d'un médicament destiné au traitement d'un trouble cardio-vasculaire, endothélial ou angiogénique chez un mammifère ;
dans laquelle le polypeptide PRO364 :
(1) comprend les aminoacides 1 à 241 indiqués dans la SEQ ID N° 3 ; ou
(2) comprend les aminoacides 26 à 241 indiqués dans la SEQ ID N° 3 ; ou
(3) comprend les résidus d'aminoacides Y à X indiqués dans la SEQ ID N° 3, où Y représente n'importe lequel des résidus d'aminoacides 1 à 26 et X représente n'importe lequel des résidus d'aminoacides 157 à 167 ; ou
(4) a une identité de séquence d'au moins 80 % avec un polypeptide PRO364 complet comprenant les résidus d'aminoacides 1 à 241 indiqués dans la SEQ ID N° 3 ou un domaine extracellulaire du polypeptide PRO364 comprenant les résidus d'aminoacides Y à X indiqués dans la SEQ ID N° 3, où Y représente n'importe lequel des résidus d'aminoacides 1 à 26 et X représente n'importe lequel des résidus d'aminoacides 157 à 167 ; ou
dans laquelle le polypeptide PRO175 :
(1) comprend les aminoacides 1 à 177 indiqués dans la SEQ ID N° 14 ; ou
(2) comprend les aminoacides 52 à 177 indiqués dans la SEQ ID N° 14 ; ou
(3) comprend les résidus d'aminoacides X à 177 indiqués dans la SEQ ID N° 3, où X représente n'importe lequel des résidus d'aminoacides 48 à 57 ; ou
(4) a une identité de séquence d'au moins 80 % avec un polypeptide PRO175 complet comprenant les résidus d'aminoacides 1 à 177 indiqués dans la SEQ ID N° 14 ou un domaine extracellulaire du polypeptide PRO175 comprenant les résidus d'aminoacides X à 177 indiqués dans la SEQ ID N° 3, où X représente n'importe lequel des résidus d'aminoacides 48 à 57.

11. Utilisation suivant la revendication 10, dans laquelle le trouble cardio-vasculaire, endothélial ou angiogénique est une hypertrophie cardiaque, un traumatisme ou un cancer.

12. Utilisation suivant la revendication 10, dans laquelle ledit mammifère est un être humain.

13. Utilisation suivant la revendication 11, dans laquelle ladite hypertrophie cardiaque est **caractérisée par** la présence d'un taux élevé de PGF_{2α}.

14. Utilisation suivant la revendication 11, dans laquelle ladite hypertrophie cardiaque a été induite par un infarctus du myocarde.

15. Utilisation suivant la revendication 14, dans laquelle l'administration dudit polypeptide PRO364 ou PRO175 est commencée dans les 48 heures après un infarctus du myocarde.

16. Utilisation suivant la revendication 11, dans laquelle le trouble cardio-vasculaire, endothélial ou $angiogénique est l'hypertrophie cardiaque et ledit polypeptide PRO364 ou PRO175 est administré conjointement avec un agent cardio-vasculaire, endothélial ou angiogénique.

17. Utilisation suivant la revendication 16, dans laquelle ledit agent cardio-vasculaire, endothélial ou angiogénique est choisi dans le groupe consistant en un médicament antihypertensif, un inhibiteur de ACE, un antagoniste du récepteur d'endothéline et un agent thrombolytique.

18. Utilisation suivant la revendication 11, dans laquelle le trouble cardio-vasculaire, endothélial ou angiogénique est un cancer et le polypeptide PRO364 ou PRO175 est administré en association avec un agent chimiothérapeutique, un inhibiteur de croissance ou un agent cytotoxique.

19. Procédé pour identifier des agonistes du polypeptide PRO364 ou PRO175, comprenant :
(a) la mise en contact de cellules et d'un composé à tester dans des conditions convenables pour la stimulation de la prolifération cellulaire par le polypeptide PRO364 ou PRO175 ; et
(b) la mesure de la prolifération des cellules pour déterminer si le composé est un agoniste efficace ;
dans lequel le polypeptide PRO364 :
(1) comprend les aminoacides 1 à 241 indiqués dans la SEQ ID N° 3 ; ou
(2) comprend les aminoacides 26 à 241 indiqués dans la SEQ ID N° 3 ; ou
(3) comprend les résidus d'aminoacides Y à X indiqués dans la SEQ ID N° 3, où Y représente n'importe lequel des résidus d'aminoacides 1 à 26 et X représente n'importe lequel des résidus d'aminoacides 157 à 167 ; ou
(4) a une identité de séquence d'au moins 80 % avec un polypeptide PRO364 complet comprenant les résidus d'aminoacides 1 à 241 indiqués dans la SEQ ID N° 3 ou un domaine extracellulaire du polypeptide PRO364 comprenant les résidus d'aminoacides Y à X indiqués dans la SEQ ID N° 3, où Y représente n'importe lequel des résidus d'aminoacides 1 à 26 et X représente n'importe lequel des résidus d'aminoacides 157 à 167 ; ou
dans lequel le polypeptide PRO175 :
(1) comprend les aminoacides 1 à 177 indiqués dans la SEQ ID N° 14 ; ou
(2) comprend les aminoacides 52 à 177 indiqués dans la SEQ ID N° 14 ; ou
(3) comprend les résidus d'aminoacides X à 177 indiqués dans la SEQ ID N° 3, où X représente n'importe lequel des résidus d'aminoacides 48 à 57 ; ou
(4) a une identité de séquence d'au moins 80 % avec un polypeptide PRO175 complet comprenant les résidus d'aminoacides 1 à 177 indiqués dans la SEQ ID N° 14 ou un domaine extracellulaire du polypeptide PRO175 comprenant les résidus d'aminoacides X à 177 indiqués dans la SEQ ID N° 3, où X représente n'importe lequel des résidus d'aminoacides 48 à 57.

20. Méthode pour identifier un composé qui inhibe l'expression ou l'activité d'un polypeptide PRO364 ou PRO175, comprenant la mise en contact d'un composé candidat avec un polypeptide PRO364 ou PRO175 dans des conditions et pendant un temps suffisants pour permettre l'interaction du composé et du polypeptide ;
dans laquelle le polypeptide PRO364 :
(1) comprend les aminoacides 1 à 241 indiqués dans la SEQ ID N° 3 ; ou
(2) comprend les aminoacides 26 à 241 indiqués dans la SEQ ID N° 3 ; ou
(3) comprend les résidus d'aminoacides Y à X indiqués dans la SEQ ID N° 3, où Y représente n'importe lequel des résidus d'aminoacides 1 à 26 et X représente n'importe lequel des résidus d'aminoacides 157 à 167 ; ou
(4) a une identité de séquence d'au moins 80 % avec un polypeptide PRO364 complet comprenant les résidus d'aminoacides 1 à 241 indiqués dans la SEQ ID N° 3 ou un domaine extracellulaire du polypeptide PRO364 comprenant les résidus d'aminoacides Y à X indiqués dans la SEQ ID N° 3, où Y représente n'importe lequel des résidus d'aminoacides 1 à 26 et X représente n'importe lequel des résidus d'aminoacides 157 à 167 ; ou
dans laquelle le polypeptide PRO175 :
(1) comprend les aminoacides 1 à 177 indiqués dans la SEQ ID N° 14 ; ou
(2) comprend les aminoacides 52 à 177 indiqués dans la SEQ ID N° 14 ; ou
(3) comprend les résidus d'aminoacides X à 177 indiqués dans la SEQ ID N° 3, où X représente n'importe lequel des résidus d'aminoacides 48 à 57 ; ou
(4) a une identité de séquence d'au moins 80 % avec un polypeptide PRO175 complet comprenant les résidus d'aminoacides 1 à 177 indiqués dans la SEQ ID N° 14 ou un domaine extracellulaire du polypeptide PRO175 comprenant les résidus d'aminoacides X à 177 indiqués dans la SEQ ID N° 3, où X représente n'importe lequel des résidus d'aminoacides 48 à 57.

21. Procédé suivant la revendication 20, comprenant les étapes consistant à :
(a) mettre en contact des cellules et un composé à tester en présence d'un polypeptide PRO364 ou PRO175 dans des conditions convenables pour la stimulation de la prolifération cellulaire par un polypeptide PRO364 ou PRO175 ; et
(b) mesurer la prolifération des cellules pour déterminer si le composé est un antagoniste efficace.

22. Méthode in vitro pour déterminer la présence d'un polypeptide PRO364 ou PRO175, comprenant l'exposition d'une cellule supposée contenir le polypeptide PRO364 ou PRO175 à un anticorps anti-PRO364 ou -PRO175 et la détermination de la liaison dudit anticorps à ladite cellule ;
dans laquelle le polypeptide PRO364 :
(1) comprend les aminoacides 1 à 241 indiqués dans la SEQ ID N° 3 ; ou
(2) comprend les aminoacides 26 à 241 indiqués dans la SEQ ID N° 3 ; ou
(3) comprend les résidus d'aminoacides Y à X indiqués dans la SEQ ID N° 3, où Y représente n'importe lequel des résidus d'aminoacides 1 à 26 et X représente n'importe lequel des résidus d'aminoacides 157 à 167 ; ou
(4) a une identité de séquence d'au moins 80 % avec un polypeptide PRO364 complet comprenant les résidus d'aminoacides 1 à 241 indiqués dans la SEQ ID N° 3 ou un domaine extracellulaire du polypeptide PRO364 comprenant les résidus d'aminoacides Y à X indiqués dans la SEQ ID N° 3, où Y représente n'importe lequel des résidus d'aminoacides 1 à 26 et X représente n'importe lequel des résidus d'aminoacides 157 à 167 ; ou
dans laquelle le polypeptide PRO175 :
(1) comprend les aminoacides 1 à 177 indiqués dans la SEQ ID N° 14 ; ou
(2) comprend les aminoacides 52 à 177 indiqués dans la SEQ ID N° 14 ; ou
(3) comprend les résidus d'aminoacides X à 177 indiqués dans la SEQ ID N° 3, où X représente n'importe lequel des résidus d'aminoacides 48 à 57 ; ou
(4) a une identité de séquence d'au moins 80 % avec un polypeptide PRO175 complet comprenant les résidus d'aminoacides 1 à 177 indiqués dans la SEQ ID N° 14 ou un domaine extracellulaire du polypeptide PRO175 comprenant les résidus d'aminoacides X à 177 indiqués dans la SEQ ID N° 3, où X représente n'importe lequel des résidus d'aminoacides 48 à 57.

23. Méthode pour le diagnostic de troubles cardio-vasculaires, endothéliaux ou angiogéniques chez un mammifère, comprenant (a) la mise en contact d'un anticorps anti-PRO364 ou -PRO175 avec un échantillon d'essai de cellules d'un tissu obtenues à partir du mammifère, et (b) la détection de la formation d'un complexe entre l'anticorps anti-PRO364 ou -PRO175 et le polypeptide PRO364 ou PRO175 dans l'échantillon d'essai.

24. Kit de diagnostic du cancer, comprenant un anticorps anti-PRO364 ou -PRO175 et un support dans un emballage convenable.

25. Kit suivant la revendication 24, comprenant en outre des instructions pour l'utilisation dudit anticorps afin de détecter le polypeptide PRO364 ou PRO175.

26. Article manufacturé, comprenant :
un récipient ;
une étiquette sur le récipient ; et
une composition comprenant un anticorps anti-PRO364 ou -PRO175 présente dans le récipient ; dans lequel l'étiquette sur le récipient indique que la composition peut être utilisée pour le traitement de troubles cardio-vasculaires, endothéliaux ou angiogéniques.

27. Utilisation d'un anticorps qui se lie à un polypeptide PRO364 ou PRO175 pour la préparation d'un médicament inhibant l'angiogenèse induite par le polypeptide PRO364 ou PRO175 chez un mammifère.

28. Utilisation suivant la revendication 27, dans laquelle le mammifère est un être humain.

29. Utilisation suivant la revendication 27, dans laquelle le mammifère a une tumeur ou un trouble rétinien.
